# FASCICULE DE BREVET EUROPEEN

(11) **EP 0 553 016 B1**
(45) Date de publication et mention de la délivrance du brevet: **07.05.1997**
(21) Numéro de dépôt: 93400111.6
(22) Date de dépôt: 19.01.1993
(51) Int. Cl.: C07C 311/35, C07C 237/20, A61K 31/18, A61K 31/165, C07D 295/08, C07D 209/52

(54) **Nouveaux amides et sulfonamides naphtaleniques, leurs procédés de préparation et les compositions pharmaceutiques qui les contiennent**
Neue Naphthalin-Amide und Sulfonamide, Verfahren zu ihrer Herstellung und sie enthaltende pharmazeutische Zusammensetzungen
New naphthalene amides and sulfonamides, process for their preparation and pharmaceutical compositions containing them

(30) Priorité: 22.01.1992 FR 9200608
(43) Date de publication de la demande: 28.07.1993
(73) Titulaire: ADIR ET COMPAGNIE, 92415 Courbevoie Cédex (FR)
(72) Inventeur: Depreux, Patrick, F-59280 Armentières (FR); Lesieur, Daniel, F-59147 Gondecourt (FR); Abdellaoui, Habib, F-59553 Cuincy (FR); Guardiola, Béatrice, F-92200 Neuilly Sur Seine (FR); Adam, Gérard, F-78600 Le Mesnil Le Roi (FR); Renard, Pierre, F-78000 Versaillles (FR); Pfeiffer, Bruno, F-95600 Eaubonne (FR)

(56) Documents cités:
- EP-A- 0 091 749
- EP-A- 0 397 044
- GB-A- 2 082 175
- GB-A- 2 162 522
- US-A- 4 217 306
- US-A- 4 900 739

## Description

La présente invention concerne de nouveaux amides et sulfonamides naphtaléniques, leurs procédés de préparation et les compositions pharmaceutiques qui les contiennent.

De nombreux dérivés d'acides possédant une structure naphtalénique sont décrits dans la littérature.

En ce qui concerne les sulfonamides, le brevet Européen EP 397044 décrit des arylsulfonamides en tant qu'antagonistes du Thromboxane et inhibiteurs de la Thromboxane synthétase.

Le brevet Américain US 4900739 décrit des sulfonamides à noyau naphtalénique utilisés comme intermédiaires de synthèse d'inhibiteurs d'aldose réductase

Le brevet anglais GB-A-2162522 décrit des sulfonamides utiles en tant qu'antimigraineux.

La publication J. Org. Chem (1990) 55 (8) pp 2563-4 décrit, entre autres, l'obtention de sulfonamides à noyau naphtalénique.

En ce qui concerne les amides, le brevet Japonais JP 90256655 décrit des composés naphtalèneacétamidiques antagonistes du récepteur au glutamate.

La publication J. Med. Chem (1985) 28 (11) pp 1721-7 décrit des composés N-alkyl 2-naphtalèneacétamidiques comme intermédiaires de synthèse d'antidépresseurs.

La demanderesse a présentement découvert de nouveaux amides et sulfonamides à noyau naphtalénique qui possèdent la propriété de se fixer avec une haute affinité sur les récepteurs sérotoninergiques. Cette affinité se double d'une sélectivité très élevée pour les récepteurs 5 HT₁ et plus particulièrement pour les récepteurs 5 HT₁D.

Les amides et sulfonamides cités dans la littérature, outre leur grande différence structurale avec les composés de l'invention, ne sont à aucun moment décrits comme possèdant une telle affinité pour les récepteurs sérotoninergiques.

La très bonne affinité des composés de l'invention pour les récepteurs sérotoninergiques, associée à leurs propriétés vasoconstrictrices et à leur faible toxicité, les rend précieux pour le traitement de douleurs dues à une vasodilatation du système vasculaire cranien et donc, en particulier, des migraines, des céphalées et des algies vasculaires de la face.

La présente invention concerne plus spécifiquement les composés naphtaléniques de formule générale (I) : 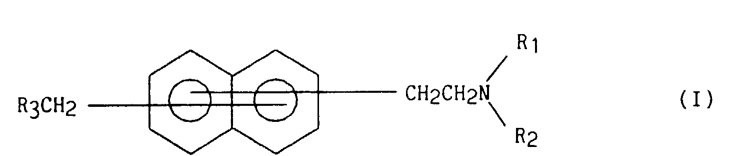 dans laquelle, les deux substituants, indépendemment l'un de l'autre, pouvant être localisés sur n'importe lequel des deux cycles du naphtalène,
- R₁ et R₂, identiques ou différents, représentent chacun indépendamment l'un de l'autre :
   - un hydrogène.
   - un alkyle linéaire ou ramifié de 1 à 6 atomes de carbone,
   - un cycloalkyle de 3 à 7 atomes de carbone, ou cycloalkyle-(C1-C4) alkyle
   - un alcène linéaire ou ramifié de 2 à 6 atomes de carbone,
   - un aryle éventuellement substitué,
   - un aralkyle éventuellement substitué dont la chaine alkyle comprend de 1 à 3 atomes de carbone,
   ou forment ensemble, avec l'atome d'azote qui les porte un système cyclique choisi parmi : 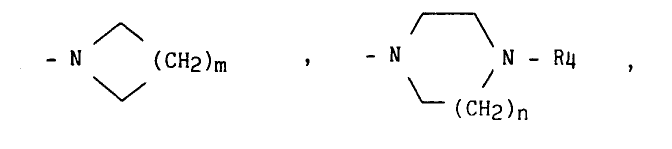 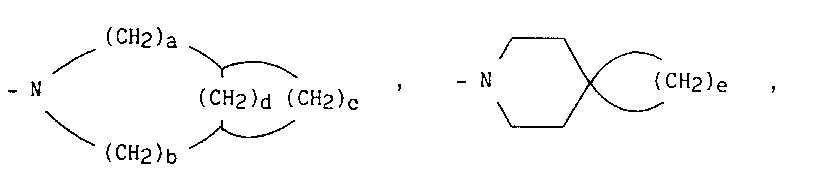 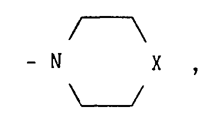
- X représente un oxygène ou un soufre,
- m, nombre entier, peut prendre les valeurs 0, 1, 2, 3, 4 ou 5,
- n, nombre entier, peut prendre les valeurs 1 ou 2,
- a et b, nombres entiers, peuvent prendre les valeurs 0, 1 ou 2 avec a + b ≠0,
- c et d, nombres entiers, peuvent prendre les valeurs 0, 1, 2, 3 ou 4 avec c + d ≠0,
- e, nombre entier peut prendre les valeurs 4 ou 5,
- R₄ représente :
   - un hydrogène,
   - un alkyle linéaire ou ramifié de 1 à 6 atomes de carbone,
   - un aryle éventuellement substitué,
   - un aralkyle éventuellement substitué dont la chaine alkyle comprend de 1 à 3 atomes de carbone,
   - un groupement 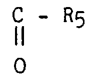 dans lequel R₅ représente un alkyle linéaire ou ramifié de 1 à 6 atomes de carbone, un phényle ou naphtyle éventuellement substitué,
- R₃ représente :
   - un groupement 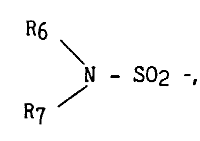
   - un groupement 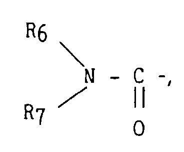
- R₆ et R₇, identiques ou différents ont la même définition que R₁ et R₂,
- leurs isomères, énantiomères, diastéréoisomères isolés ou sous forme de mélange,
- leurs sels d'addition à un acide minéral ou organique pharmaceutiquement acceptable,
- par groupement aryle on entend groupement, phényle, naphtyle, pyrimidyle, pyridyle,
- les expressions aryle éventuellement substitué et aralkyle éventuellement substitué signifient que le, ou les, noyaux aromatiques peuvent être substitués par un ou plusieurs hydroxy, halogène, trifluorométhyle, nitro, alkyle linéaire ou ramifié de 1 à 6 atomes de carbone ou alcoxy linéaire ou ramifié de 1 à 6 atomes de carbone.

La présente invention s'étend également au procédé de préparation des composés de formule générale (I) caractérisé en ce que l'on part d'un composé naphtalénique de formule (II) : 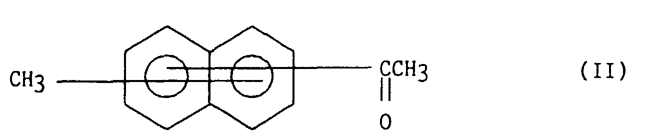 que l'on traite par du N-bromosuccinimide de manière à obtenir le composé bromé de formule (III) : 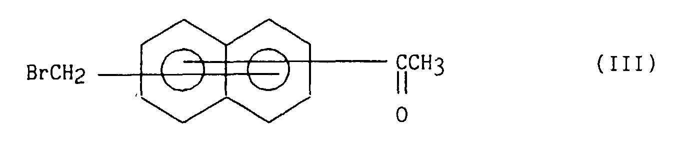 que l'on peut :
1) soit traiter par du sulfite de sodium dans un mélange acétone-eau de manière à obtenir le méthanesulfonate de formule (IV) : 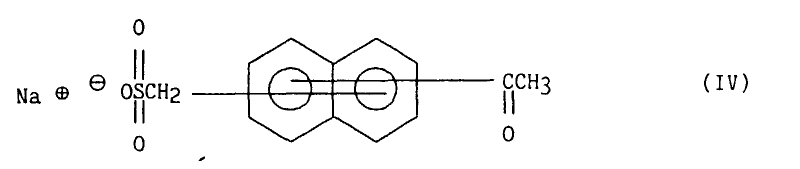 que l'on traite ensuite avec de l'oxychlorure de phosphore de manière à obtenir le chlorure de sulfonyle de formule (V) : 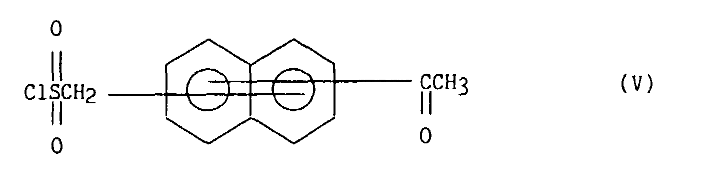 que l'on fait réagir ensuite avec une amine de formule (VI) : 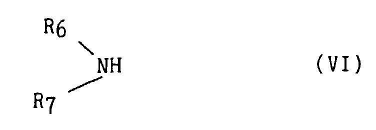 dans laquelle R₆ et R₇ ont la même signification que dans les composés de formule générale (I), de manière à obtenir le composé sulfonamidique de formule (VII) : 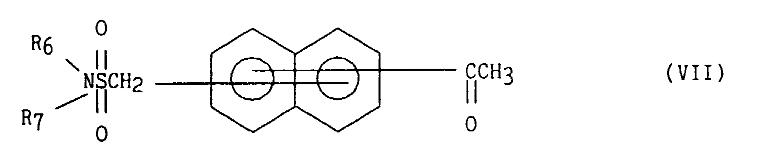 dans laquelle R₆ et R₇ ont la même signification que dans les composés de formule générale (I), que l'on traite ensuite par du brome de manière à obtenir le composé bromé de formule (VIII) : 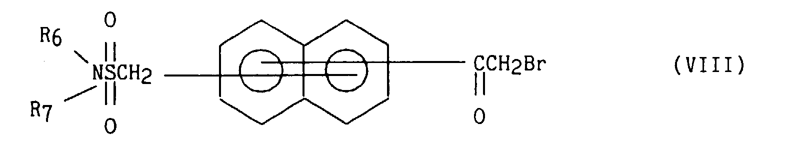 dans laquelle R₆ et R₇ ont la même signification que dans les composés de formule générale (I), que l'on réduit ensuite par du triéthylsilane dans l'acide trifluoroacétique de manière à obtenir le composé de formule (IX) : 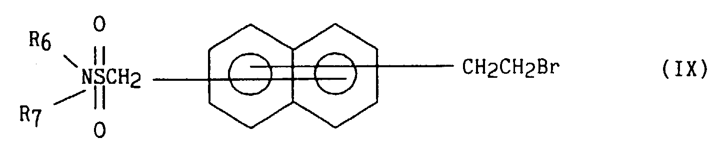 dans laquelle R₆ et R₇ ont la même signification que dans les composés de formule générale (I), que l'on fait réagir :
   - soit avec une amine de formule (X) : 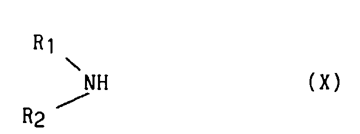 dans laquelle R₁ et R₂ ont la même signification que dans les composés de formule générale (I), de manière à obtenir le composé de formule (I_{A1}) : 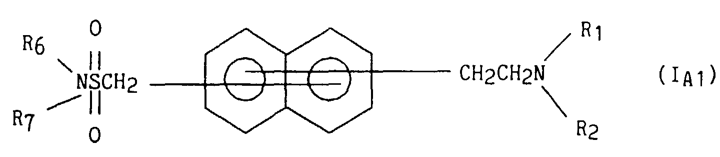 dans laquelle R₁, R₂, R₆ et R₇ ont la même signification que dans les composés de formule générale (I),
   - soit avec du phtalimide potassique dans la DMF pour obtenir le composé de formule (XVII_{A}) : 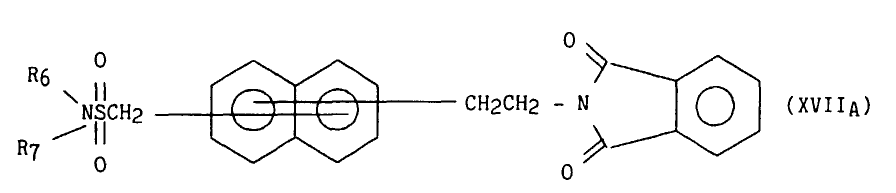 dans laquelle R₆ et R₇ ont la même signification que dans les composés de formule générale (I), que l'on fait réagir en milieu alcoolique avec de l'hydrate d'hydrazine pour obtenir l'amine primaire de formule (IA₂) 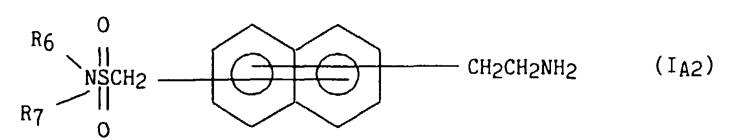 dans laquelle R₆ et R₇ ont la même signification que dans les composés de formule générale (I),
2) soit faire réagir avec un cyanure alcalin dans le DMSO ou en milieu alcoolique, de manière à obtenir le composé cyanométhylé de formule (XI) 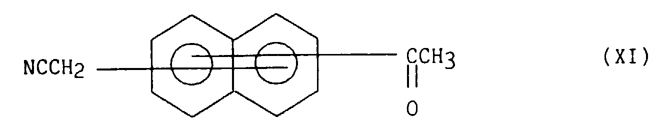 que l'on hydrolyse ensuite, soit en milieu hydroalcoolique avec un hydroxyde de métal alcalin, soit en milieu acide, de manière à obtenir l'acide naphtylacétique de formule (XII) : 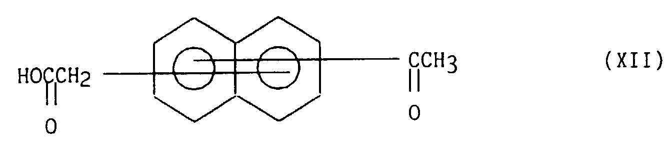 que l'on traite ensuite de manière classique par du chlorure de thionyle, de manière à obtenir le chlorure d'acide de formule (XIII) : 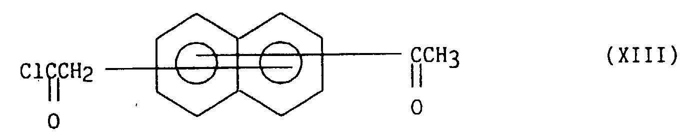 que l'on fait réagir ensuite avec une amine de formule (VI) : 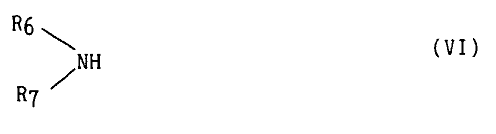 dans laquelle R₆ et R₇ ont la même signification que dans les composés de formule générale (I), de manière à obtenir le composé amidique de formule (XIV) : 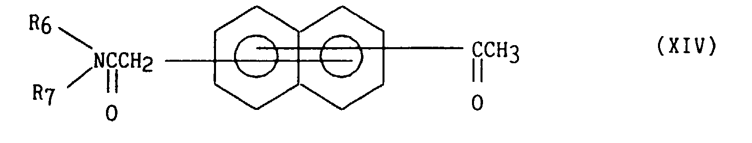 dans laquelle R₆ et R₇ ont la même signification que dans les composés de formule générale (I), que l'on traite ensuite par du brome de manière à obtenir le composé bromé de formule (XV) : 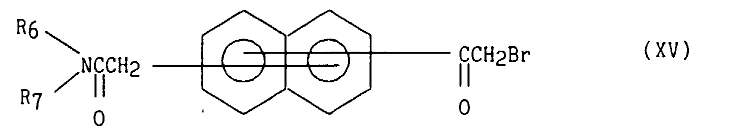 dans laquelle R₆ et R₇ ont la même signification que dans les composés de formule générale (I), que l'on réduit ensuite par du triéthylsilane dans l'acide trifluoroacétique de manière à obtenir le composé de formule (XVI) : 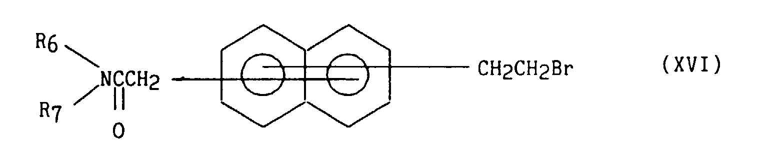 dans laquelle R₆ et R₇ ont la même signification que dans les composés de formule générale (I), que l'on fait réagir :
   - soit avec une amine de formule (X) : 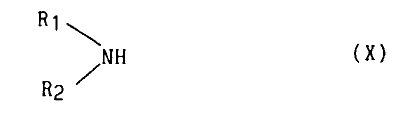 dans laquelle R₁ et R₂ ont la même signification que dans les composés de formule générale (I), de manière à obtenir le composé de formule (I_{B1}) : 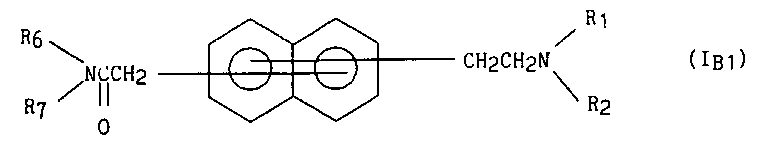 dans laquelle R₁, R₂, R₆ et R₇ ont la même signification que dans les composés de formule générale (I),
   - soit avec du phtalimide potassique dans la DMF pour obtenir le composé de formule (XVII_{B}) : 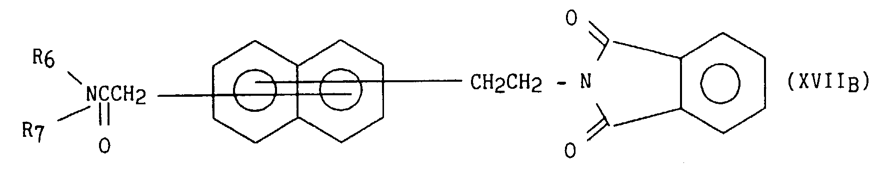 dans laquelle R₆ et R₇ ont la même signification que dans les composés de formule générale (I), que l'on fait réagir en milieu alcoolique avec de l'hydrate d'hydrazine pour obtenir l'amine primaire de formule (I_{B2}) 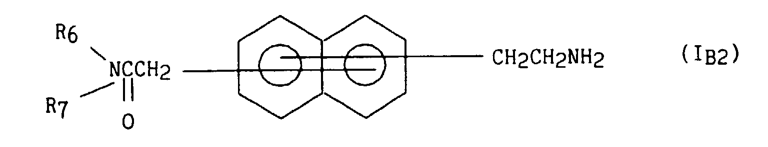 dans laquelle R₆ et R₇ ont la même signification que dans les composés de formule générale (I),
   étant entendu que les composés de formules générales (I_{A1}), (I_{A2}), (I_{B1}) et (I_{B2}) représentent l'ensemble des composés de formule (I) et peuvent, si on le désire, être salifiés par un acide pharmaceutiquement acceptable et si nécessaire être séparés en leurs différents isomères.

Les composés de formule générale (I) possèdent d'intéressantes propriétés pharmacologiques.

Les études de détermination d'affinité ont montré que les composés de l'invention se comportent comme de puissants ligands des récepteurs 5 HT₁ et plus particulièrement 5 HT₁D. Cette affinité se double d'une haute sélectivité par rapport aux autres récepteurs tels que les récepteurs α adrénergiques, dopaminergiques, gabaergiques, histaminergiques.

Les composés de formule générale (I) sont peu toxiques et possèdent de bonnes propriétés vasoconstrictrices qui les rendent précieux dans le traitement de troubles dus à une vasodilatation du système vasculaire, et donc en particulier des migraines, des céphalées et des algies vasculaires de la face, ainsi que des troubles de la circulation artérielle et veineuse.

La présente invention a également pour objet les compositions pharmaceutiques renfermant comme principe actif au moins un composé de formule générale (I), ou un de ses sels d'addition à un acide pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, paquets, les gélules, glossettes, tablettes, suppositoires, crèmes, pommades, gels dermiques etc...

La posologie varie selon l'âge et le poids du patient, la nature et la sévérité de l'affection ainsi que la voie d'administration. Celle-ci peut être orale, nasale, cutanée, rectale ou parentérale.

D'une manière générale, la posologie unitaire s'échelonne entre 0,05 et 50 mg de une à trois fois par jour.

Les exemples suivants illustrent l'invention et ne la limitent en aucune manière.

### EXEMPLE 1 : 2-{1-[2-(DIMETHYLAMINO)ETHYL]NAPHT-7-YL} N-METHYL METHANESULFONAMIDE

### STADE I : 1-ACETYL 7-METHYL NAPHTALENE ET 6-ACETYL 2-METHYL NAPHTALENE

Charger 940 g (7,04 moles) de chlorure d'aluminium et 2 litres de chloroforme dans un réacteur sous atmosphère d'argon. Agiter la suspension puis ajouter 552 g (7,04 moles) de chlorure d'acétyle puis une solution de 1 Kg (7,04 moles) de 2-méthyl naphtalène dans 3,3 litres de chloroforme. Agiter ensuite le milieu réactionnel pendant 1 heure à température ambiante, puis l'hydrolyser en le versant sur un mélange eau glace. Extraire au chlorure de méthylène puis mettre à sec sous pression partielle.

L'huile brute obtenue est distillée sous vide.

On obtient le 1-acétyl 7-méthyl naphtalène avec un rendement de 13 % Eb 0,06 mm Hg : 114-115° C

Parallelement, on isole le 6-acétyl 2-méthyl naphtalène avec un rendement de 28 % Eb 0,06 mm Hg : 118-120°C

### STADE II : 1-ACETYL 7-BROMOMETHYL NAPHTALENE

Charger 100 g (0,54 mole) de 1-acétyl 7-méthyl naphtalène, 97 g (0,54 mole) de N-bromosuccinimide, 14 g de peroxyde de benzoyle et 1,4 litre de tétrachlorure de carbone dans un réacteur sous atmosphère d'azote.

Chauffer une heure au reflux puis refroidir et éliminer l'insoluble par filtration.

Laver la phase organique avec de l'eau puis la mettre à sec.

Le produit brut, obtenu avec un rendement quasi quantitatif, est ensuite purifié par deux chromatographies sur colonne de silice.
Eluant de la première colonne Heptane 80 % acétate d'éthyle 20 %
Eluant de la seconde colonne Toluène 70 % cyclohexane 30 %
**Point de fusion** : 68° C
**Infrarouge** (pastille KBr)
1660 cm-1 νC = 0
1360 cm⁻¹ 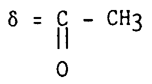 840, 760 et 725 cm-1 δCH
**RMN** (CDCl₃, TMS) :

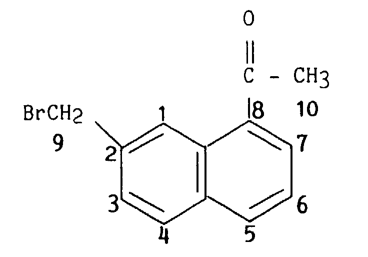

| | | | | | | | | |
|---|---|---|---|---|---|---|---|---|
| N° proton | 1 | 3 | 4 | 5 | 6 | 7 | 9 | 10 |
| δ(ppm) | 8,79 | 7,55 | 7,83 | 7,94 | 7,48 | 7,94 | 4,65 | 2,72 |

### STADE III : 2-(1-ACETYL NAPHT-7-YL) METHANESULFONATE DE SODIUM

Dissoudre 20 g (0,076 mole) de 1-acétyl 7-bromométhyl naphtalène dans 70 cm³ d'acétone. Ajouter ensuite sous agitation une solution de 9,57 g (0,076 mole) de sulfite de sodium dans 130 cm³ d'eau .

Chauffer le milieu réactionnel à reflux pendant deux heures puis refroidir, filtrer et mettre à sec le filtrat sous pression partielle

Recristalliser le résidu solide obtenu dans de l'éthanol à 95°

On obtient ainsi 18,49 g (85 %) de 2-(1-acétyl napht-7-yl) méthane sulfonate de sodium.
**Point de fusion** : > 260° C

| **Microanalyse :** | | |
|---|---|---|
| calculé | C(54,53) | H(3,87) |
| mesuré | C(54,26) | H(3,99) |

**Infrarouge** (Pastille KBr)
3040 - 2920 cm-1 ν CH (alkyle)
1660 cm-1 ν C = O
1590 et 1560 cm-1 ν C = C
1192 - 1175 cm-1 ν SO₂
1065 - 1050 cm-1 ν SO₂
**RMN** (DMSO, d₆) δ (ppm) 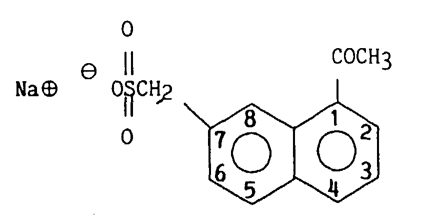 2,74 (s, 3H) CH₃
3,92 (s, 2H) CH₂
7,44 - 8,13 (massif, 5H) H₂, H₃, H₄, H₅, H₆
8,48 (1H) H₈

### STADE IV : CHLORURE DE L'ACIDE 2-(1-ACETYL NAPHT-7-YL) METHANESULFONIQUE

Charger dans un ballon sous atmosphère d'argon 11,44 g (0,04 mole) de 2-(1-acétyl napht-7-yl) méthanesulfonate de sodium, 14,6 cm3 (0,15 mole) d'oxychlorure de phosphore, 20 cm3 de sulfolane et 20 cm3 d'acétonitrile anhydre.

Chauffer le milieu réactionnel sous agitation pendant 40 minutes à 70° C.

Après refroidissement, hydrolyser le milieu réactionnel en le versant sur de la glace, isoler le précipité formé par filtration, laver à l'eau et sécher.

On obtient ainsi 9,61 g (85 %) de chlorure de l'acide 2-(1-acétyl napht-7-yl) méthanesulfonique.
**Point de fusion** :129-130° C
**Infrarouge** (Pastille KBr)
   3090 - 2600 cm-1 ν CH (alkyle)
   1660 cm-1 ν C = O
   1590 et 1560 cm-1 ν C = C (aromatique)
   1360 et 1155 cm-1 ν SO₂
**RMN** (DMSO, d₆) δ (ppm) 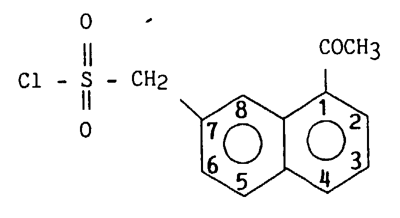 2,75 (s, 3H) CH₃
   5,06 (s, 2H) CH₂
   7,50 - 8,08 (massif, 5H) H₂, H₃, H₄, H₅, H₆
   9,00 (1H) H₈

### STADE V : 2-(1-ACETYL NAPHT-7-YL) N-METHYL METHANESULFONAMIDE

Dissoudre 10,5 g (0,037 mole) de chlorure de l'acide 2-(1-acétyl napht-7-yl) méthanesulfonique dans 200 cm3 d'acétate d'éthyle. Refroidir à 0° C et ajouter goutte à goutte 9 cm3 (0,11 mole) d'une solution aqueuse à 40 % de méthylamine.

Agiter 30 minutes à température ambiante, laver la phase organique avec de l'eau, la concentrer au tiers de son volume initial et isoler le précipité obtenu par filtration.

Le produit brut est recristallisé dans l'éthanol à 95°.

On obtient ainsi 7,9 g (77 %) de 2-(1-acétyl napht-7-yl) N-méthyl méthanesulfonamide.
**Point de fusion** :127 - 129° C

| **Microanalyse :** | | | |
|---|---|---|---|
| calculé | C(60,63) | H(5,45) | N(5,05) |
| mesuré | C(60,53) | H(5,15) | N(5,22) |

**Infrarouge** (Pastille KBr)
3240 cm-1 ν NH (sulfonamide)
3040 - 2920 cm-1 ν CH (alkyle)
1660 cm-1 ν C = O
1590 et 1560 cm-1 ν C = C (aromatique)
1310 cm-1 ν SO₂
**RMN** (DMSO, d₆) δ (ppm) 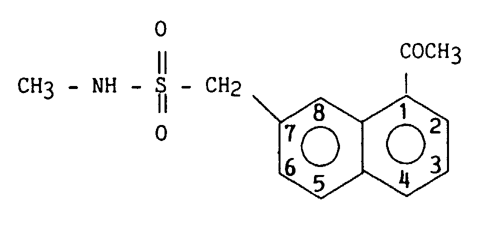 2,61 (3H) CH₃NH
2,75 (s, 3H) 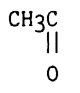 4,55 (s, 2H) CH₂SO₂
6,96 (m, 1H) NH (échangeable dans D₂O)
7,50 - 8,70 (massif, 5H) H₂, H₃, H₄, H₅, H₆
8,63 (1H) H₈

### STADE VI : 2-(1-BROMOACETYL NAPHT-7-YL) N-METHYL METHANESULFONAMIDE

Dissoudre à 60° C 6,3 g (0,022 mole) de 2-(1-acétyl napht-7-yl) N-méthyl méthanesulfonamide dans 100 cm3 d'acide acétique.

Ajouter goutte à goutte 1,17 cm3 (0,022 mole) de brome dans 80 cm3 d'acide acétique.

Une fois l'addition terminée poursuivre l'agitation pendant 2 heures à 60°C

Après refroidissement, verser le milieu réactionnel dans 150 cm3 d'eau, isoler le précipité formé par filtration, laver avec un minimum d'acétonitrile et recristalliser dans l'acétonitrile.

On obtient 6,58 g (84 %) de 2-(1-bromoacétyl napht-7-yl) N-méthyl méthanesulfonamide.
**Point de fusion** :159 - 161° C

| **Microanalyse :** | | | |
|---|---|---|---|
| calculé | C(47,19) | H(3,96) | N(3,93) |
| mesuré | C(47,16) | H(3,85) | N(3,90) |

**Infrarouge** (Pastille KBr)
3260 cm-1 ν NH
1675 - 1655 cm-1 ν C = O
1590 et 1565 cm-1 ν C = C (aromatique)
**RMN** (DMSO, d₆) δ (ppm) 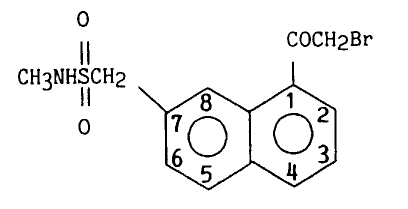 2,61 (d, 3H) CH₃NH (J CH₃NH = 4,6 Hz, pas de couplage dans D₂O)
4,50 (s, 2H) CH₂SO₂
5,07 (s, 2H) CH₂Br
6,96 (q, 1H) NH (JNHCH₃ = 4,6 Hz, pas de couplage dans D₂O)
8,00 - 8,80 (massif, 5H) H₂,H₃, H₄, H₅, H₆
8,50 (1H) H₈

### STADE VII : 2-[1-(2-BROMOETHYL)NAPTH-7-YL] N-METHYL METHANESULFONAMIDE

Dissoudre sous atmosphère d'argon 10,4 g (0,029 mole) de 2-(1-bromoacétyl napth-7-yl) N-méthyl méthanesulfonamide dans 16 cm3 d'acide trifluoroacétique puis ajouter goutte à goutte 10,26 cm3 (0,064 mole) de triéthylsilane.

Agiter 72 heures à température ambiante puis verser le milieu réactionnel dans 360 cm3 d'eau glacée.

Isoler le précipité formé par filtration, le laver à l'eau puis,après séchage, le recristalliser dans l'éthanol à 95 °

On obtient ainsi 7,94 g (80 %) de 2-[1-(2-bromoéthyl)napth-7-yl] N-méthyl méthanesulfonamide
**Point de fusion** :86 - 88° C

| **Microanalyse :** | | | |
|---|---|---|---|
| calculé | C(49,12) | H(4,71) | N(4,09) |
| mesuré | C(49,23) | H(4,69) | N(3,97) |

**Infrarouge** (Pastille KBr)
3270 cm-1 ν NH
1590 cm-1 ν C = C (aromatique)
1310 cm-1 ν SO₂ (sulfonamide)
**RMN** (DMSO, d₆) δ (ppm) 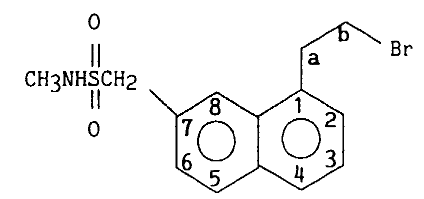 2,61 (d, 3H) CH₃NH (J CH₃NH = 5,7 Hz, pas de couplage dans D₂O) 3,50 - 4,00 (massif, 4H) CH₂a et CH₂b
4,57 (s, 2H) CH₂SO₂
7,00 (q, 1H) NHCH₃ (JNHCH₃ = 5,7 Hz, pas de couplage dans D₂O) 7,50 - 8,08 (massif, 6H) H₂,H₃, H₄, H₅, H₆, H₈

### STADE VIII : 2-{1-[2-(DIMETHYLAMINO)ETHYL]NAPHT-7-YL} N-METHYL METHANESULFONAMIDE

Introduire dans un autoclave une solution de 2-[1-(2-bromoéthyl)napht-7-yl] N-méthyl méthanesulfonamide dans 30 cm3 d'éthanol à 95°.

Ajouter ensuite 7,4 cm3 (0,096 mole) d'une solution aqueuse à 40 % de diméthylamine et chauffer 8 heures à 80° C.

Après refroidissement mettre à sec, reprendre le résidu par 30 cm3 d'eau, acidifier, laver la phase aqueuse avec de l'éther, alcaliniser, isoler le précipité formé par filtration, le laver à l'eau, puis, après séchage, le recristalliser dans de l'éthanol absolu.

On obtient ainsi 1,18 g (40 %) de 2-{1-[2-(diméthylamino)éthyl]napht-7-yl} N-méthyl méthanesulfonamide
**Point de fusion** : 149 - 150° C

| **Microanalyse :** | | | |
|---|---|---|---|
| calculé | C(62,71) | H(7,23) | N(9,14) |
| mesuré | C(62,89) | H(7,33) | N(9,12) |

**Infrarouge** (Pastille KBr)
3080 - 3040 cm-1 ν NH
2970 - 2760 cm-1 ν CH
1590 cm-1 ν C = C (aromatique)
1310 cm-1 ν SO₂
**RMN** (DMSO, d₆) δ (ppm) 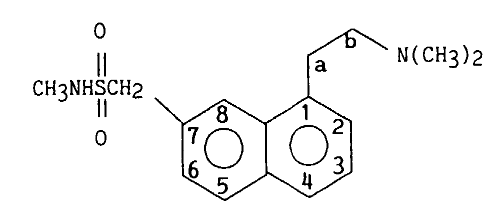 2,26 (d, 6H) N(CH₃)₂
2,35 - 2,50 (m, 2H) CH₂b
2,60 (d, 3H) CH₃NH (JCH₃NH = 4,2 Hz, pas de couplage dans D₂O)
3,10 (m, 2H) CH2a
4,54 (s, 2H) CH₂SO₂
6,94 (q, 1H) NHCH₃ (JNHCH₃ = 4,2 Hz, pas de couplage dans D₂O)
7,38 - 8,05 (massif, 6H) H₂, H₃, H₄, H₅, H₆, H₈

### EXEMPLE 2 : 2-[1-(2-AMINOETHYL)NAPHT-7-YL] N-METHYL METHANESULFONAMIDE

**STADE I :** 2-[1-(2-PHTALIMIDOETHYL) NAPHT-7-YL] N-METHYL METHANE SULFONAMIDE
Dissoudre 3 g (8,7 mmoles) de 2-[1-(2-bromoéthyl)napht-7-yl] N-méthyl méthanesulfonamide (Exemple 1 stade VII) dans 20 cm3 de diméthylformanide puis additionner 1,61 g (8,7 mmoles) de phtalimide potassique et chauffer à reflux pendant 48 heures.
Après refroidissement, verser le milieu réactionnel dans 150 cm3 d'eau et isoler le produit qui précipite par filtration puis, après séchage, le recristalliser dans la diméthylformamide.
On obtient ainsi 1,77 g (50 %) de 2-[1-(2-phtalimidoéthyl) napht-7-yl] N-méthyl méthanesulfonamide.
**Point de fusion** : 248 - 251° C

| **Microanalyse :** | | | |
|---|---|---|---|
| calculé | C(64,68) | H(4,93) | N(6,85) |
| mesuré | C(64,33) | H(5,01) | N(6,99) |

**Infrarouge** (Pastille KBr)
3310 cm-1 ν NH
3000 - 2900 cm-1 ν CH
1760 - 1700 cm-1 ν C = O (phtalimide)
**RMN** (DMSO, d₆) δ (ppm) 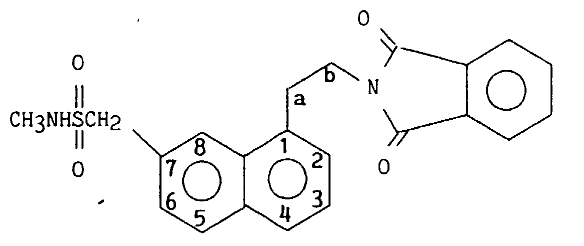 2,60 (d, 3H) CH₃NH (JCH₃NH = 4,2 Hz, pas de couplage dans D₂O) 4,00 (m, 4H) CH₂a, CH₂b
4,54 (s, 2H) CH₂SO₂
7,00 (q, 1H) NHCH₃ (JNHCH₃ = 4,2 Hz, pas de couplage dans D₂O) 8,00 - 7,32 (massif 9H) H₂, H₃, H₄, H₅, H₆ + phtalimide
8,30 (signal, 1H) H₈
**STADE II :** 2-[1-(2-AMINOETHYL) NAPHT-7-YL] N-METHYL METHANE SULFONAMIDE
Mettre en suspension 3 g (7,3 mmoles) de 2-[1-( 2-phtalimido éthyl)napht-7-yl] N-méthyl méthanesulfonamide dans 80 cm3 d'alcool à 95° puis chauffer à reflux et additionner goutte à goutte 8 cm3 d'hydrate d'hydrazine à 98 % jusqu'à dissolution totale de la matière première.
Poursuivre le chauffage pendant deux heures puis, après refroidissemnt , éliminer par filtration le précipité de phtalhydrazide.
Mettre à sec les jus de filtration, reprendre le résidu par 50 cm3 d'acool absolu puis faire barbotter de l'acide chlorhydrique jusqu'à précipitation du chlorhydrate de l'amine qui est isolé par filtration, séché puis recristallisé dans l'éthanol absolu.
On obtient ainsi 0,92 g (40 %) de chlorhydrate de 2-[1-(2-aminoéthyl) napht-7-yl] N-méthyl méthane sulfonamide
**Point de fusion** : 233 - 237° C

| **Microanalyse :** | | | |
|---|---|---|---|
| calculé | C(53,40) | H(6,08) | N(8,89) |
| mesuré | C(54,14) | H(6,20) | N(8,86) |

**Infrarouge** (Pastille KBr)
3300 - 2500 cm-1 ν NH⁺ (chlorhydrate)
1590 cm-1 ν C = C (aromatique)
1310 cm-1 ν SO₂
**RMN** (DMSO, d₆) δ (ppm) 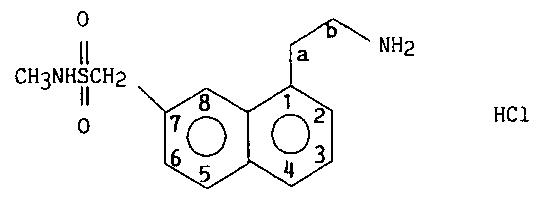 2,63 (d, 3H) CH₃NH (JCH₃NH = 4,61 Hz, pas de couplage dans D₂O) 3,25 (m, 4H) CH₂a, CH₂b
4,57 (s, 2H) CH₂SO₂
7,00 (q, 1H) NHCH₃ (JNHCH₃ = 4,61 Hz, pas de couplage dans D₂O) 7,46 - 8,00 (massif 6H) H₂, H₃, H₄, H₅, H₆, H₈
8,25 (signal, 2H) NH⁺ (disparait dans D₂O)

### EXEMPLE 3 : CHLORHYDRATE DE 2-[1-(2-MORPHOLINOETHYL)NAPHT-7-YL] N-METHYL METHANESULFONAMIDE

Dissoudre 3,42 g (0,01 mole) de 2-[1-(2-bromoéthyl)napht-7-yl] N-méthyl méthanesulfonamide dans 40 cm3 d'acétone.

Ajouter goutte à goutte 1,74 g (0,02 mole) de morpholine et chauffer 24 heures à reflux.

Après refroidissement, mettre à sec le milieu réactionnel, reprendre le résidu par de l'eau, acidifier, laver avec de l'acétate d'éthyle, alcaliniser et extraire avec de l'éther.

Laver la phase éthérée avec de l'eau, sécher puis faire barbotter un courant d'acide chlorhydrique gazeux.

Isoler par filtration le précipité formé et le recristalliser dans du méthanol.

On obtient ainsi 1,31 g (34 %) de chlorhydrate de 2-[1-(2-morpholinoéthyl)napth-7-yl] N-méthyl méthanesulfonamide.
**Point de fusion** : 216 - 218° C

| **Microanalyse :** | | | |
|---|---|---|---|
| calculé | C(56,16) | H(6,54) | N(7,27) |
| mesuré | C(56,01) | H(6,62) | N(7,36) |

**Infrarouge** (Pastille KBr)
3010 cm-1 ν NH (sulfonamide)
2900 cm-1 ν CH (alkyle)
2800 - 2520 cm-1 ν NH (amine)
1590 cm-1 ν C = C (aromatique)
**RMN** (DMSO, d₆) δ (ppm) 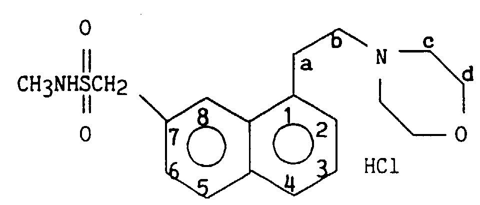 2,64 (d, 3H) CH₃NH (JCH₃NH = 4,3 Hz, pas de couplage dans D₂O) 3,18 - 4,00 (massif, 12H) CH₂a, CH₂b, CH₂c, CH₂d
4,59 (s, 2H) CH₂SO₂
7,00 (q, 1H) NHCH₃ (JNHCH₃ = 4,3 Hz, pas de couplage dans D₂O) 7,50 - 8,00 (massif, 5H) H₂, H₃, H₄, H₅, H₆
8,40 (1H) H₈
11,89 (1H) NH⁺ (échangeable dans D₂O)

### EXEMPLE 4 : 2-{1-[2-(3-AZABICYCLO[3,3,0]OCT-3-YL)ETHYL]NAPHT-7-YL} N-METHYL METHANESULFONAMIDE

En procédant comme pour l'exemple 3 mais en remplaçant la morpholine par le 3-azabicyclo [3,3,0] octane on obtient le 2-{1-[2-(3-azabicyclo [3,3,0] oct-3-yl) éthyl]napthy-7-yl} N-méthyl méthanesulfonamide avec un rendement de 32 %
**Point de fusion** : 130 - 132° C

| **Microanalyse :** | | | |
|---|---|---|---|
| calculé | C(67,70) | H(7,57) | N(7,52) |
| mesuré | C(67,78) | H(7,69) | N(7,38) |

**Infrarouge** (Pastille KBr)
3040 - 3060 cm-1 ν NH (sulfonamide)
2780 - 2940 cm-1 ν CH
1320 cm-1 ν SO₂
**RMN** (DMSO, d₆) δ (ppm) 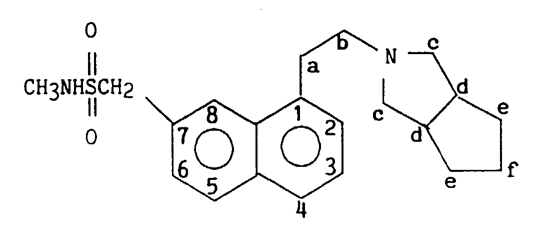 1,50 (m, 6H) CH₂e, CH₂f
2,09 - 2,75 (massif, 11H) CH₂b, CH₂c, CH₂d et CH₃N
3,32 (m, 2H) CH₂a
4,53 (s, 2H) CH₂SO₂
7,00 (m, 1H) NHCH₃ (échangeable dans D₂O)
7,38 - 8,09 (massif, 6H) H₂, H₃, H₄, H₅, H₆, H₈

### EXEMPLE 5 : CHLORHYDRATE DE 2-[1-(2-PIPERIDINOETHYL)NAPHT-7-YL] N-METHYL METHANESULFONAMIDE

En procédant comme pour l'exemple 3 mais en remplaçant la morpholine par la pipéridine on obtient le chlorhydrate de 2-[1-(2-pipéridinoéthyl)napht-7yl] N-méthyl methanesulfonamide
**Point de fusion** : 202 - 204° C

| **Microanalyse :** | | | |
|---|---|---|---|
| calculé | C(59,58) | H(7,10) | N(7,31) |
| mesuré | C(59,43) | H(7,34) | N(7,35) |

**Infrarouge** (Pastille KBr)
3000 - 3020 cm-1 ν NH (sulfonamide)
2900 cm-1 ν CH (alkyle)
2500 - 2800 cm-1 ν NH (chlorhydrate)
1595 cm-1 ν C = C (aromatique)
**RMN** (DMSO, d₆) δ (ppm) 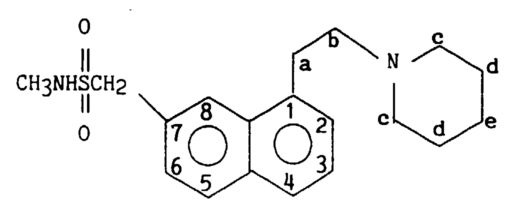 1,83 (massif, 6H) CH₂d, CH₂e
2,67 (d, 3H) CH₃NH (JCH₃NH = 4,3 Hz, pas de couplage dans D₂O)
3,00 - 3,72 (massif, 8H) CH₂a, CH₂b, CH₂c
4,59 (s, 2H) CH₂SO₂
7,00 (q, 1H) NHCH₃ (JNHCH₃ = 4,3 Hz, pas de couplage dans D₂O)
7,50 - 8,00 (massif, 5H) H₂, H₃, H₄, H₅, H₆
8,37 (1H) H₈
11,00 (1H) NH⁺ (échangeable dans D₂O)

### EXEMPLE 6 : CHLORHYDRATE DE 2-{1-[2-(4-METATRIFLUOROMETHYLPHENYL PIPERAZIN-1-YL) ETHYL] NAPHT-7-YL} N-METHYL METHANESULFONAMIDE

Ajouter 2,02 g (0,007 mole) de chlorhydrate de métatrifluorométhyl phényl pipérazine, puis 2 cm3 (0,014 mole) de triéthylamine à une solution de 2,5 g (0,007 mole) de 2-[1-(2-bromoéthyl) napht-7-yl] N-méthyl méthanesulfonamide dans 40 cm3 d'acétone.

Chauffer à reflux pendant 48 heures puis mettre à sec, reprendre le résidu par de l'eau, acidifier, laver avec de l'acétate d'éthyle, alcaliniser, extraire avec de l'acétate d'éthyle puis mettre à sec après séchage.

Reprendre le résidu dans de l'éthanol absolu, faire barbotter de l'acide chlorhydrique gazeux, isoler le chlorhydrate par filtration et le recristalliser dans l'acétonitrile.

On obtient ainsi 0,81 g (22 %) de chlorhydrate de 2-{1-[2-(4-métatrifluorométhylphényl pipérazin-1-yl) éthyl] napth-7-yl} N-méthyl méthanesulfonamide.
**Point de fusion** : 234 - 236° C

| **Microanalyse :** | | | |
|---|---|---|---|
| calculé | C(56,86) | H(5,53) | N(7,95) |
| mesuré | C(56,84) | H(5,10) | N(7,96) |

**Infrarouge** (Pastille KBr)
3090 cm-1 ν NH (sulfonamide)
2929 - 2820 cm-1 ν CH (alkyle)
2680 - 2400 cm-1 ν NH (chlorhydrate)
1610 - 1590 cm-1 ν C = C (aromatique)
1320 cm-1 v SO₂
**RMN** (DMSO, d₆) δ (ppm) 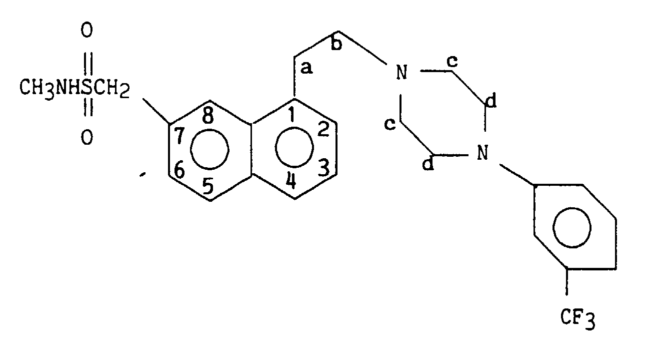 2,64 (d, 3H) CH₃NH (JCH₃NH = 4,3 Hz, pas de couplage dans D₂O) 3,24 - 4,10 (massif, 12H) CH₂a, CH₂b, CH₂c, CH₂d
4,59 (s, 2H) CH₂SO₂
7,00 (q, 1H) NHCH₃ (JNHCH₃ = 4,3 Hz, pas de couplage dans D₂O) 7,13 - 8,05 (massif, 9H) H₂, H₃, H₄, H₅, H₆, protons aromatiques 8,37 (1H) H₈
11,5 (m, 1H) NH⁺ (échangeable dans D₂O)

### EXEMPLES 7 à 13

En procédant comme dans l'exemple 6 mais en remplaçant la 1-(métatrifluorométhylphényl) pipérazine par :
- la 1-(napht-1-yl) pipérazine, on obtient :
   - **EXEMPLE 7 :**: **CHLORHYDRATE DE 2-{1-[2-(4-(NAPHT-1-YL) PIPERAZIN-1-YL) ETHYL] NAPHT-7-YL} N-METHYL METHANESULFONAMIDE**
- la 1-(pyrid-2-yl) pipérazine, on obtient :
   - **EXEMPLE 8 :**: **CHLORHYDRATE DE 2-{1-[2-(4-(PYRID-2-YL) PIPERAZIN-1-YL) ETHYL] NAPHT-7-YL} N-METHYL METHANESULFONAMIDE**
- la 1-(pyrimid-2-yl) pipérazine, on obtient :
   - **EXEMPLE 9 :**: **CHLORHYDRATE DE 2-{1-[2-(4-(PYRIMID-2-YL) PIPERAZIN-1-YL) ETHYL] NAPHT-7-YL} N-METHYL METHANESULFONAMIDE**
- la 1-benzyl pipérazine, on obtient :
   - **EXEMPLE 10 :**: **CHLORHYDRATE DE 2-{1-[2-(4-BENZYL PIPERAZIN-1-YL) ETHYL] NAPHT-7-YL} N-METHYL METHANESULFONAMIDE**
- la 1-méthyl pipérazine, on obtient :
   - **EXEMPLE 11 :**: **CHLORHYDRATE DE 2-{1-[2-(4-METHYL PIPERAZIN-1-YL) ETHYL] NAPHT-7-YL} N-METHYL METHANESULFONAMIDE**
- la 3-azaspiro [5,5] undécane, on obtient :
   - **EXEMPLE 12 :**: **CHLORHYDRATE DE 2-{1-[2-(3-AZASPIRO [5,5] UNDECAN-3-YL) ETHYL] NAPHT-7-YL} N-METHYL METHANESULFONAMIDE**
- le 3-azabicyclo [3,2,2] nonane, on obtient :
   - **EXEMPLE 13 :**: **CHLORHYDRATE DE 2-{1-[2-(3-AZABICYCLO [3,2,2] NONAN-3-YL) ETHYL]NAPHT-7-YL} N-METHYL METHANESULFONAMIDE**

### EXEMPLES 14 A 21

en procédant comme dans l'exemple 3 mais en remplaçant la morpholine par :
- la pyrrolidine, on obtient :
   - **EXEMPLE 14 :**: **CHLORHYDRATE DE 2-[1-(2-PYRROLIDINO ETHYL)NAPHT-7-YL] N**-**METHYL METHANESULFONAMIDE**
- la thiomorpholine, on obtient :
   - **EXEMPLE 15 :**: **CHLORHYDRATE DE 2-[1-(2-THIOMORPHOLINO ETHYL)NAPHT-7-YL] N-METHYL METHANESULFONAMIDE**
- l'hexaméthylèneimine, on obtient :
   - **EXEMPLE 16 :**: **CHLORHYDRATE DE 2-[1-(2-HEXAMETHYLENEIMINO ETHYL)NAPHT-7-YL] N-METHYL METHANESULFONAMIDE**
- l'heptaméthyléneimine, on obtient :
   - **EXEMPLE 17 :**: **CHLORHYDRATE DE 2-[1-(2-HEPTAMETHYLENEIMINO ETHYL)NAPHT-7-YL] N-METHYL METHANESULFONAMIDE**
- la di N-propyl amine, on obtient :
   - **EXEMPLE 18 :**: **CHLORHYDRATE DE 2-{1-[2-(N,N-DIPROPYLAMINO) ETHYL]NAPHT-7-YL} N-METHYL METHANESULFONAMIDE**
**Point de fusion** : 177 - 180° C
- la N-benzyl méthylamine, on obtient :
   - **EXEMPLE 19 :**: **CHLORHYDRATE DE 2-{1-[2-(N-BENZYL N-METHYLAMINO) ETHYL] NAPHT-7-YL} N-METHYL METHANESULFONAMIDE**
**Point de fusion** : 187 - 189° C
- la N-méthyl aniline, on obtient :
   - **EXEMPLE 20 :**: **CHLORHYDRATE DE 2-{1-[2-(N-METHYL N-PHENYLAMINO) ETHYL]NAPHT-7-YL} N-METHYL METHANESULFONAMIDE**
- la cyclohexylamine on obtient :
   - **EXEMPLE 21 :**: **CHLORHYDRATE DE 2-{1-[2-(N-CYCLOHEXYLAMINO) ETHYL]NAPHT-7-YL} N-METHYL METHANESULFONAMIDE**

### EXEMPLES 22 A 23

En procédant comme dans l'exemple 1 stade VIII mais en remplaçant la diméthylamine par :
- la méthylamine en solution aqueuse à 40 % , on obtient
   - **EXEMPLE 22 :**: **2-{1-[2-(N-METHYLAMINO)ETHYL]NAPHT-7-YL} N-METHYL METHANESULFONAMIDE**
**Point de fusion** : 145 - 148° C
- la N-méthyl éthylamine, on obtient :
   - **EXEMPLE 23 :**: **2-{1-[2-(N-METHYL N-ETHYL)AMINO ETHYL]NAPHT-7-YL} N-METHYL METHANESULFONAMIDE**
   - **EXEMPLE 24 :**: **2-{1-[2-(DIMETHYLAMINO)ETHYL]NAPHT-7-YL} N-BENZYLMETHANE SULFONAMIDE**

En procédant comme pour l'exemple 1 mais en remplaçant dans le stade VIII le 2-[1-(2-bromoéthyl)napht-7-yl] N-méthyl méthanesulfonamide par le 2-[1-(2-bromoéthyl)napht-7-yl] N-benzyl méthanesulfonamide on obtient le 2-{1-[2-(diméthylamino)éthyl] napht-7-yl} N-benzylméthane sulfonamide sous forme de chlorhydrate avec un rendement de 55 %
**Point de fusion** : 151 - 154° C (chlorhydrate)
**Infrarouge** (Pastille KBr)
   3120 - 3020 cm-1 ν NH (sulfonamide)
   2960 - 2800 cm-1 ν CH
   2700 - 2400 cm-1 ν NH⁺ (chlorhydrate)
   1590 cm-1 ν C = C (aromatique)
   1310 cm-1 ν SO₂ (sulfonamide)
**RMN** (DMSO, d₆) δ (ppm) 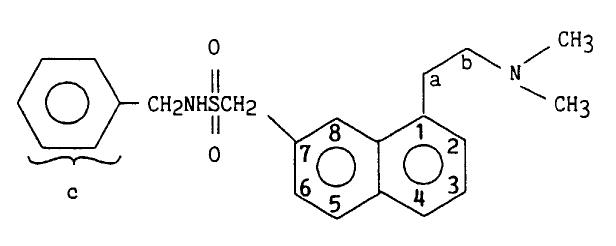 2,85 (s, 6H) N(CH₃)₂
   3,30 (m, 2H) Hb
   3,50 (m, 2H) Ha
   4,17 (d, 2H) CH₂NH
   4,60 (s, 2H) CH₂SO₂
   7,30 (massif, 5H) Hc
   7,50 - 8,00 (massif, 6H) H₂, H₃, H₄, H₅, H₆ et NHCH₃ (disparait dans D₂O)
   8,28 (signal, 1H) H₈

Le 2-[1-(2-bromoéthyl)napht-7-yl] N-benzyl méthanesulfonamide est préparé en deux étapes à partir du 2-(1-acétyl napht-7-yl) N-benzyl méthanesulfonamide comme dans l'exemple I.

2-(1-acétyl napht-7-yl) N-benzyl méthanesulfonamide
**Point de fusion** : 131° C
**Infrarouge** (Pastille KBr)
   3300 - 3220 cm-1 ν NH
   1660 - 1640 cm-1 ν C = O
**RMN** (DMSO, d₆) δ (ppm) 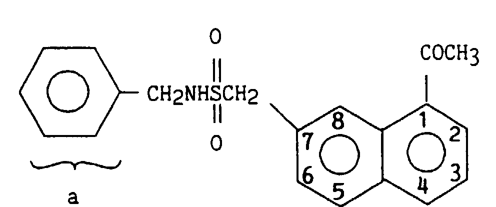 2,75 (s, 3H) CH₃
   4,25 (m, 3H) CH₂ - NH
   4,45 (s, 2H) CH₂SO₂
   7,3 - 8,06 (m, 10H) H₂, H₃, H₄, H₅, H₆ Ha
   8,7 (s, 1H) H₈

### EXEMPLE 25 : 1-[2-(DIMETHYLAMINO) ETHYL] 7-MORPHOLINOSULFONYLMETHYL NAPHTALENE

En procédant comme pour l'exemple 19 mais à partir de 1-acétyl 7-morpholinosulfonylméthyl naphtalène on obtient le 1-[2-(diméthylamino) éthyl] 7-morpholinosulfonylméthyl naphtalène avec un rendement de 35 %
**Point de fusion** : 114 - 117° C
**Infrarouge** (Pastille KBr)
   3960 - 2820 cm-1 ν CH alkyle
   1590 cm-1 ν C = C (aromatique)
   1340 - 1320 cm-1 ν SO₂ (sulfonamide)
**RMN** (CDCl₃) δ (ppm) 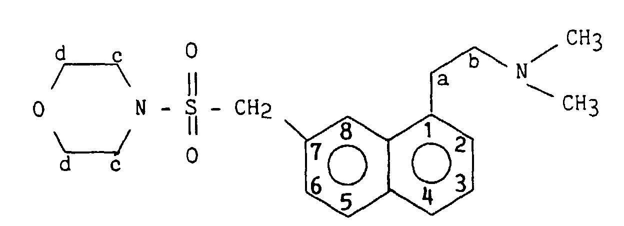 2,42 (s, 6H) -N(CH₃)₂
   2,74 (m, 2H) Hb
   3,14 (m, 2H) Hc
   3,29 (m, 2H) Ha
   3,59 (m, 4H) Hd
   4,46 (s, 2H) CH₂SO₂
   7,40 - 7,96 (massif, 5H) H₂, H₃, H₄, H₅, H₆
   8,13 (signal, 1H) H₈

### 1-acétyl 7-(morpholinosulfonylméthyl) naphtalène

**Point de fusion** : 162° C (acétate d'éthyle)
**Infrarouge** (Pastille KBr)
   3000 - 2800 cm-1 ν CH
   1670 - 1660 cm-1 ν C = O
**RMN** (CDCl₃) δ (ppm) 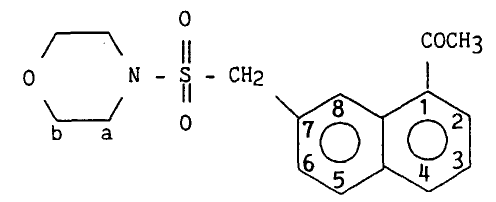 2,80 (s, 3H) CH₃
   3,15 (m, 4H) Ha
   3,60 (m, 4H) Hb
   4,45 (s, 2H) CH₂
   7,6 - 8,10 (m, 5H)
   8,90 (s, 1H)

### EXEMPLES 26 A 28

En procédant comme dans l'exemple 24 mais en remplaçant 2-[1-(2-bromoéthyl) napht-7-yl] N-benzyl méthanesulfonamide par :
- le 2-[1-(2-bromoéthyl)napht-7-yl] N,N-diméthylméthanesulfonamide, on obtient :
   - **EXEMPLE 26 :**: **2-{1-[2-(DIMETHYLAMINO) ETHYL]NAPHT-7-YL} N,N-DIMETHYL METHANESULFONAMIDE**
   **Point de fusion** : 110-113°C
   **Infrarouge** (Pastille KBr)
   2980 - 2720 cm⁻¹ ν CH alkyle
   1590 cm⁻¹ ν C=C (aromatique)
   1330 cm⁻¹ ν SO2 (sulfonamide)
- le 2-[1-(2-bromoéthyl) napht-7-yl] N-cyclopentyl méthanesulfonamide, on obtient :
   - **EXEMPLE 27 :**: **2-{1-[2-(DIMETHYLAMINO) ETHYL]NAPHT-7-YL} N-CYCLOPENTYL METHANESULFONAMIDE**
- le 2-[1-(2-bromoéthyl) napht-7-yl] N-tétraméthylène méthanesulfonamide, on obtient :
   - **EXEMPLE 28 :**: **2-{1-[2-(DIMETHYLAMINO) ETHYL]NAPHT-7-YL} N-TETRAMETHYLENE METHANESULFONAMIDE**
   - **EXEMPLE 29 :**: **2-{6-[2-(DIMETHYLAMINO) ETHYL]NAPHT-2-YL} N-METHYL METHANESULFONAMIDE**

**STADE I** : 6-ACETYL 2-BROMOMETHYL NAPHTALENE
On procède comme dans le stade II de l'exemple 1 en remplaçant le 1-acétyl 7-méthyl naphtalène par le 6-acétyl 2-méthyl naphtalène (exemple 1 stade I).
On obtient le 6-acétyl 2-bromométhyl naphtalène avec un rendement de 24 %
**Point de fusion** : 80° C
**Infrarouge** (pastille KBr)
1690 cm-1 ΥC = 0
1360 cm⁻¹ 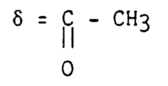 815 cm-1 ν CH
**RMN** (CDCl₃, TMS) :

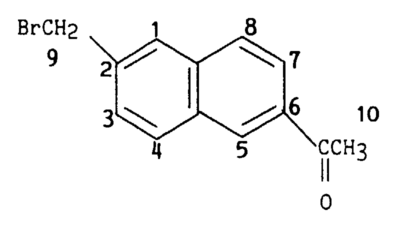

| | | | | | | | | |
|---|---|---|---|---|---|---|---|---|
| N° proton | 1 | 3 | 4 | 5 | 7 | 8 | 9 | 10 |
| δ(ppm) | 7,84 | 7,56 | 7,93 | 8,42 | 8,04 | 7,83 | 4,65 | 2,72 |

**STADE II** : 2-(6-ACETYL NAPHT-2-YL) METHANESULFONATE DE SODIUM
On procède comme pour le stade III de l'exemple 1 en remplacant le 1-acétyl 7-bromométhyl naphtalène par le 6-acétyl 2-bromométhyl naphtalène.
On obtient ainsi le 2-(6-acétyl napht-2-yl) méthanesulfonate de sodium avec un rendement de 87 %
**Point de fusion** : > 260° C

| **Microanalyse :** | | |
|---|---|---|
| calculé | C(52,08) | H(4,20) |
| mesuré | C(52,05) | H(4,12) |

**Infrarouge** (Pastille KBr)
3040 - 2900 cm-1 ν CH (alkyle)
1675 cm-1 ν C = O
1620 cm-1 ν C = C (aromatique)
1060 cm-1 ν SO₂
**RMN** (DMSO, d₆) δ (ppm) 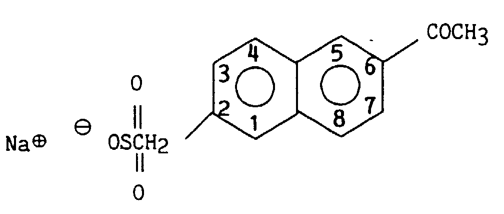 2,71 (s, 3H) CH₃
3,84 (s, 2H) CH₂
7,57 - 8,07 (massif, 5H) H₁, H₃, H₄, H₇, H₈
8,63 (1H) H₅
**STADE III** : CHLORURE DE L'ACIDE 2-(6-ACETYL NAPHT-2-YL) METHANE SULFONIQUE
On procède comme pour le stade IV de l'exemple 1 en remplaçant le 2-(1-acétyl napht-7-yl) méthanesulfonate de sodium par le 2-(6-acétyl napht-2-yl) méthanesulfonate de sodium.
On obtient ainsi le chlorure de l'acide 2-(6-acétyl napht-2-yl) méthane sulfonique avec un rendement de 96 %
**Point de fusion** : 105 - 107° C
**Infrarouge** (Pastille KBr)
   3000 - 2900 cm-1 ν CH (alkyle)
   1670 cm-1 ν C = 0
   1620 cm-1 ν C = C (aromatique)
   1160 cm-1 ν SO₂
**RMN** (DMSO, d₆) δ (ppm) 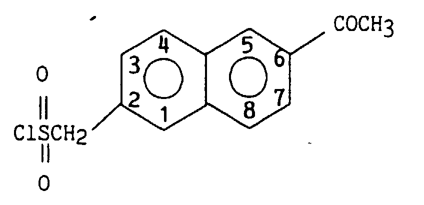 2,75 (s, 3H) CH₃
   5,07 (s, 2H) CH₂
   7,57 - 8,19 (massif, 5H) H₁, H₃, H₄, H₇, H₈
   8,50 (1H) H₅

**STADE IV** : 2-(6-ACETYL NAPHT-2-YL) N-METHYL METHANESULFONAMIDE
On procède comme pour le stade V de l'exemple 1 en remplaçant le chlorure de l'acide 2-(1-acétyl napht-7-yl) méthanesulfonique par le chlorure de l'acide 2-(6-acétyl napht-2-yl) méthanesulfonique.
On obtient ainsi le 2-(6-acétyl napht-2-yl) N-méthyl méthanesulfonamide avec un rendement de 70 %
**Point de fusion** : 167 - 169° C

| **Microanalyse :** | | | |
|---|---|---|---|
| calculé | C(60,63) | H(5,45) | N(5,05) |
| mesuré | C(61,05) | H(5,54) | N(4,97) |

**Infrarouge** (Pastille KBr)
3230 cm-1 ν NH
3040 - 2800 cm-1 ν CH (alkyle)
1660 cm-1 ν C = 0
1625 cm-1 ν C = C (aromatique)
1330 cm-1 ν SO₂
**RMN** (DMSO, d₆) δ (ppm) 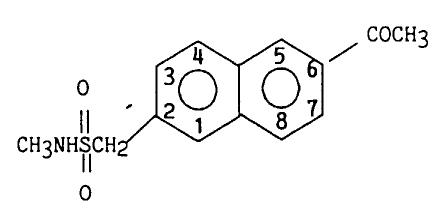 2,61 (d, 3H) CH₃NH (JCH₃NH = 4,6 Hz, pas de couplage dans D₂O)
2,67 (s, 3H) 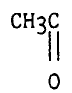 4,57 (s, 2H) CH₂SO₂
7,00 (q, 1H) NHCH₃ (JNHCH₃ = 4,6 Hz, pas de couplage dans D₂O)
7,57 - 7,67 (dd, 1H) H₃ ou H₇ (Jo = 7,6 Hz, Jm = 1,5 Hz)
8,00 - 8,19 (massif, 4H) H₁, H₄, H₈, H₃ ou H₇
8,67 (1H) H₅
**STADE V** : 2-(6-BROMOACETYL NAPHT-2-YL)N-METHYL METHANESULFONAMIDE
On procède comme pour le stade VI de l'exemple 1 en remplaçant le 2-(1-acétyl napht-7-yl) N-méthyl méthanesulfonamide par le 2-(6-acétyl napht-2-yl) N-méthyl méthanesulfonamide.
On obtient le 2-(6-bromoacétyl napht-2-yl) N-méthyl méthanesulfonamide avec un rendement de 87 %
**Point de fusion** : 208 - 210° C

| **Microanalyse :** | | | |
|---|---|---|---|
| calculé | C(46,03) | H(4,13) | N(3,83) |
| mesuré | C(46,34) | H(3,74) | N(3,81) |

**Infrarouge** (Pastille KBr)
3240 cm-1 ν NH
3000 - 2800 cm-1 ν CH (alkyle)
1685 - 1665 cm-1 ν C = O
1625 cm-1 ν C = C (aromatique)
1305 cm-1 ν SO₂
**RMN** (DMSO, d₆) δ (ppm) 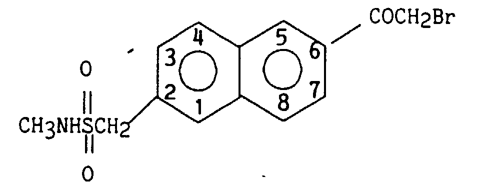 2,61 (d, 3H) CH₃NH (JCH₃NH = 4,6 Hz, pas de couplage dans D₂O)
4,57 (s, 2H) CH₂SO₂
7,00 (q, 1H) NHCH₃ (JNHCH₃ = 4,6 Hz, pas de couplage dans D₂O)
7,59 - 7,69 (dd, 1H) H₃ ou H₇ (Jo = 7,6 Hz, Jm = 1,5 Hz)
8,00 - 8,19 (massif, 4H) H₁, H₄, H₈, H₃ ou H₇
8,75 (1H) H₅
**STADE VI** : 2-[6-(2-BROMOETHYL) NAPHT-2-YL] N-METHYL METHANESULFONAMIDE
On procède comme pour le stade VII de l'exemple 1 en remplaçant le 2-(1-bromoacétyl napht-7-yl) N-méthyl méthanesulfonamide par le 2-(6-bromoacétyl napht-2-yl) N-méthyl méthanesulfonamide.
On obtient ainsi le 2-[6-(2-bromoéthyl) napht-2-yl] N-méthyl méthanesulfonamide avec un rendement de 70 %.
**Point de fusion** : 155 - 157° C

| **Microanalyse :** | | | |
|---|---|---|---|
| calculé | C(49,12) | H(4,71) | N(4,09) |
| mesuré | C(49,66) | H(4,70) | N(4,42) |

**Infrarouge** (Pastille KBr)
3265 cm-1 ν NH
1600 cm-1 ν C = C (aromatique)
1310 cm-1 ν SO₂
**RMN** (DMSO, d₆) δ (ppm) 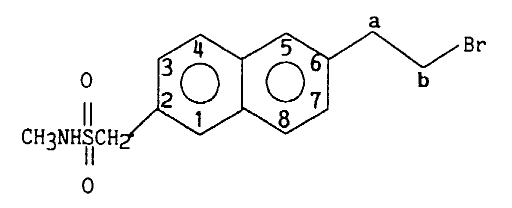 2,60 (d, 3H) CH₃NH (JCH₃NH = 5,7 Hz, disparait dans D₂O)
3,32 (t, 2H) Ha (Ja,b = 7,1 Hz)
3,84 (t, 2H) Hb (Jb,a = 7,1 Hz)
4,50 (s, 2H) CH₂SO₂
6,84 (q, 1H) NHCH₃ (JNHCH₃ = 5,7 Hz, disparait dans D₂O)
7,42 - 7,94 (massif, 6H) H₁, H₃, H₄, H₅, H₇, H₈
**STADE VII** : 2-{6-[2-(DIMETHYLAMINO) ETHYL] NAPHT-2-YL} N-METHYL METHANESULFONAMIDE
On procède comme pour le stade VIII de l'exemple 1 en remplaçant le 2-[1-(2-bromoéthyl) napht-7-yl] N-méthyl méthanesulfonamide par le 2-[6(2-bromoéthyl) napht-2-yl] N-méthyl méthanesulfonamide
On obtient ainsi le 2-{6-[2-(diméthylamino) éthyl] napht-2-yl} N-méthyl méthanesulfonamide avec un rendement de 38 %.
**Point de fusion** : 177 - 179° C

| **Microanalyse :** | | | |
|---|---|---|---|
| calculé | C(62,71) | H(7,23) | N(9,14) |
| mesuré | C(62,70) | H(7,32) | N(8,90) |

**Infrarouge** (Pastille KBr)
3020 - 3040 cm-1 ν NH (sulfonamide)
3950 - 2760 cm-1 ν CH (alkyle)
1600 cm-1 ν C = C (aromatique)
1320 cm-1 ν SO₂
**RMN** (DMSO, d₆) δ (ppm) 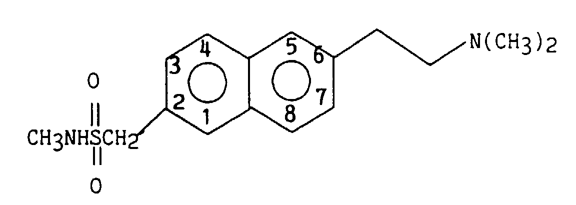 2,21 (s, 6H) N(CH₃)₂
2,35 - 2,50 (m, 2H) Hb
2,60 (d, 3H) CH₃NH (JCH₃NH = 4,2 Hz, disparait dans D₂O)
2,79 - 3,00 (m, 2H) Ha
4,57 (s, 2H) CH₂SO₂
6,91 (q, 1H) NHCH₃ (JNHCH₃ = 4,2 Hz, disparait dans D₂O)
7,38 - 7,50 (dd, 2H) H₃ et H₇ (Jo = 8,4 Hz, Jm = 2,8 Hz)
7,71 - 7,87 (massif, 4H) H₁, H₄, H₅, H₈

### EXEMPLES 30 A 37

En procédant comme dans les exemples 2 à 6 mais en remplaçant le 2-[1-(2-bromoéthyl) napht-7-yl] N-méthyl méthanesulfonamide par le 2-[6-(2-bromoéthyl) napht-2-yl] N-méthyl méthanesulfonamide on obtient :
- **EXEMPLE 30 :**: **2-[6-(2-AMINOETHYL) NAPHT-2-YL] N-METHYL METHANE SULFONAMIDE**
- **EXEMPLE 31 :**: **CHLORHYDRATE DE 2-[6-(2-MORPHOLINOETHYL) NAPHT-2-YL] N-METHYL METHANESULFONAMIDE**
- **EXEMPLE 32** :: **2-{6-[2-(3-AZABICYCLO [3,3,0] OCT-3-YL) ETHYL] NAPHT-2-YL} N-METHYL METHANESULFONAMIDE**
- **EXEMPLE 33 :**: **CHLORHYDRATE DE 2-[6-(2-PIPERIDINO ETHYL) NAPHT-2-YL] N-METHYL METHANESULFONAMIDE**
- **EXEMPLE 34 :**: **CHLORHYDRATE DE 2-{6-[2-(4-METATRIFLUOROMETHYL PHENYL PIPERAZIN-1-YL) ETHYL] NAPHT-2-YL} N-METHYL METHANESULFONAMIDE**
- **EXEMPLE 35 :**: **CHLORHYDRATE DE 2-{6-[2-(4-PARAMETHOXYPHENYL PIPERAZIN-1-YL) ETHYL] NAPHT-2-YL} N-METHYL METHANESULFONAMIDE**
- **EXEMPLE 36 :**: **CHLORHYDRATE DE 2-{6-[2-(4-(2,3,4-TRIMETHOXYBENZYL PIPERAZIN-1-YL) ETHYL] NAPHT-2-YL} N-METHYL METHANESULFONAMIDE**
- **EXEMPLE 37 :**: **CHLORHYDRATE DE 2-{6-[2-(4-PHENYL HOMOPIPERAZIN-1-YL) ETHYL] NAPHT-2-YL} N-METHYL METHANESULFONAMIDE**
- **EXEMPLE 38 :**: **2-{6-[2-(4-BENZOYL PIPERAZIN-1-YL) ETHYL] NAPHT-2-YL} N-METHYL METHANESULFONAMIDE**

En procédant comme pour l'exemple 34 mais en remplacant la 1-(métatrifluorométhyl phényl) pipérazine par la 1-benzoyl pipérazine on obtient le 2-{6-[2-(4-benzoyl piperazin-1-yl) éthyl] napht-2-yl} N-méthyl methanesulfonamide

En procédant de la même façon, on obtient :
- **EXEMPLE 39 :**: **2-{1-[2-(4-ACETYL PIPERAZIN-1-YL} ETHYL] YAPHT-7-YL) N-METHYL METHANESULFONAMIDE**

### EXEMPLES 40 A 53

En procédant comme dans les exemples 12 à 23 mais en remplaçant le 2-[1-(2-bromoéthyl) napht-7-yl] N-méthyl méthanesulfonamide par le 2-[6-bromoéthyl napht-2-yl] N-méthyl méthanesulfonamide on obtient :
- **EXEMPLE 40 :**: **CHLORHYDRATE DE 2-{6-[2-(3-AZASPIRO [5,5] UNDECAN-3-YL) ETHYL] NAPHT-2-YL} N-METHYL METHANESULFONAMIDE**
- **EXEMPLE 41 :**: **CHLORHYDRATE DE 2-{6-[2-(3-AZABICYCLO [3,2,2] NONAN-3-YL) ETHYL] NAPHT-2-YL} N-METHYL METHANESULFONAMIDE**
- **EXEMPLE 42 :**: **CHLORHYDRATE DE 2-[6-(2-PYRROLIDINOETHYL) NAPHT-2-YL] N-METHYL METHANESULFONAMIDE**
- **EXEMPLE 43 :**: **CHLORHYDRATE DE 2-[6-(2-THIOMORPHOLINOETHYL) NAPHT-2-YL} N-METHYL METHANESULFONAMIDE**
- **EXEMPLE 44 :**: **CHLORHYDRATE DE 2-[6-(2-HEXAMETHYLENEIMINOETHYL) NAPHT-2-YL] N-METHYL METHANESULFONAMIDE**
- **EXEMPLE 45 :**: **CHLORHYDRATE DE 2-[6-(2-HEPTAMETHYLENEIMINOETHYL) NAPHT-2-YL] N-METHYL METHANESULFONAMIDE**
- **EXEMPLE 46 :**: **CHLORHYDRATE DE 2-{6-[2-(N,N-DIPROPYL) AMINOETHYL] NAPHT-2-YL} N-METHYL METHANESULFONAMIDE**
- **EXEMPLE 47 :**: **CHLORHYDRATE DE 2-{6-[2-(N-BENZYL N-METHYL AMINO) ETHYL] NAPHT-2-YL} N-METHYL METHANESULFONAMIDE**
- **EXEMPLE 48 :**: **CHLORHYDRATE DE 2-{6-[2-(N-METHYL N-PHENYL AMINO) ETHYL] NAPHT-2-YL} N-METHYL METHANESULFONAMIDE**
- **EXEMPLE 49 :**: **CHLORHYDRATE DE 2-{6-[2-(N-CYCLOHEXYLAMINO) ETHYL] NAPHT-2-YL} N-METHYL METHANESULFONAMIDE**
- **EXEMPLE 50 :**: **2-{6-[2-(N-METHYLAMINO) ETHYL] NAPHT-2-YL} N-METHYL METHANESULFONAMIDE**
- **EXEMPLE 51 :**: **2-{6-[2-(N-METHYL N-ETHYLAMINO) ETHYL] NAPHT-2-YL} N-METHYL METHANESULFONAMIDE**
- **EXEMPLE 52 :**: **CHLORHYDRATE DE 2-{6-[2-(N-4-FLUOROPHENYL AMINO) ETHYL] NAPHT-2-YL} N-METHYL METHANESULFONAMIDE**
- **EXEMPLE 53 :**: **CHLORHYDRATE DE 2-{6-[2-(N- 3,4-DICHLOROPHENYL AMINO) ETHYL] NAPHT-2-YL} N-METHYL METHANESULFONAMIDE**
- **EXEMPLE 54 :**: **2-{6-[2-(N-ALLYLAMINO) ETHYL] NAPHT-2-YL} N-METHYL METHANESULFONAMIDE**

En procédant comme pour l'exemple 29 mais en remplaçant la diméthylamine par l'allylamine on obtient la 2-{6-[2-(N-allylamino) éthyl] napht-2-yl} N-méthyl méthanesulfonamide
- **EXEMPLE 55**: **2-{6-[2-(DIMETHYLAMIYO) ETHYL] NAPHT-2-YL} N-BENZYL METHANESULFONAMIDE**

En procédant comme dans l'exemple 24 mais en remplaçant le 2-[1-(2-bromoéthyl)napht-7-yl] N-benzyl méthanesulfonamide par le 2-[6-(2-bromoéthyl)napht-2-yl] N-benzyl méthanesulfonamide on obtient le 2-{6-[2-(diméthylamino) éthyl] napht-2-yl} N-benzyl méthanesulfonamide.

### EXEMPLES 56 A 59

En procédant comme pour l'exemple 55 mais en remplaçant le 2-[6-(2-bromoéthyl)napht-2-yl] N-benzyl méthanesulfonamide par :
- le 6-(2-bromoéthyl) 2-morpholinosulfonylméthyl naphtalène, on obtient :
   - **EXEMPLE 56 :**: **6-[2-(DIMETHYLAMINO) ETHYL] 2-(MORPHOLINO SULFONYMETHYL) NAPHTALENE**
- le 2-[6-(2-bromoéthyl) napht-2-yl] N,N-diméthyl méthane sulfonamide, on obtient :
   - **EXEMPLE 57 :**: **2-{6-[2-(DIMETHYLAMINO) ETHYL] NAPHT-2-YL} N,N-DIMETHYL METHANESULFONAMIDE**
- le 2-[6-(2-bromoéthyl) napht-2-yl] N-cyclopentylméthane sulfonamide, on obtient :
   - **EXEMPLE 58 :**: **2-{6-[2-(DIMETHYLAMINO) ETHYL] NAPHT-2-YL} N-CYCLOPENTYL METHANESULFONAMIDE**
- le 2-[6-(2-bromoéthyl) napht-2-yl] N-tétraméthylène méthane sulfonamide, on obtient :
   - **EXEMPLE 59 :**: **2-{6-[2-(DIMETHYLAMINO) ETHYL] NAPHT-2-YL} N-TETRAMETHYLENE METHANESULFONAMIDE**
   - **EXEMPLE 60 :**: **2-{7-[2-(DIMETHYLAMINO) ETHYL] NAPHT-1-YL} N-METHYL METHANESULFONAMIDE**

En procédant comme pour l'exemple 1 mais en remplaçant le 1-acétyl 7-méthyl naphtalène par le 7-acétyl 1-méthyl naphtalène (Bull. Soc. Chim. Fr. (1978) pp 104-108) on obtient le 2-{7-[2-(diméthylamino) éthyl] napht-1-yl} N-méthyl méthanesulfonamide
**Infrarouge** (Pastille KBr)
3030 - 3070 cm-1 ν NH
1600 cm-1 ν C = C (aromatique)
1320 cm-1 ν SO₂
- **EXEMPLE 61 :**: **2-{7-[2-(DIMETHYLAMINO) ETHYL] NAPHT-1-YL} N-METHYL ACETAMIDE**

On procède de la même façon que pour le 2-{1-[2-(diméthylamino) éthyl] napht-7-yl} N-méthyl méthanesulfonamide (Exemple 1) en remplaçant dans le stade V le chlorure de l'acide 2-(1-acétyl napht-7-yl) méthane sulfonique par le chlorure de l'acide 2-(1-acétyl napht-7-yl) acétique.

On obtient ainsi le 2-{1-[2-(diméthylamino) éthyl] napht-7-yl} N-méthyl acétamide avec un rendement de 35 %
**Point de fusion** : 159 - 163° C (chlorhydrate)
**Infrarouge** (Pastille KBr) (chlorhydrate)
   3330 - 3060 cm-1 ν NH
   3030 - 2800 cm-1 ν CH
   2250 - 1700 cm-1 ν NH⁺ chlorhydrate
   1670 - 1660 cm-1 ν C = O amide
**RMN** (DMSO, d₆) δ (ppm) 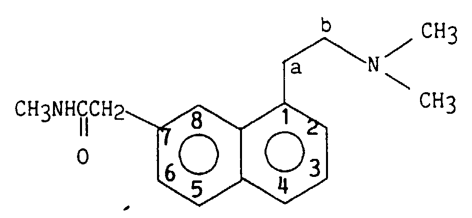 2,61 (m, 3H) CH₃NH
   2,86 (d, 6H) (CH₃)₂ N⁺
   3,44 (m, 4H) Ha, Hb
   3,66 (s, 2H) CH₂CO
   6,63 (signal, 1H) NHCH₃ (disparait dans D₂O)
   7,38-7,94 (massif, 5H) H₂, H₃, H₄, H₅, H₆
   8,22 (signal, 1H) H₈
   11,05 (signal , 1H) NH⁺ (disparait dans D₂O)

Le chlorure de l'acide 2-(1-acétyl napht-7-yl) acétique est obtenu de manière classique par traitement avec du chlorure de thionyle de l'acide 2-(1-acétyl napht-7-yl) acétique qui est préparé de la manière suivante :
STADE I : 2-(1-ACETYL NAPHT-7-YL) ACETONITRILE
   Méthode A
      Chauffer à 60° C pendant 30 minutes une suspension de cyanure de sodium dans 25 cm3 de DMSO puis additionner progressivement 10 g de 1-acétyl 7-bromométhyl naphtalène.
      Maintenir le chauffage à 60° C pendant 90 minutes puis refroidir et verser le milieu réactionnel dans 300 cm3 d'eau.
      Le précipité qui se forme est isolé par filtration, lavé à l'eau, séché puis recristallisé dans l'éthanol.
      On obtient ainsi le 2-(1-acétyl napht-7-yl) acétonitrile avec un rendement de 96 %.
   Méthode B
      Chauffer à reflux pendant 10 heures un mélange de 15 g de 1-acétyl 7-bromométhyl naphtalène et de 3 g de cyanure de sodium dans 150 cm3 d'éthanol.
      Après refroidissement et mise à sec, reprendre le milieu réactionnel par de la soude diluée, extraire à l'éther puis remettre à sec.
      On obtient ainsi le 2-(1-acétyl napht-7-yl) acétonitrile avec un rendement voisin de 50 %.
      **Point de fusion :** 88° C
      **Infrarouge** (Pastille KBr)
         3100 - 2900 cm-1 v CH
         2240 cm-1 v C ≡ N
         1660 - 1650 cm-1 v C = O
      **RMN** (CDCl₃) δ (ppm) 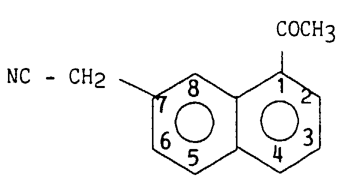 2,78 (s, 3H) CH₃
         3,92 (s, 2H) CH₂
         7,42 - 8,07 (m, 5H) H₂, H₃, H₄, H₅, H₆
         8,78 (s, 1H) H₈
STADE II : ACIDE 2-(1-ACETYL NAPHT-7-YL) ACETIQUE
   Méthode A
      Dissoudre à chaud 3 g de 2-(1-acétyl napht-7-yl) acétonitrile dans 10 cm3 d'acide acétique.
      Ajouter 40 cm3 d'acide chlorhydrique concentré puis chauffer à reflux pendant 3 heures.
      Après refroidissement, le milieu réactionnel est versé dans 200 cm3 d'eau et le précipité formé isolé par filtration, lavé à l'eau, séché puis recristallisé dans l'éthanol à 95 °.
      On obtient ainsi l'acide 2-(1-acétyl napht-7-yl) acétique avec un rendement de 80 %.
   Méthode B
      Chauffer à 100° C pendant 48 heures un mélange constitué d'une solution de 15 g de 2-(1-acétyl napht-7-yl) acétonitrile dans 125 cm3 d'éther monométhylique de l'éthylèneglycol à laquelle on a additionné 0,5 moles d'hydroxyde de potassium dans 75 cm3 d'eau.
      Après refroidissement, la phase aqueuse est lavée avec de l'éther puis acidifiée avec de l'acide chlorhydrique 1N.
      L'acide qui précipite est isolé par filtration.
      On obtient ainsi l'acide 2-(1-acétyl napht-7-yl) acétique avec un rendement de 60 %
      **Point de fusion** : 131-132° C
      **Infrarouge** (Pastille KBr)
         3100 - 2700 cm-1 ν OH
         1710 - 1700 cm-1 νC = O (acide)
         1660 cm-1 ν C = O (cétone)
      **RMN** (CDCl₃) δ (ppm) 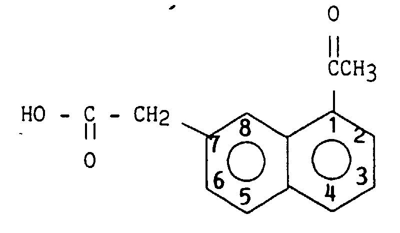 2,76 (s, 3H) CH₃
         3,88 (s, 2H) CH₂
         6,00 (s, 1H) OH (échangeable dans D₂O)
         7,38 - 8,00 (m, 5H) H₂, H3, H₄, H₅, H₆
         8,69 (s, 1H) H8

### EXEMPLE 62 : 2-{6-[2-(DIMETHYLAMINO) ETHYL] NAPHT-2-YL} N-METHYL ACETAMIDE

On procède comme pour l'exemple 61 en remplaçant le chlorure de l'acide 2-(1-acétyl napht-7-yl) acétique par le chlorure de l'acide 2-(6-acétyl napht-2-yl) acétique obtenu de la même façon à partir du 6-acétyl 2-bromométhyl naphtalène.

### EXEMPLE 63 : COMPOSITIONS PHARMACEUTIQUES

Comprimés dosés à 5 mg de 2-{1-[2-(diméthylamino) éthyl] napht-7-yl} N-méthyl méthanesulfonamide

| Formule de préparation pour 1000 comprimés | |
|---|---|
| 2-{1-[2-(diméthylamino) éthyl] napht-7-yl} N-méthyl méthanesulfonamide | 5 g |
| Amidon de blé | 20 g |
| Amidon de mais | 20 g |
| Lactose | 70 g |
| Stéarate de magnésium | 1 g |
| Silice | 1 g |
| Hydroxypropylcellulose | 2 g |

### ETUDE PHARMACOLOGIQUE DES DERIVES DE L'INVENTION

### EXEMPLE A : DETERMINATION IN VITRO DE L'AFFINITE POUR LES RECEPTEURS SEROTONINERGIQUES

Les tests d'affinité in vitro des composés de l'invention pour les récepteurs 5 HT₁A, 5 HT₁C, 5 HT₁D, 5 HT₂, 5 HT₃, D₁, D₂, α₁, α₂ ont été effectués selon les techniques classiques par déplacement d'un radio ligand de référence

| **RECEPTEUR** | **RADIOLIGAND** | **TISSU UTILISE** |
|---|---|---|
| 5 HT₁A | 8-OH DPAT | Hippocampe |
| 5 HT₁C | N-Methyl Mesulergine | Cortex frontal, hippocampe |
| 5 HT₁D | 5-OH Tryptamine | Cortex + striatum + GP |
| 5 HT₂ | Amino iodo ketansérine | Cortex frontal |
| 5 HT₃ | BRL 43694 | Area postrema NTS |

Les résultats de ces études de binding montrent que les composés de l'invention possèdent une grande affinité pour les récepteurs sérotoninergiques associée à une forte sélectivité pour les récepteurs 5HT₁D

Cette sélectivité pour les récepteurs 5 HT₁D est également très importante (au moins un facteur 100) par rapport aux récepteurs dopaminergiques D₁, D₂, et adrénergiques α₁, α₂.

Le 2-{1-[2-(diméthylamino) éthyl] napht-7-yl} N-méthyl méthanesulfonamide par exemple, présente une affinité de l'ordre de 10⁻⁷ M pour les récepteurs 5HT₁D alors que son affinité pour les autres récepteurs sérotoninergiques est comprise entre 10⁻⁶ M et 10⁻⁵ M et son affinité pour les récepteurs D1, D2, α₁, α₂ inférieure à 10⁻⁵ M.

### EXEMPLE B : ETUDE DE LA TOXICITE AIGUE

La toxicité aigue "Per Os" des composés de l'invention a été évaluée après administration orale des composés à tester à des lots de 10 souris "SWISS" (poids moyen 22 g)

Les animaux sont observés à intervalles réguliers au cours de la première journée et quotidiennement pendant les deux semaines suivant le traitement. La DL 50 entrainant la mort de 50 % des animaux a été évaluée.

Pour la plupart des composés testés, la DL 50 est supérieure à 1 g Kg⁻¹ ce qui indique une faible toxicité aigue "Per Os".

### EXEMPLE C : MISE EN EVIDENCE IN VITRO D'UNE ACTIVITE VASOCONSTRICTRICE AU NIVEAU DE L'ARTERE BASILIAIRE DE CHIEN

Les expériences sont effectuées sur des fragments annulaires d'artère basiliaire de chien dépourvus d'endothélium placés dans une chambre d'organe permettant la mesure de contractions isométriques en présence ou non de Kétansérine.

Les études effectuées montrent que les produits de l'invention, comme la sérotonine, sont capables d'induire des contractions des fragments artériels. Le fait que ces contractions ne soient pas antagonisées par la Kétansérine montre que ces contractions ne sont pas médiées par les récepteurs 5HT₂.

Le 2-{1-[2-(diméthylamino) éthyl] napht-7-yl} N-méthyl méthanesulfonamide par exemple, possède un CE50 de l'ordre de 10⁻⁶ M dans ce test.

Le même produit testé à la même dose sur des fragments annulaires d'artère coronaire ou sur des fragments de veine saphène n'a montré aucun effet significatif.

## Revendications (Revendications pour l'(les) Etat(s) contractant(s) suivant(s): AT, BE, CH, DE, DK, FR, GB, GR, IE, IT, LI, LU, NL, PT, SE)

1. Composés de formule générale (I) : 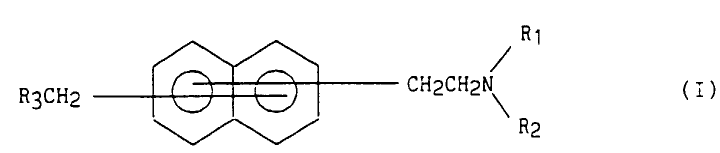 dans laquelle, les deux substituants, indépendemment l'un de l'autre, pouvant être localisés sur n'importe lequel des deux cycles du naphtalène,
- R₁ et R₂, identiques ou différents, représentent chacun indépendamment l'un de l'autre :
- un hydrogène,
- un alkyle linéaire ou ramifié de 1 à 6 atomes de carbone,
- un cycloalkyle de 3 à 7 atomes de carbone, ou cycloalkyle-(C₁-C₄) alkyle,
- un alcène linéaire ou ramifié de 2 à 6 atomes de carbone,
- un aryle éventuellement substitué,
- un aralkyle éventuellement substitué dont la chaine alkyle comprend de 1 à 3 atomes de carbone,
ou forment ensemble, avec l'atome d'azote qui les porte un système cyclique choisi parmi : 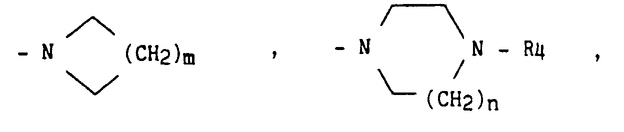 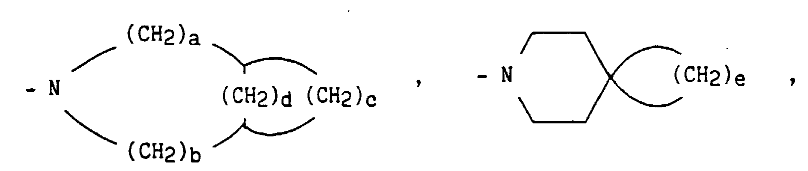 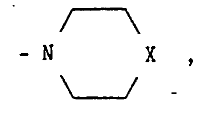
- X représente un oxygène ou un soufre,
- m, nombre entier, peut prendre les valeurs 0, 1, 2, 3, 4 ou 5,
- n, nombre entier, peut prendre les valeurs 1 ou 2,
- a et b, nombres entiers, peuvent prendre les valeurs 0, 1 ou 2 avec a + b ≠ 0,
- c et d, nombres entiers, peuvent prendre les valeurs 0, 1, 2, 3 ou 4 avec c + d ≠ 0,
- e, nombre entier peut prendre les valeurs 4 ou 5,
- R₄ représente :
- un hydrogène,
- un alkyle linéaire ou ramifié de 1 à 6 atomes de carbone,
- un aryle éventuellement substitué,
- un aralkyle éventuellement substitué dont la chaine alkyle comprend de 1 à 3 atomes de carbone,
- un groupement 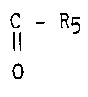 dans lequel R₅ représente un alkyle linéaire ou ramifié de 1 à 6 atomes de carbone, un phényle ou naphtyle éventuellement substitué,
- R₃ représente :
- un groupement 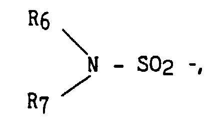
- un groupement 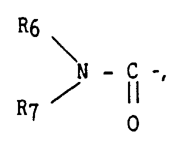
- R₆ et R₇, identiques ou différents ont la même définition que R₁ et R₂,
- leurs isomères, énantiomères, diastéréoisomères isolés ou sous forme de mélange,
- leurs sels d'addition à un acide minéral ou organique pharmaceutiquement acceptable,
- par groupement aryle on entend groupement, phényle, naphtyle, pyrimidyle, pyridyle,
- les expressions aryle éventuellement substitué et aralkyle éventuellement substitué signifient que le, ou les, noyaux aromatiques peuvent être substitués par un ou plusieurs radicaux choisis parmi hydroxy, halogène, trifluorométhyle, nitro, alkyle linéaire ou ramifié de 1 à 6 atomes de carbone ou alcoxy linéaire ou ramifié de 1 à 6 atomes de carbone.

2. Composés selon la revendication 1 de formule générale (I_{A}) : 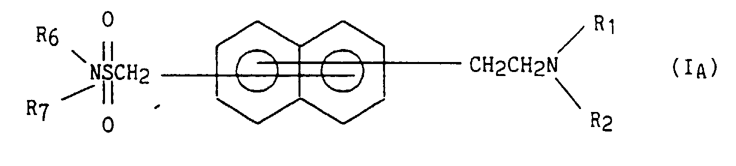 dans laquelle, les deux substituants, indépendemment l'un de l'autre, pouvant être localisés sur n'importe lequel des deux cycles du naphtalène, R₁, R₂, R₆ et R₇ sont tels que définis dans la revendication 1 et ont la même signification que dans les composés de formule générale (I), leurs isomères, énantiomères, diastéréoisomères, isolés ou sous forme de mélange ainsi que leurs sels d'addition à un acide minéral ou organique pharmaceutiquement acceptable.

3. Composés selon la revendication 1 de formule générale (I_{B}): 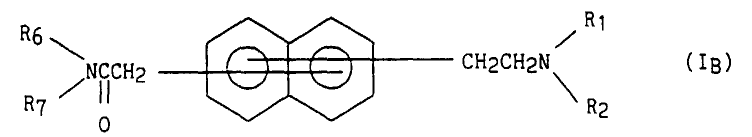 dans laquelle, les deux substituants, indépendemment l'un de l'autre, pouvant être localisés sur n'importe lequel des deux cycles du naphtalène, R₁, R₂, R₆ et R₇ sont tels que définis dans la revendication 1 et ont la même signification que dans les composés de formule générale (I), leurs isomères, énantiomères, diastéréoisomères, isolés ou sous forme de mélange ainsi que leurs sels d'addition à un acide minéral ou organique pharmaceutiquement acceptable.

4. Composés de formule générale (I) selon la revendication 1 pour lesquels les deux substituants se trouvent sur les sommets 1 et 7 du noyau naphtalénique et qui correspondent aux composés de formule générale suivante : 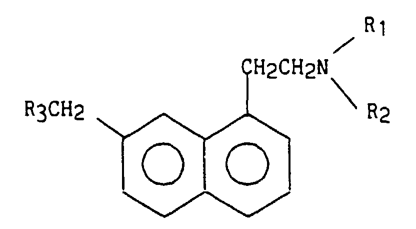 dans laquelle, R₁, R₂ et R₃ sont tels que définis dans la revendication 1 et ont la même signification que dans les composés de formule générale (I), leurs isomères, énantiomères, diastéréoisomères, isolés ou sous forme de mélange ainsi que leurs sels d'addition à un acide minéral ou organique pharmaceutiquement acceptable.

5. Composés de formule générale (I) selon la revendication 1 pour lesquels les deux substituants se trouvent sur les sommets 1 et 7 du noyau naphtalénique et qui correspondent aux composés de formule générale suivante : 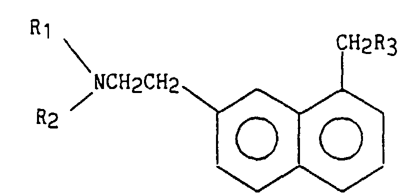 dans laquelle R₁, R₂ et R₃ sont tels que définis dans la revendication 1 et ont la même signification que dans les composés de formule générale (I), leurs isomères, énantiomères, diastéréoisomères, isolés ou sous forme de mélange ainsi que leurs sels d'addition à un acide minéral ou organique pharmaceutiquement acceptable.

6. Composés selon la revendication 1 qui est le 2-{1-[2-(diméthylamino) éthyl] napht-7-yl} N-méthyl méthanesulfonamide dont la formule est représentée ci dessous, ainsi que ses sels d'addition à un acide minéral ou organique pharmaceutiquement acceptable.

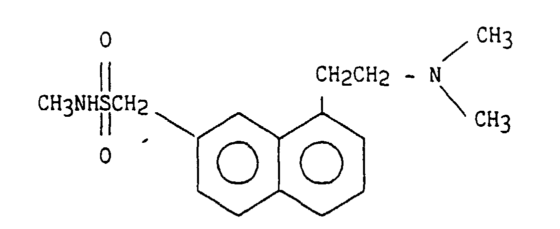

7. Composé selon la revendication 1 qui est le 2-{7-[2-(diméthylamino) éthyl] napht-1-yl} N-méthyl méthanesulfonamide dont la formule est représentée ci dessous, ainsi que ses sels d'addition à un acide minéral ou organique pharmaceutiquement acceptable.

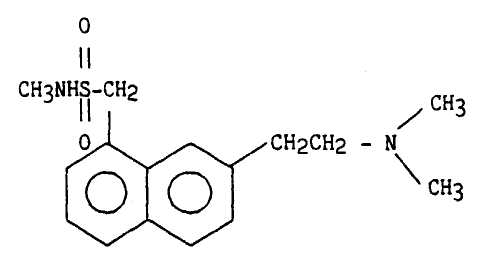

8. Composé selon la revendication 1 qui est le 2-{1-[2-(N-méthyl N-éthyl amino) éthyl] napht-7-yl} N-méthyl méthanesulfonamide dont la formule est représentée ci dessous, ainsi que ses sels d'addition à un acide minéral ou organique pharmaceutiquement acceptable.

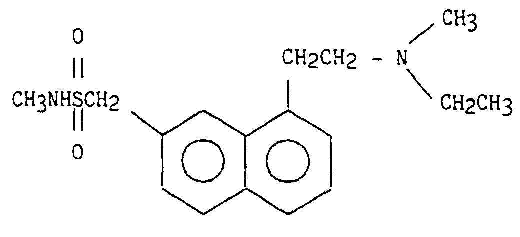

9. Composé selon la revendication 1 qui est le 2-{1-[2-(4-métatrifluorométhylphényl pipérazin-1-yl) éthyl] napht-7-yl} N-méthyl méthanesulfonamide dont la formule est représentée ci dessous, ainsi que ses sels d'addition à un acide minéral ou organique pharmaceutiquement acceptable.

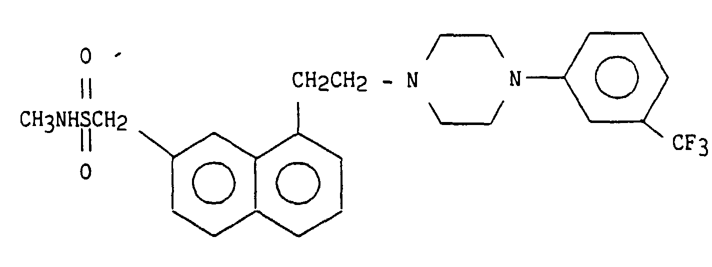

10. Composé selon la revendication 1 qui est le 2-{1-[2-(diméthyl amino) éthyl] napht-7-yl} N-méthyl acétamide dont la formule est représentée ci dessous ainsi que ses sels d'addition à un acide minéral ou organique pharmaceutiquement acceptable.

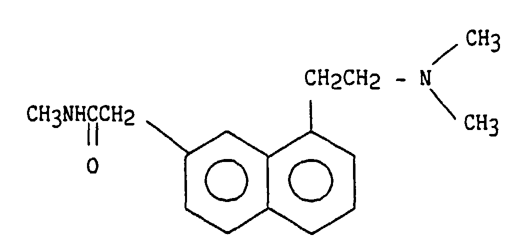

11. Composé selon la revendication 1 qui est le 2-{1-[2-(diméthyl amino) éthyl] napht-7-yl} N,N-diméthyl méthanesulfonamide dont la formule est représentée ci dessous ainsi que ses sels d'addition à un acide minéral ou organique pharmaceutiquement acceptable.

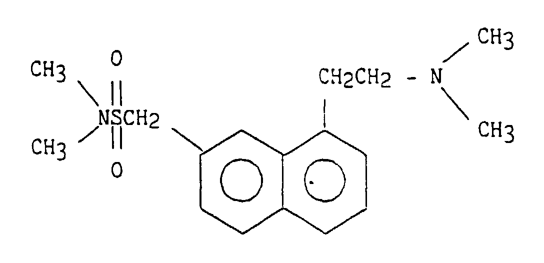

12. Composé selon la revendication 1 qui est le 2-[1-(2-piperidino éthyl) napht-7-yl] N-méthyl méthanesulfonamide dont la formule est représentée ci dessous ainsi que ses sels d'addition à un acide minéral ou organique pharmaceutiquement acceptable.

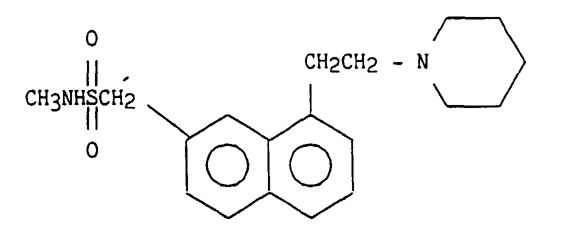

13. Composé selon la revendication 1 qui est le 2-{1-[2-(N-méthylamino) éthyl] napht-7-yl} N-méthyl méthanesulfonamide dont la formule est représentée ci dessous ainsi que ses sels d'addition à un acide minéral ou organique pharmaceutiquement acceptable.

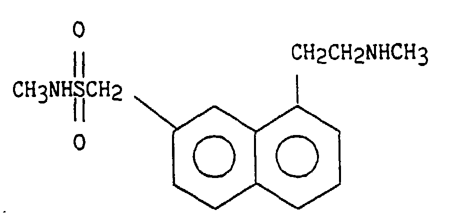

14. Procédé de préparation des composés de formule générale (I) caractérisé en ce que l'on part d'un composé naphtalénique de formule (II) : 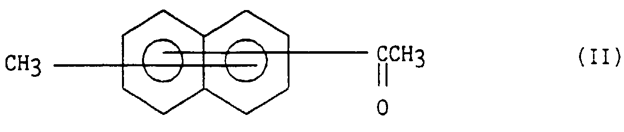 que l'on traite par du N-bromosuccinimide de manière à obtenir le composé bromé de formule (III) : 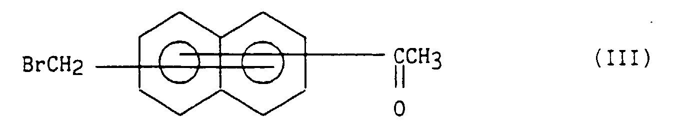 que l'on peut :
a) soit traiter par du sulfite de sodium dans un mélange acétone-eau de manière à obtenir le méthanesulfonate de formule (IV) : 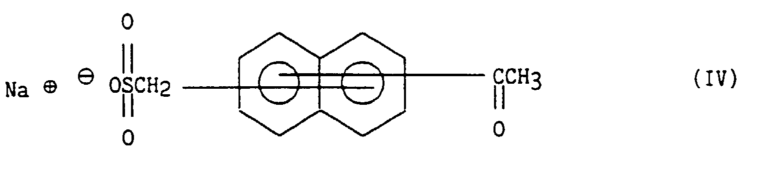 que l'on traite ensuite avec de l'oxychlorure de phosphore de manière à obtenir le chlorure de sulfonyle de formule (V) : 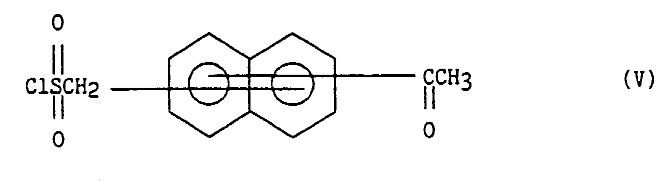 que l'on fait réagir ensuite avec une amine de formule (VI) : 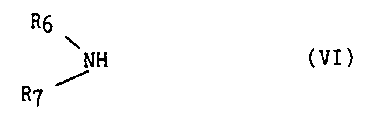 dans laquelle R₆ et R₇ ont la même signification que dans les composés de formule générale (I), de manière à obtenir le composé sulfonamidique de formule (VII) : 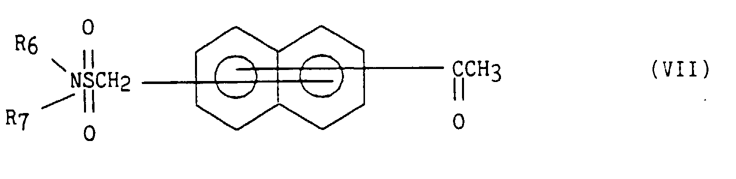 dans laquelle R₆ et R₇ ont la même signification que dans les composés de formule générale (I), que l'on traite ensuite par du brome de manière à obtenir le composé bromé de formule (VIII) : 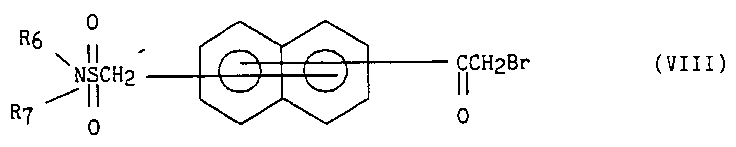 dans laquelle R₆ et R₇ ont la même signification que dans les composés de formule générale (I), que l'on réduit ensuite par du triéthylsilane dans l'acide trifluoroacétique de manière à obtenir le composé de formule (IX) : 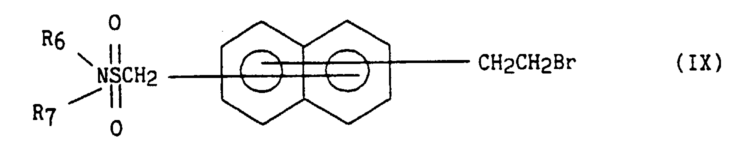 dans laquelle R₆ et R₇ ont la même signification que dans les composés de formule générale (I), que l'on l'on fait réagir :
- soit avec une amine de formule (X) : 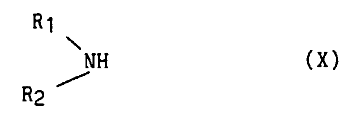 dans laquelle R₁ et R₂ ont la même signification que dans les composés de formule générale (I), de manière à obtenir le composé de formule (I_{A1}) : 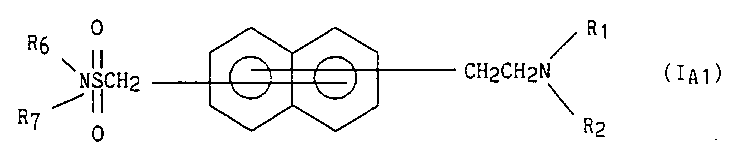 dans laquelle R₁, R₂, R₆ et R₇ ont la même signification que dans les composés de formule générale (I),
- soit avec du phtalimide potassique dans la DMF pour obtenir le composé de formule (XVII_{A}) : 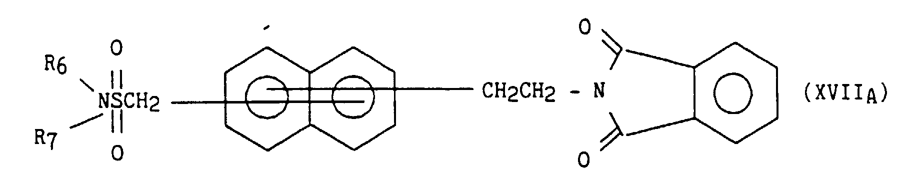 dans laquelle R₆ et R₇ ont la même signification que dans les composés de formule générale (I), que l'on fait réagir en milieu alcoolique avec de l'hydrate d'hydrazine pour obtenir l'amine primaire de formule (IA₂) 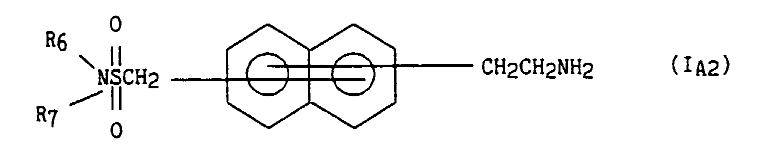 dans laquelle R₆ et R₇ ont la même signification que dans les composés de formule générale (I),
b) soit faire réagir avec un cyanure alcalin dans le DMSO ou en milieu alcoolique, de manière à obtenir le composé cyanométhylé de formule (XI) : 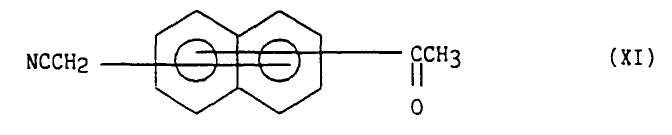 que l'on hydrolyse ensuite, soit en milieu hydroalcoolique avec un hydroxyde de métal alcalin,soit en milieu acide, de manière à obtenir l'acide naphtylacétique de formule (XII) : 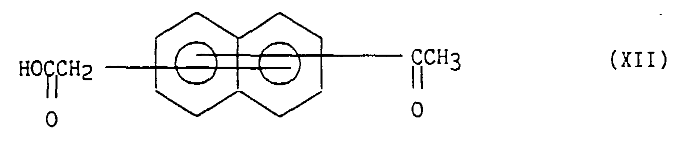 que l'on traite ensuite de manière classique par du chlorure de thionyle, de manière à obtenir le chlorure d'acide de formule (XIII) : 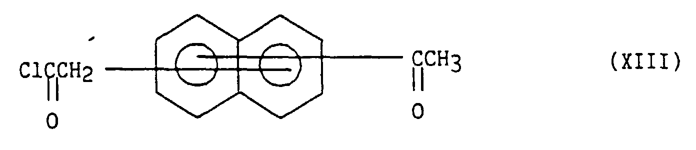 que l'on fait réagir ensuite avec une amine de formule (VI) : 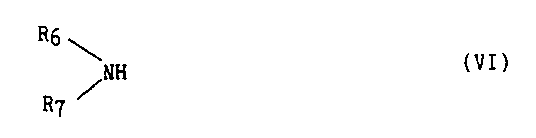 dans laquelle R₆ et R₇ ont la même signification que dans les composés de formule générale (I), de manière à obtenir le composé amidique de formule (XIV) : 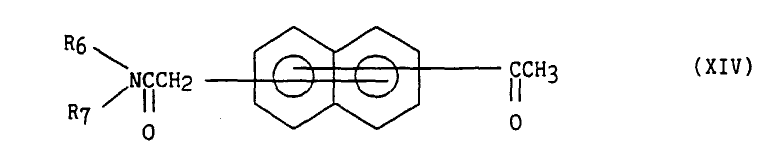 dans laquelle R₆ et R₇ ont la même signification que dans les composés de formule générale (I) que l'on traite ensuite par du brome de manière à obtenir le composé bromé de formule (XV) : 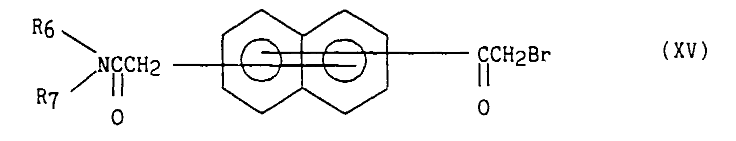 dans laquelle R₆ et R₇ ont la même signification que dans les composés de formule générale (I), que l'on réduit ensuite par du triéthylsilane dans l'acide trifluoroacétique de manière à obtenir le composé de formule (XVI) : 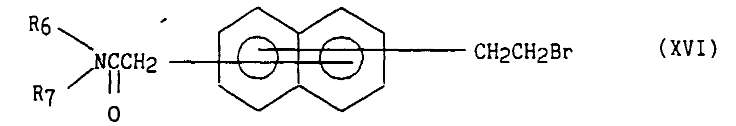 dans laquelle R₆ et R₇ ont la même signification que dans les composés de formule générale (I), que l'on fait réagir :
- soit avec une amine de formule (X) : 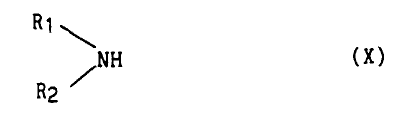 dans laquelle R₁ et R₂ ont la même signification que dans les composés de formule générale (I), de manière à obtenir le composé de formule (I_{B1}) : 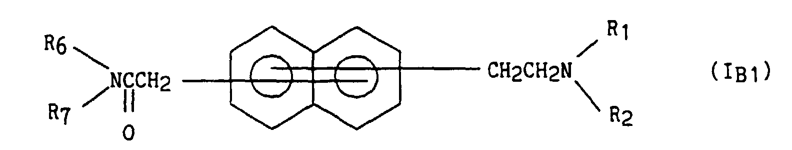 dans laquelle R₁, R₂, R₆ et R₇ ont la même signification que dans les composés de formule générale (I),
- soit avec du phtalimide potassique dans la DMF pour obtenir le composé de formule (XVII_{B}) : 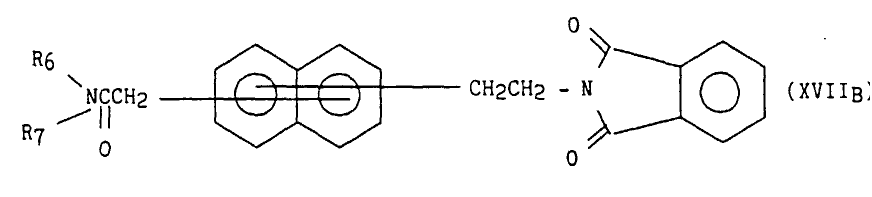 dans laquelle R₆ et R₇ ont la même signification que dans les composés de formule générale (I), que l'on fait réagir en milieu alcoolique avec de l'hydrate d'hydrazine pour obtenir l'amine primaire de formule (IB₂) 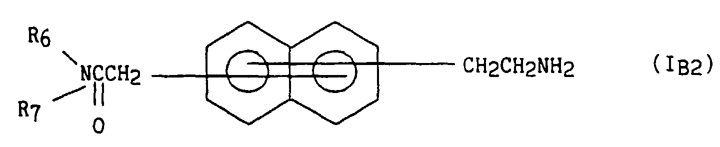 dans laquelle R₆ et R₇ ont la même signification que dans les composés de formule générale (I),
étant entendu que les composés de formules générales (I_{A1}), (I_{A2}), (I_{B1}) et (I_{B2}) représentent l'ensemble des composés de formule (I) et peuvent, si on le désire, être salifiés par un acide pharmaceutiquement acceptable et si nécessaire être séparés en leurs différents isomères.

15. Composition pharmaceutique contenant comme principe actif au moins un composé selon l'une des revendications 1 à 13 en combinaison avec un ou plusieurs excipients ou véhicules inertes, non toxiques pharmaceutiquement acceptables.

16. Composition pharmaceutique selon la revendication 15 contenant au moins un principe actif selon l'une des revendications 1 à 13 utilisable pour le traitement de troubles dus à une vasodilatation du système vasculaire plus particulièrement des migraines, des céphalées, des algies vasculaires de la face, et des troubles de la circulation artérielle et veineuse.

## Revendications (Revendications pour l'(les) Etat(s) contractant(s) suivant(s): ES)

1. Procédé de préparation des composés de formule générale (I) : 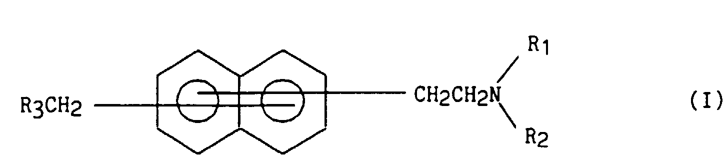 dans laquelle, les deux substituants, indépendemment l'un de l'autre, pouvant être localisés sur n'importe lequel des deux cycles du naphtalène,
- R₁ et R₂, identiques ou différents, représentent chacun indépendamment l'un de l'autre :
- un hydrogène,
- un alkyle linéaire ou ramifié de 1 à 6 atomes de carbone,
- un cycloalkyle de 3 à 7 atomes de carbone, ou cycloalkyle-(C₁-C₄) alkyle,
- un alcène linéaire ou ramifié de 2 à 6 atomes de carbone,
- un aryle éventuellement substitué,
- un aralkyle éventuellement substitué dont la chaine alkyle comprend de 1 à 3 atomes de carbone,
ou forment ensemble, avec l'atome d'azote qui les porte un système cyclique choisi parmi : 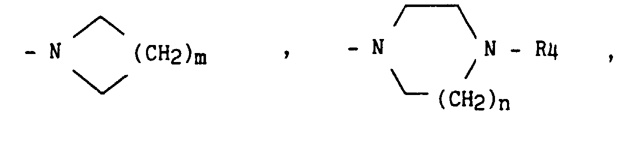 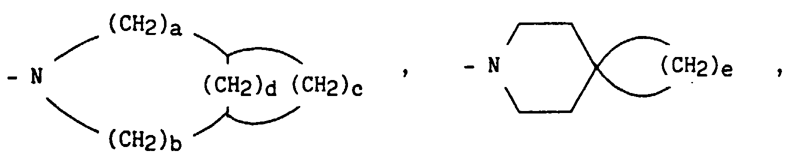 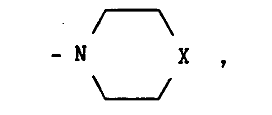
- X représente un oxygène ou un soufre,
- m, nombre entier, peut prendre les valeurs 0, 1, 2, 3, 4 ou 5,
- n, nombre entier, peut prendre les valeurs 1 ou 2,
- a et b, nombres entiers, peuvent prendre les valeurs 0, 1 ou 2 avec a + b ≠ 0,
- c et d, nombres entiers, peuvent prendre les valeurs 0, 1, 2, 3 ou 4 avec c + d ≠ 0,
- e, nombre entier peut prendre les valeurs 4 ou 5,
- R₄ représente :
- un hydrogène,
- un alkyle linéaire ou ramifié de 1 à 6 atomes de carbone,
- un aryle éventuellement substitué,
- un aralkyle éventuellement substitué dont la chaine alkyle comprend de 1 à 3 atomes de carbone,
- un groupement 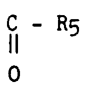 dans lequel R₅ représente un alkyle linéaire ou ramifié de 1 à 6 atomes de carbone, un phényle ou naphtyle éventuellement substitué,
- R₃ représente :
- un groupement 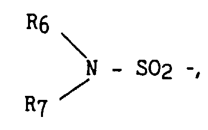
- un groupement 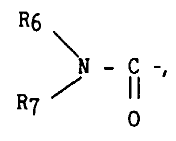
- R₆ et R₇, identiques ou différents ont la même définition que R₁ et R₂,
- de leurs isomères, énantiomères, et diastéréoisomères isolés ou sous forme de mélange,
- de leurs sels d'addition à un acide minéral ou organique pharmaceutiquement acceptable,
étant entendu que :
- par groupement aryle on entend groupement, phényle, naphtyle, pyrimidyle, pyridyle,
- les expressions aryle éventuellement substitué et aralkyle éventuellement substitué signifient que le, ou les, noyaux aromatiques peuvent être substitués par un ou plusieurs radicaux choisis parmi hydroxy, halogène, trifluorométhyle, nitro, alkyle linéaire ou ramifié de 1 à 6 atomes de carbone ou alcoxy linéaire ou ramifié de 1 à 6 atomes de carbone,
caractérisé en ce que l'on part d'un composé naphtalénique de formule (II) : 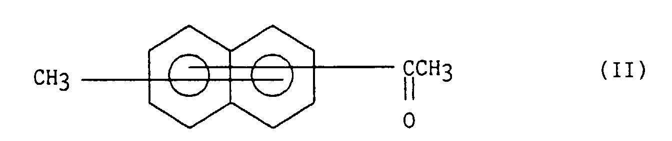 que l'on traite par du N-bromosuccinimide de manière à obtenir le composé bromé de formule (III) : 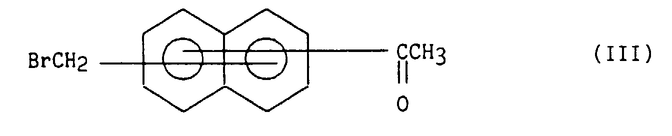 que l'on peut :
a) soit traiter par du sulfite de sodium dans un mélange acétone-eau de manière à obtenir le méthanesulfonate de formule (IV) : 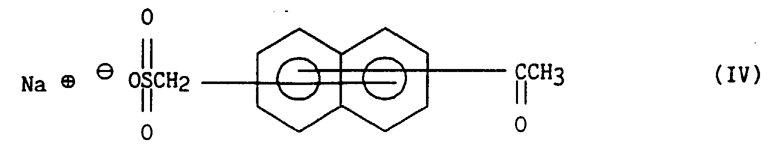 que l'on traite ensuite avec de l'oxychlorure de phosphore de manière à obtenir le chlorure de sulfonyle de formule (V) : 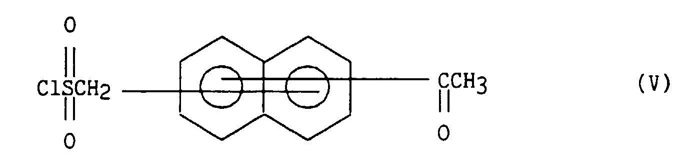 que l'on fait réagir ensuite avec une amine de formule (VI) : 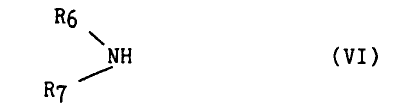 dans laquelle R₆ et R₇ ont la même signification que dans les composés de formule générale (I), de manière à obtenir le composé sulfonamidique de formule (VII) : 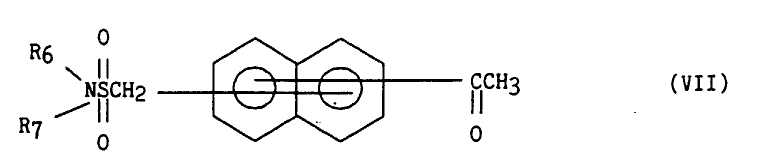 dans laquelle R₆ et R₇ ont la même signification que dans les composés de formule générale (I), que l'on traite ensuite par du brome de manière à obtenir le composé bromé de formule (VIII) : 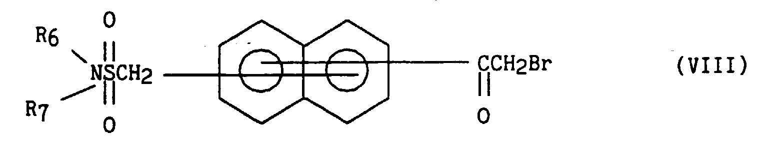 dans laquelle R₆ et R₇ ont la même signification que dans les composés de formule générale (I), que l'on réduit ensuite par du triéthylsilane dans l'acide trifluoroacétique de manière à obtenir le composé de formule (IX) : 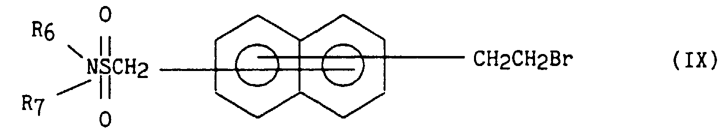 dans laquelle R₆ et R₇ ont la même signification que dans les composés de formule générale (I), que l'on l'on fait réagir :
- soit avec une amine de formule (X) : 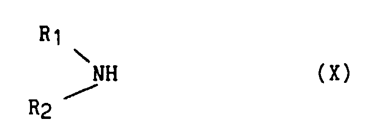 dans laquelle R₁ et R₂ ont la même signification que dans les composés de formule générale (I), de manière à obtenir le composé de formule (I_{A1}) : 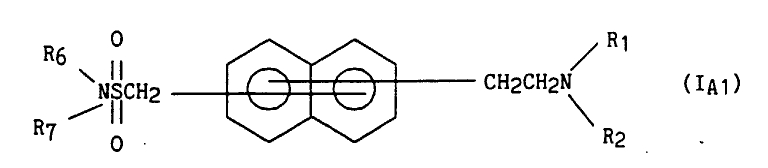 dans laquelle R₁, R₂, R₆ et R₇ ont la même signification que dans les composés de formule générale (I),
- soit avec du phtalimide potassique dans la DMF pour obtenir le composé de formule (XVII_{A}) : 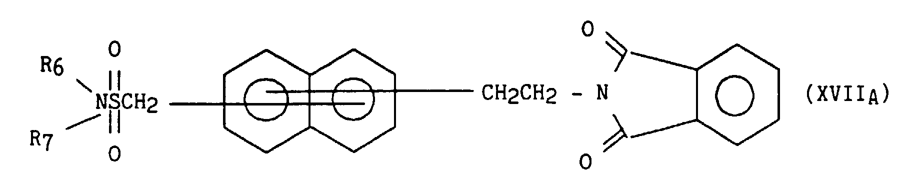 dans laquelle R₆ et R₇ ont la même signification que dans les composés de formule générale (I), que l'on fait réagir en milieu alcoolique avec de l'hydrate d'hydrazine pour obtenir l'amine primaire de formule (IA₂) 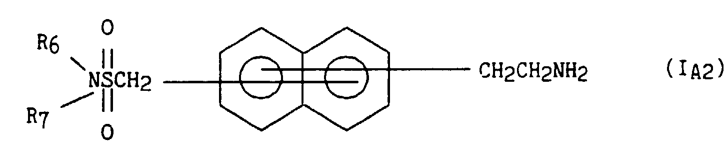 dans laquelle R₆ et R₇ ont la même signification que dans les composés de formule générale (I),
b) soit faire réagir avec un cyanure alcalin dans le DMSO ou en milieu alcoolique, de manière à obtenir le composé cyanométhylé de formule (XI) : 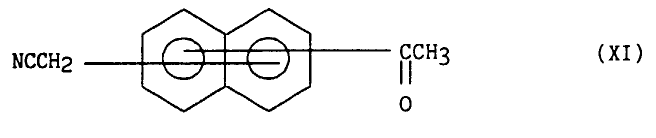 que l'on hydrolyse ensuite, soit en milieu hydroalcoolique avec un hydroxyde de métal alcalin,soit en milieu acide, de manière à obtenir l'acide naphtylacétique de formule (XII) : 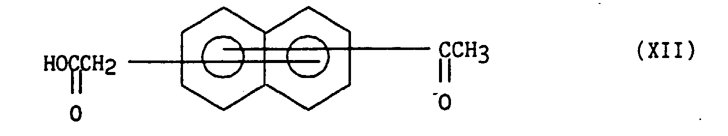 que l'on traite ensuite de manière classique par du chlorure de thionyle, de manière à obtenir le chlorure d'acide de formule (XIII) : 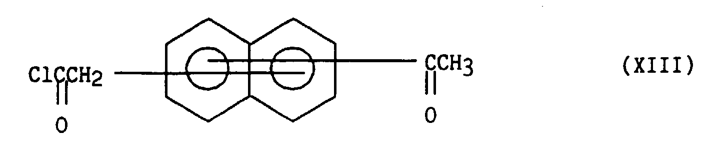 que l'on fait réagir ensuite avec une amine de formule (VI) : 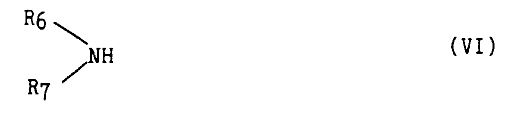 dans laquelle R₆ et R₇ ont la même signification que dans les composés de formule générale (I), de manière à obtenir le composé amidique de formule (XIV) : 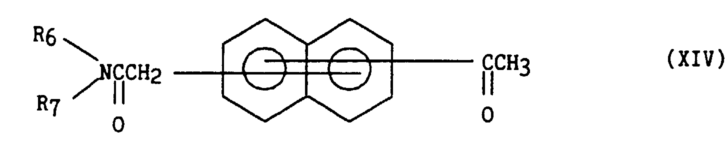 dans laquelle R₆ et R₇ ont la même signification que dans les composés de formule générale (I) que l'on traite ensuite par du brome de manière à obtenir le composé bromé de formule (XV) : 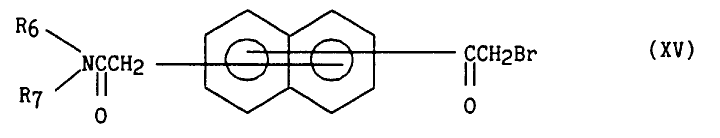 dans laquelle R₆ et R₇ ont la même signification que dans les composés de formule générale (I), que l'on réduit ensuite par du triéthylsilane dans l'acide trifluoroacétique de manière à obtenir le composé de formule (XVI) : 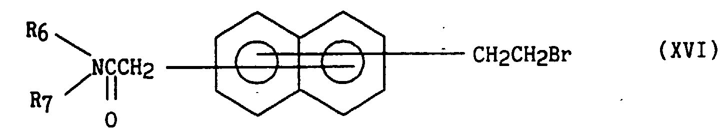 dans laquelle R₆ et R₇ ont la même signification que dans les composés de formule générale (I), que l'on fait réagir :
- soit avec une amine de formule (X) : 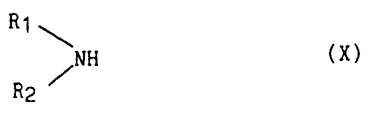 dans laquelle R₁ et R₂ ont la même signification que dans les composés de formule générale (I), de manière à obtenir le composé de formule (I_{B1}) : 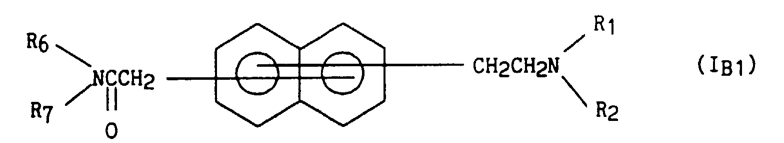 dans laquelle R₁, R₂, R₆ et R₇ ont la même signification que dans les composés de formule générale (I),
- soit avec du phtalimide potassique dans la DMF pour obtenir le composé de formule (XVII_{B}) : 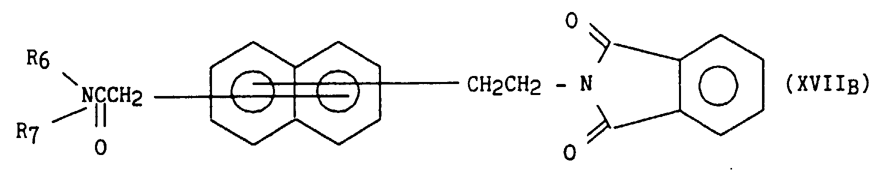 dans laquelle R₆ et R₇ ont la même signification que dans les composés de formule générale (I), que l'on fait réagir en milieu alcoolique avec de l'hydrate d'hydrazine pour obtenir l'amine primaire de formule (IB₂) 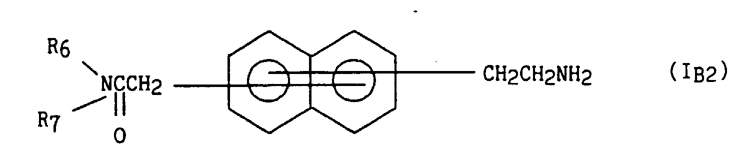 dans laquelle R₆ et R₇ ont la même signification que dans les composés de formule générale (I),
étant entendu que les composés de formules générales (I_{A1}), (I_{A2}), (I_{B1}) et (I_{B2}) représentent l'ensemble des composés de formule (I) et peuvent, si on le désire, être salifiés par un acide pharmaceutiquement acceptable et si nécessaire être séparés en leurs différents isomères.

2. Procédé de préparation selon la revendication 1 des composés de formule générale (I_{A}) : 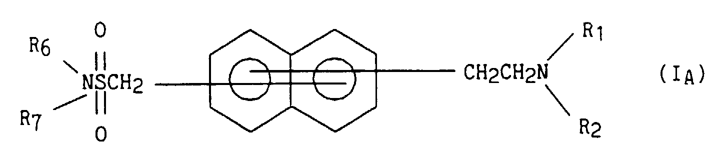 dans laquelle, les deux substituants, indépendemment l'un de l'autre, pouvant être localisés sur n'importe lequel des deux cycles du naphtalène, R₁, R₂, R₆ et R₇ sont tels que définis dans la revendication 1, de leurs isomères, énantiomères, et diastéréoisomères, isolés ou sous forme de mélange ainsi que de leurs sels d'addition à un acide minéral ou organique pharmaceutiquement acceptable.

3. Procédé de préparation selon la revendication 1 des composés de la formule générale (I_{B}) : 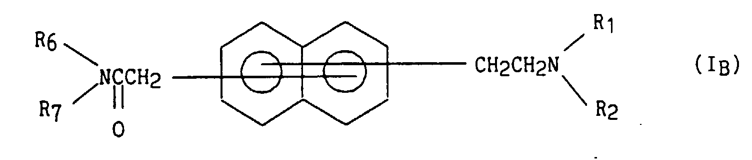 dans laquelle, les deux substituants, indépendemment l'un de l'autre, pouvant être localisés sur n'importe lequel des deux cycles du naphtalène, R₁, R₂, R₆ et R₇ sont tels que définis dans la revendication 1, de leurs isomères, énantiomères, et diastéréoisomères, isolés ou sous forme de mélange ainsi que de leurs sels d'addition à un acide minéral ou organique pharmaceutiquement acceptable.

4. Procédé de préparation selon la revendication 1 des composés pour lesquels les deux substituants se trouvent sur les sommets 1 et 7 du noyau naphtalénique et qui correspondent aux composés de formule générale suivante : 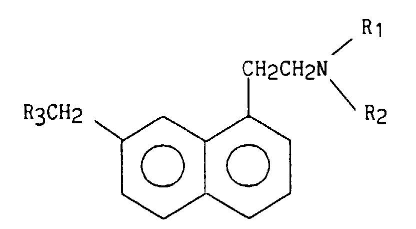 dans laquelle, R₁, R₂ et R₃ sont tels que définis dans la revendication 1, de leurs isomères, énantiomères, et diastéréoisomères, isolés ou sous forme de mélange ainsi que de leurs sels d'addition à un acide minéral ou organique pharmaceutiquement acceptable.

5. Procédé de préparation selon la revendication 1 des composés de formule générale (I) pour lesquels les deux substituants se trouvent sur les sommets 1 et 7 du noyau naphtalénique et qui correspondent aux composés de formule générale suivante : 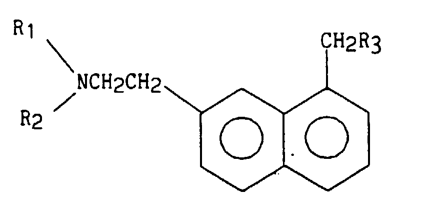 dans laquelle R₁, R₂ et R₃ sont tels que définis dans la revendication 1, de leurs isomères, énantiomères, et diastéréoisomères, isolés ou sous forme de mélange ainsi que de de leurs sels d'addition à un acide minéral ou organique pharmaceutiquement acceptable.

6. Procédé de préparation selon la revendication 1 du composé qui est le 2-{1-[2-(diméthylamino) éthyl] napht-7-yl} N-méthyl méthanesulfonamide dont la formule est représentée ci dessous, ainsi que de ses sels d'addition à un acide minéral ou organique pharmaceutiquement acceptable.

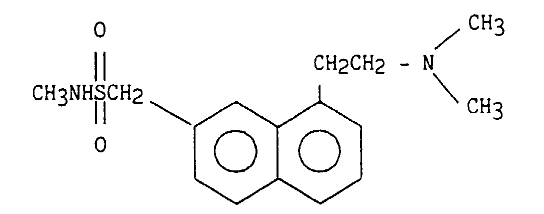

7. Procédé de préparation selon la revendication 1 du composé qui est le 2-{7-[2-(diméthylamino) éthyl] napht-1-yl} N-méthyl méthanesulfonamide dont la formule est représentée ci dessous, ainsi que de ses sels d'addition à un acide minéral ou organique pharmaceutiquement acceptable.

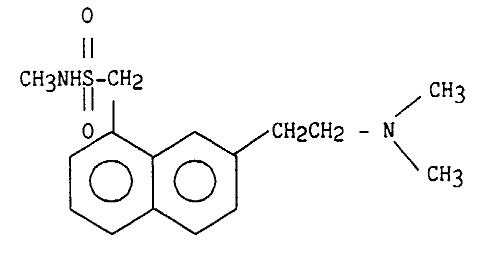

8. Procédé de préparation selon la revendication 1 du composé qui est le 2-{1-[2-(N-méthyl N-éthyl amino) éthyl] napht-7-yl} N-méthyl méthanesulfonamide dont la formule est représentée ci dessous, ainsi que de ses sels d'addition à un acide minéral ou organique pharmaceutiquement acceptable.

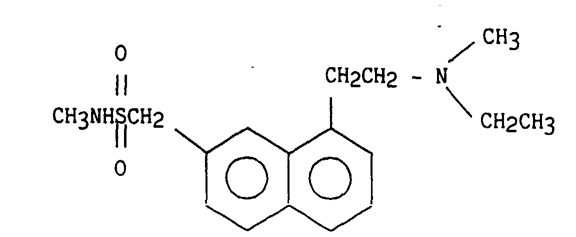

9. Procédé de préparation selon la revendication 1 du composé qui est le 2-{1-[2-(4-métatrifluorométhylphényl pipérazin-1-yl) éthyl] napht-7-yl} N-méthyl méthanesulfonamide dont la formule est représentée ci dessous, ainsi que de ses sels d'addition à un acide minéral ou organique pharmaceutiquement acceptable.

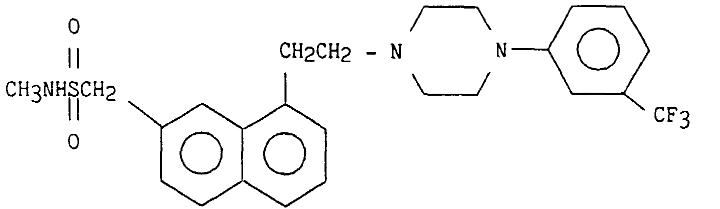

10. Procédé de préparation selon la revendication 1 du composé qui est le 2-{1-[2-(diméthyl amino) éthyl] napht-7-yl} N-méthyl acétamide dont la formule est représentée ci dessous ainsi que de ses sels d'addition à un acide minéral ou organique pharmaceutiquement acceptable.

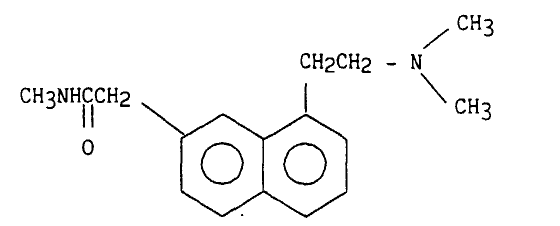

11. Procédé de préparation selon la revendication 1 du composé qui est le 2-{1-[2-(diméthyl amino) éthyl] napht-7-yl} N,N-diméthyl méthanesulfonamide dont la formule est représentée ci dessous ainsi que de ses sels d'addition à un acide minéral ou organique pharmaceutiquement acceptable.

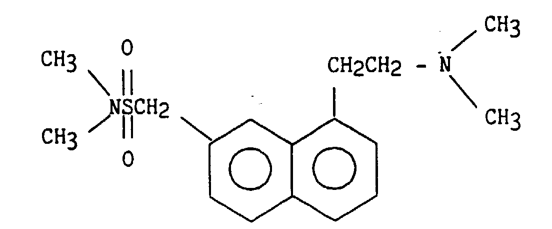

12. Procédé de préparation selon la revendication 1 du composé qui est le 2-[1-(2-piperidino éthyl) napht-7-yl] N-méthyl méthanesulfonamide dont la formule est représentée ci dessous ainsi que de ses sels d'addition à un acide minéral ou organique pharmaceutiquement acceptable.

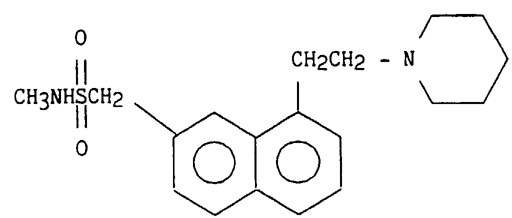

13. Procédé de préparation selon la revendication 1 du composé qui est le 2-{1-[2-(N-méthylamino) éthyl] napht-7-yl} N-méthyl méthanesulfonamide dont la formule est représentée ci dessous ainsi que de ses sels d'addition à un acide minéral ou organique pharmaceutiquement acceptable.

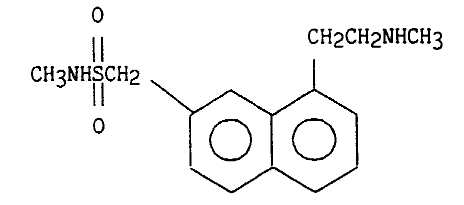

14. Procédé de préparation d'une composition pharmaceutique contenant comme principe actif au moins un composé préparé selon l'une des revendications 1 à 13 en combinaison avec un ou plusieurs excipients ou véhicules inertes, non toxiques pharmaceutiquement acceptables.

15. Procédé de préparation d'une composition pharmaceutique selon la revendication 14 utilisable pour le traitement de troubles dus à une vasodilatation du système vasculaire plus particulièrement des migraines, des céphalées, des algies vasculaires de la face, et des troubles de la circulation artérielle et veineuse.

## Patentansprüche (Patentansprüche für folgende(n) Vertragsstaat(en): AT, BE, CH, DE, DK, FR, GB, GR, IE, IT, LI, LU, NL, PT, SE)

1. Verbindungen der allgemeinen Formel (I): 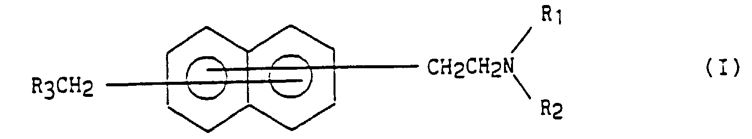 in der die beiden Substituenten unabhängig voneinander an irgendeinem der beiden Ringe des Naphthalins gebunden sind,
- R₁ und R₂, die gleichartig oder verschieden sind, jeweils unabhängig voneinander:
- ein Wasserstoffatom,
- eine geradkettige oder verzweigte Alkylgruppe mit 1 bis 6 Kohlenstoffatomen,
- eine Cycloalkylgruppe mit 3 bis 7 Kohlenstoffatomen oder eine Cycloalkyl-(C₁-C₄)-alkylgruppe,
- eine geradkettige oder verzweigte Alkengruppe mit 2 bis 6 Kohlenstoffatomen,
- eine gegebenenfalls substituierte Arylgruppe,
- eine gegebenenfalls substituierte Aralkylgruppe, deren Alkylkette 1 bis 3 Kohlenstoffatome aufweist, bedeuten,
oder gemeinsam mit dem sie tragenden Stickstoffatom ein cyclisches System ausgewählt aus: 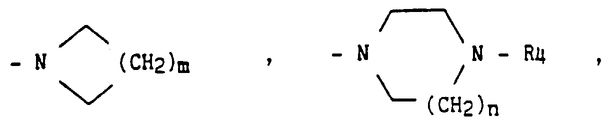 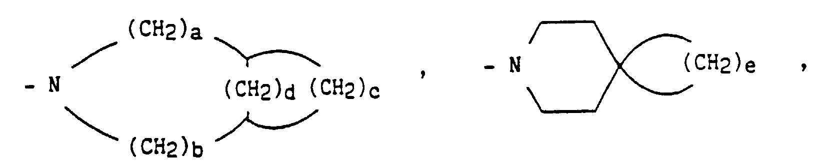 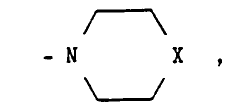 bilden, worin
- X ein Sauerstoffatom oder ein Schwefelatom,
- m eine ganze Zahl mit Werten von 0, 1, 2, 3, 4 oder 5,
- n eine ganze Zahl mit Werten von 1 oder 2,
- a und b ganze Zahlen mit Werten von 0, 1 oder 2 mit der Maßgabe, daß a + b ≠ 0 ist,
- c und d ganze Zahlen mit Werten von 0, 1, 2, 3 oder 4 mit der Maßgabe, daß c + d # 0 ist.
- e eine ganze Zahl mit Werten von 4 oder 5 und
- R₄:
- ein Wasserstoffatom,
- eine geradkettige oder verzweigte Alkylgruppe mit 1 bis 6 Kohlenstoffatomen,
- eine gegebenenfalls substituierte Arylgruppe,
- eine gegebenenfalls substituierte Aralkylgruppe, deren Alkylkette 1 bis 3 Kohlenstoffatome aufweist,
- eine Gruppe 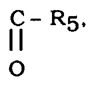 in der R₅ eine geradkettige oder verzweigte Alkylgruppe mit 1 bis 6 Kohlenstoffatomen, eine Phenylgruppe oder eine gegebenenfalls substituierte Naphthylgruppe bedeutet, darstellen, und
- R₃:
- eine Gruppe 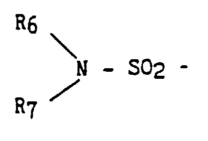 oder
- eine Gruppe 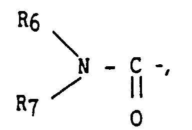
- worin R₆ und R₇, die gleichartig oder verschieden sein können, die gleiche Bedeutung besitzen, wie R₁ und R₂,
bedeutet,
- deren Isomere, Enantiomere und Diastereoisomere in isolierter Form oder in Form einer Mischung und
- deren Additionssalze mit einer anorganischen oder organischen, pharmazeutisch annehmbaren Säure,
- wobei unter einer Arylgruppe eine Phenyl-, Naphthyl-, Pyrimidyl- oder Pyridylgruppe zu verstehen ist und
- die Begriffe gegebenenfalls substituierte Arylgruppe und gegebenenfalls substituierte Aralkylgruppe bedeuten, daß der oder die aromatischen Kerne durch einen oder mehrere Reste substituiert sind. die aus Hydroxylgruppen. Halogenatomen, Trifluormethylgruppen, Nitrogruppen, geradkettigen oder verzweigten Alkylgruppen mit 1 bis 6 Kohlenstoffatomen oder geradkettigen oder verzweigten Alkoxygruppen mit 1 bis 6 Kohlenstoffatomen ausgewählt sind.

2. Verbindungen nach Anspruch 1 der allgemeinen Formel (I_{A}): 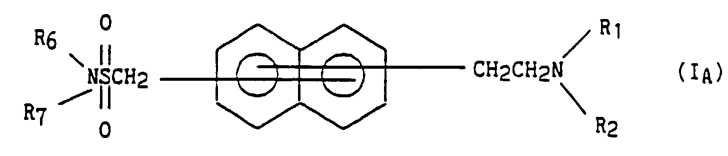 in der die beiden Substituenten unabhängig voneinander an irgendeinem der beiden Ringe des Naphthalins gebunden sein können und R₁, R₂, R₆ und R₇ die in Anspruch 1 angegebenen Bedeutungen besitzen und die gleiche Bedeutung besitzen wie bei den Verbindungen der allgemeinen Formel (I), deren Isomere, Enantiomere und Diastereoisomere in isolierter Form oder in Form einer Mischung sowie deren Additionssalze mit einer pharmazeutisch annehmbaren anorganischen oder organischen Säure.

3. Verbindungen nach Anspruch 1 der allgemeinen Formel (I_{B}): 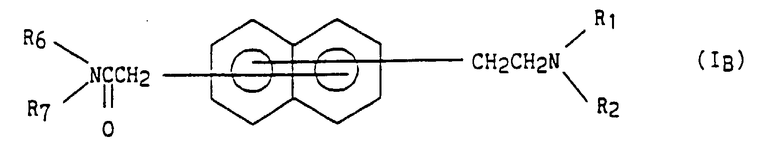 in der die beiden Substituenten unabhängig voneinander an irgendeinem der beiden Ringe des Naphthalins gebunden sein können und R₁, R₂, R₆ und R₇ die in Anspruch 1 angegebenen Bedeutungen besitzen und die gleiche Bedeutung besitzen wie bei den Verbindungen der allgemeinen Formel (I), deren Isomere, Enantiomere und Diastereoisomere in isolierter Form oder in Form einer Mischung sowie deren Additionssalze mit einer pharmazeutisch annehmbaren anorganischen oder organischen Säure.

4. Verbindungen der allgemeinen Formel (I) nach Anspruch 1, worin die beiden Substituenten sich in den Stellungen 1 und 7 des Naphthalinkerns befinden und welche den Verbindungen der folgenden allgemeinen Formel entsprechen: 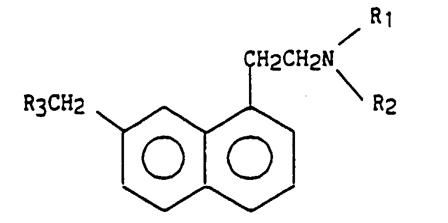 in der R₁, R₂ und R₃ die in Anspruch 1 angegebenen Bedeutungen besitzen und die gleiche Bedeutung besitzen wie bei den Verbindungen der allgemeinen Formel (I), deren Isomere. Enantiomere und Diastereoisomere in isolierter Form oder in Form einer Mischung sowie deren Additionssalze mit einer anorganischen oder organischen, pharmazeutisch annehmbaren Säure.

5. Verbindungen der allgemeinen Formel (I) nach Anspruch 1, in denen die beiden Substituenten in den Stellungen 1 und 7 des Naphthalinkerns stehen und welche den Verbindungen der allgemeinen Formel entsprechen: 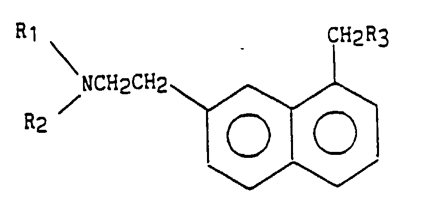 in der R₁, R₂ und R₃ die in Anspruch 1 angegebenen Bedeutungen besitzen und die gleiche Bedeutung besitzen wie bei den Verbindungen der allgemeinen Formel (I), deren Isomere. Enantiomere und Diastereoisomere in isolierter Form oder in Form einer Mischung sowie deren Additionssalze mit einer anorganischen oder organischen, pharmazeutisch annehbmaren Säure.

6. Verbindung nach Anspruch 1, nämlich 2-{1-[2-(Dimethylamino)-ethyl]-naphth,7-yl},N,methyl,methansulfonamid der Formel 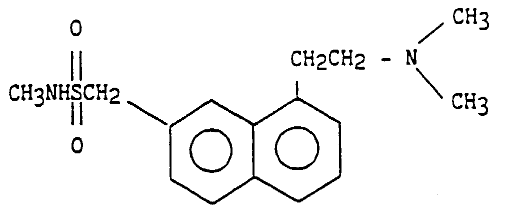 sowie dessen Additionssalze mit einer anorganischen oder organischen, pharmazeutisch annehmbaren Säure.

7. Verbindung nach Anspruch 1, nämlich 2-{7-[2-(Dimethylamino)-ethyl]-naphth-1-yl}-N-methyl-methansulfonamid der folgenden Formel 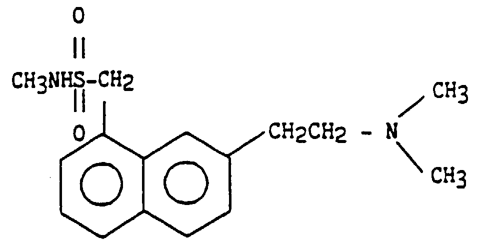 sowie dessen Additionsalze mit einer anorganischen oder organischen, pharmazeutisch annehmbaren Säure.

8. Verbindung nach Anspruch 1, nämlich 2-{1-[2-(N-Methyl-N,ethyl-amino)-ethyl]-naphth-7-yl}-N-methyl-methansulfonamid der folgenden Formel 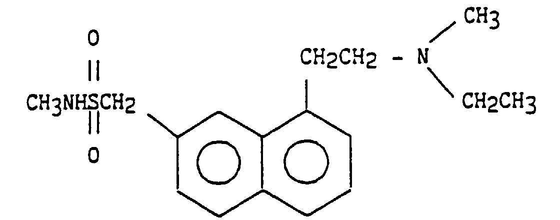 sowie dessen Additionssalze mit einer anorganischen oder organischen, pharmazeutisch annehmbaren Säure.

9. Verbindung nach Anspruch 1, nämlich 2-{1-[2-(4-meta-Trifluormethylphenyl-piperazin-1-yl),ethyl]-naphth-7,yl}-N-methyl-methansulfonamid der folgenden Formel 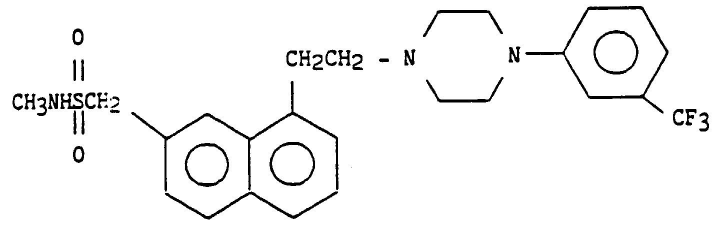 sowie dessen Additionssalze mit einer anorganischen oder organischen, pharmazeutisch annehmbaren Säure.

10. Verbindung nach Anspruch 1, nämlich 2-{1-[2-(Dimethylamino)-ethyl]-naphth-7-yl}-N-methyl-acetamid der folgenden Formel 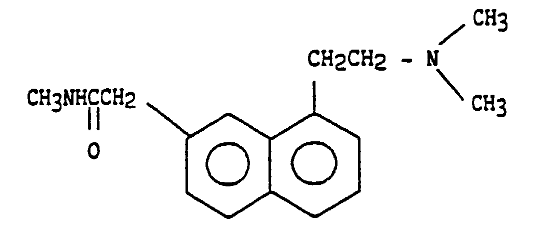 sowie dessen Additionssalze mit einer anorganischen oder organischen, pharmazeutisch annehmbaren Säure.

11. Verbindung nach Anspruch 1, nämlich 2-{1-[2-(Dimethylamino)-ethyl]-naphth-7-yl}-N,N-dimethyl-methansulfonamid der folgenden Formel 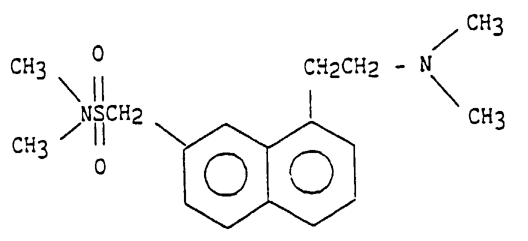 sowie dessen Additionssalze mit einer anorganischen oder organischen, pharmazeutisch annehmbaren Säure.

12. Verbindung nach Anspruch 1, nämlich 2-[1-[2-Piperidinoethyl)-naphth-7-yl]-N-methyl-methansulfonamid der folgenden Formel 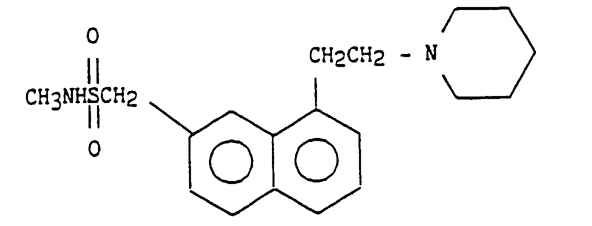 sowie dessen Additionssalze mit einer anorganischen oder organischen, pharmazeutisch annehmbaren Säure.

13. Verbindung nach Anspruch 1, nämlich 2-{1-[2-(N-Methylamino)-ethyl]-naphth-7-yl}-N-methyl-methansulfonamid der folgenden Formel 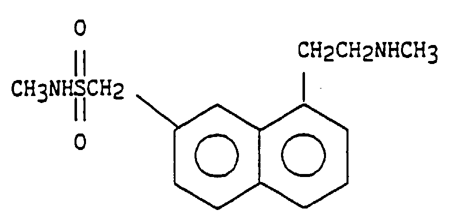 sowie dessen Additionssalze mit einer anorganischen oder organischen, pharmazeutisch annehmbaren Säure.

14. Verfahren zur Herstellung der Verbindungen der allgemeinen Formel (I), **dadurch gekennzeichnet**, daß man von einer Naphthalinverbindung der Formel (II) ausgeht: 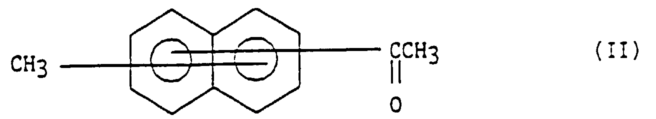 welche man mit N-Bromsuccinimid behandelt zur Bildung der Bromverbindung der Formel (III): 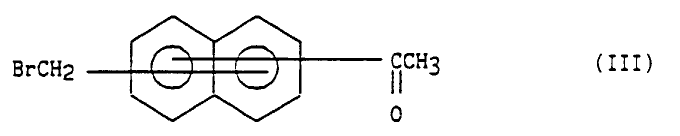 welche man:
a) entweder mit Natriumsulfit in einer Aceton/Wasser-Mischung behandeln kann zur Bildung des Methansulfonats der Formel (IV): 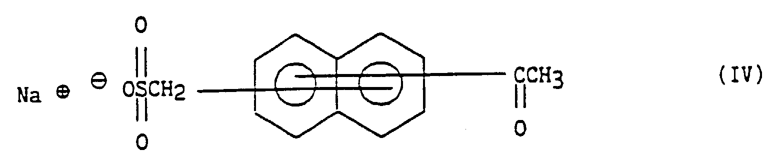 welches man anschließend mit Phosphoroxidchlorid behandelt zur Bildung des Sulfonylchlorids der Formel (V): 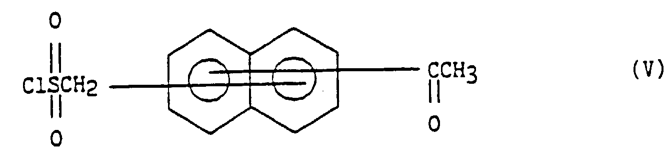 welches man anschließend mit einem Amin der Formel (VI) umsetzt: 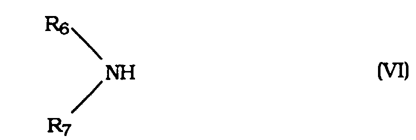 in der R₆ und R₇ die bezüglich der allgemeinen Formel (I) angegebenen Bedeutungen besitzen, so daß man die Sulfonamidverbindung der Formel (VII) erhält: 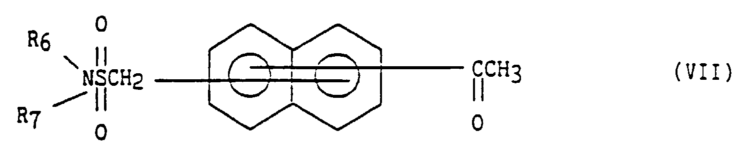 in der R₆ und R₇ die bezüglich der allgemeinen Formel (I) angegebenen Bedeutungen besitzen, welche man anschließend mit Brom behandelt zur Bildung der Bromverbindung der Formel (VIII): 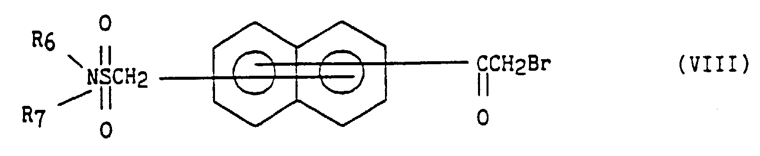 in der R₆ und R₇ die bezüglich der allgemeinen Formel (I) angegebenen Bedeutungen besitzen, welche man anschließend mit Triethylsilan in Trifluoressigsäure reduziert zur Bildung der Verbindung der Formel (IX): 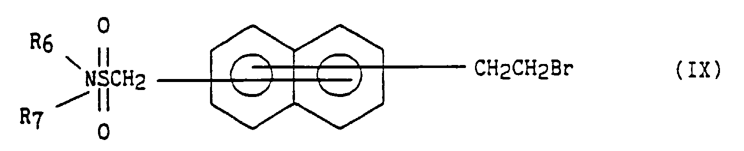 in der R₆ und R₇ die bezüglich der Verbindungen der allgemeinen Formel (I) angegebenen Bedeutungen besitzen, welche man:
- entweder mit einem Amin der Formel (X): 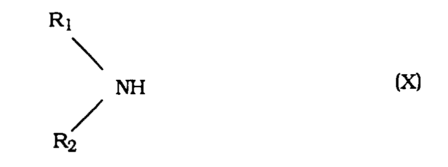 in der R₁ und R₂ die bezüglich der Verbindungen der allgemeinen Formel (I) angegebenen Bedeutungen besitzen, umsetzt zur Bildung der Verbindung der Formel (I_{A1}): 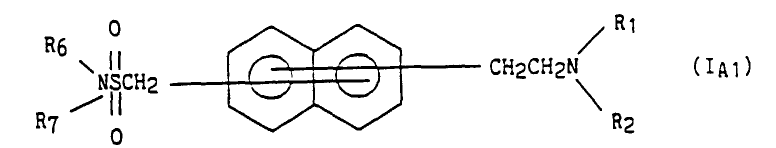 in der R₁, R₂, R₆ und R₇ die bezüglich der Verbindungen der allgemeinen Formel (I) angegebenen Bedeutungen besitzen,
- oder mit Kaliumphthalimid in DMF umsetzt zur Bildung der Verbindung der Formel (XVII_{A}): 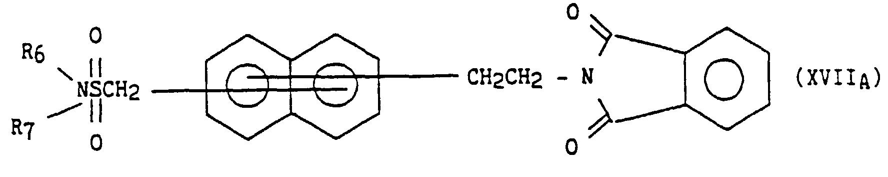 in der R₆ und R₇ die bezüglich der Verbindungen der allgemeinen Formel (I) angegebenen Bedeutungen besitzen, welche man in alkoholischem Medium mit Hydrazinhydrat umsetzt zur Bildung des primären Amins der Formel (I_{A2}): 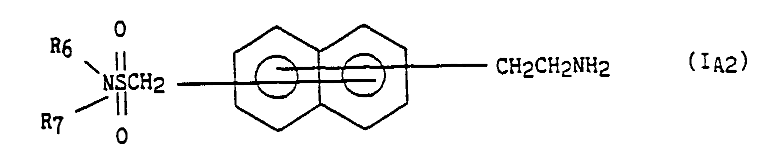 in der R₆ und R₇ die bezüglich der Verbindungen der allgemeinen Formel (I) angegebenen Bedeutungen besitzen,
b) oder mit einem Alkalimetallcyanid in DMSO oder in alkoholischem Medium umsetzt zur Bildung der Cyanomethylverbindung der Formel (XI): 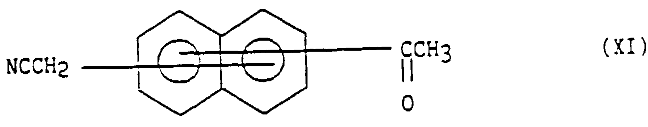 welche man anschließend entweder in wäßrig-alkoholischem Medium mit einem Alkalimetallhydroxid oder ein saurem Medium hydrolysiert zur Bildung der Naphthylessigsäure der Formel (XII): 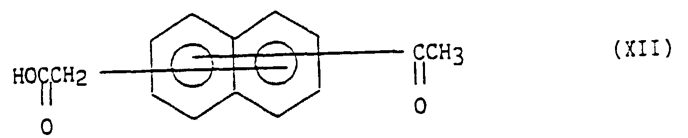 welche man anschließend in klassischer Weise mit Thionylchlorid behandelt zur Bildung des Säurechlorids der Formel (XIII): 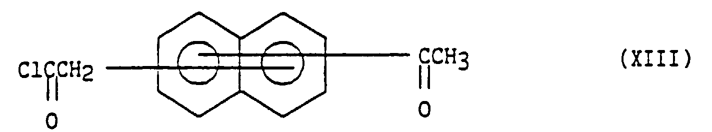 welches man anschließend mit einem Amin der Formel (VI) umsetzt: 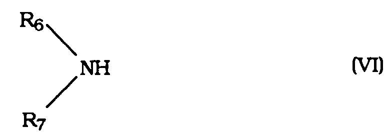 in der R₆ und R₇ die bezüglich der Verbindungen der allgemeinen Formel (I) angegebenen Bedeutungen besitzen, so daß man die Amidverbindung der Formel (XIV) erhält: 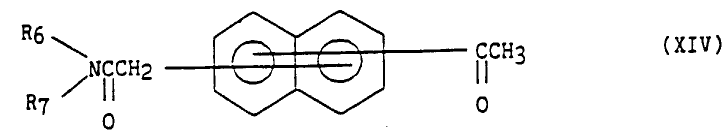 in der R₆ und R₇ die bezüglich der Verbindungen der allgemeinen Formel (I) angegebenen Bedeutungen besitzen, welche man anschließend mit Brom behandelt zur Bildung der Bromverbindung der Formel (XV): 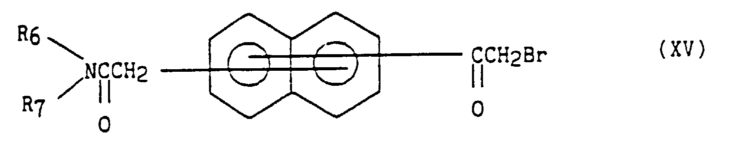 in der R₆ und R₇ die bezüglich der verbindungen der allgemeinen Formel (I) angegebenen Bedeutungen besitzen, welche man anschließend mit Triethylsilan in Trifluoressigsäure reduziert zur Bildung der Verbindung der Formel (XVI): 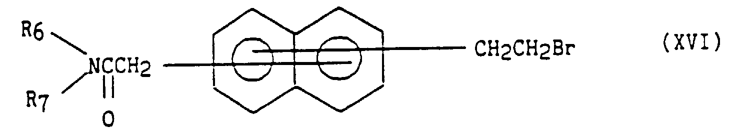 in der R₆ und R₇ die bezüglich der Verbindungen der allgemeinen Formel (I) angegebenen Bedeutungen besitzen, welche man:
- entweder mit einem Amin der Formel (X): 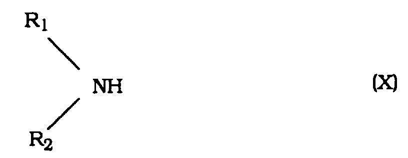 in der R₁ und R₂ die bezüglich der Verbindungen der allgemeinen Formel (I) angegebenen Bedeutungen besitzen, umsetzt zur Bildung der Verbindung der Formel (I_{B1}): 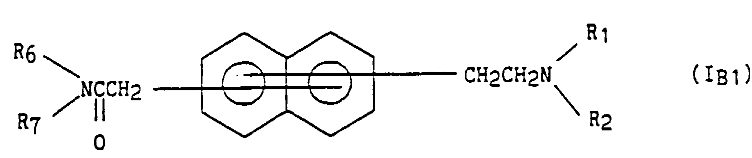 in der R₁, R₂, R₆ und R₇ die bezüglich der Verbindungen der allgemeinen Formel (I) angegebenen Bedeutungen besitzen,
- oder mit Kaliumphthalimid in DMF umsetzt zur Bildung der Verbindungen der Formel (XVII_{B}): 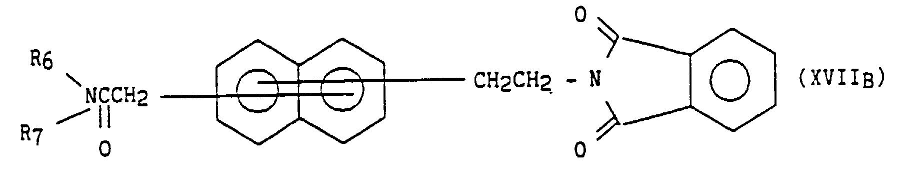 in der R₆ und R₇ die bezüglich der Verbindungen der allgemeinen Formel (I) angegebenen Bedeutungen besitzen, welche man in wäßrig-alkoholischem Medium mit Hydrazinhydrat umsetzt zur Bildung des primären Amins der Formel (I_{B2}): 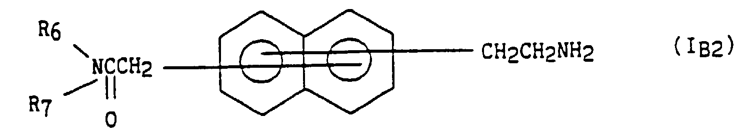 in der R₆ und R₇ die bezüglich der Verbindungen der allgemeinen Formel (I) angegebenen Bedeutungen besitzen,
wobei es sich versteht, daß die Verbindungen der allgemeinen Formeln (I_{A1}), (I_{A2}), (I_{B1}) und (I_{B2}) gemeinsam die Verbindungen der Formel (I) bilden und gewünschtenfalls mit einer pharmazeutisch annehmbaren Säure in ihre Salze überführt werden können und erforderlichenfalls in ihre verschiedenen Isomere aufgetrennt werden können.

15. Pharmazeutische Zubereitung enthaltend als Wirkstoff mindestens eine Verbindung nach einem der Ansprüche 1 bis 13 in Kombination mit einem oder mehreren inerten, nichttoxischen, pharmazeutisch annehmbaren Trägermaterialien oder Bindemitteln.

16. Pharmazeutische Zubereitung nach Anspruch 15 enthaltend mindestens einen Wirkstoff nach einem der Ansprüche 1 bis 13 zur Verwendung bei der Behandlung von Störungen als Folge einer Gefäßerweiterung des vaskulären Systems und insbesondere von Migränen, Kopfschmerzen, Gesichtsgefäßschmerzen und Störungen des arteriellen und venösen Kreislaufs.

## Patentansprüche (Patentansprüche für folgende(n) Vertragsstaat(en): ES)

1. Verfahren zur Herstellung der Verbindungen der allgemeinen Formel (I): 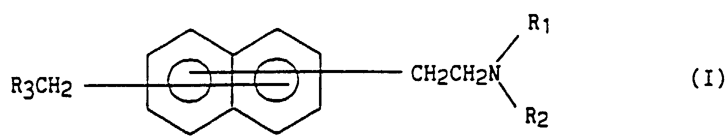 in der die beiden Substituenten unabhängig voneinander an irgendeinem der beiden Ringe des Naphthalins gebunden sind,
- R₁ und R₂, die gleichartig oder verschieden sind, jeweils unabhängig voneinander:
- ein Wasserstoffatom,
- eine geradkettige oder verzweigte Alkylgruppe mit 1 bis 6 Kohlenstoffatomen,
- eine Cycloalkylgruppe mit 3 bis 7 Kohlenstoffatomen oder eine Cycloalkyl-(C₁-C₄)-alkylgruppe,
- eine geradkettige oder verzweigte Alkengruppe mit 2 bis 6 Kohlenstoffatomen,
- eine gegebenenfalls substituierte Arylgruppe,
- eine gegebenenfalls substituierte Aralkylgruppe, deren Alkylkette 1 bis 3 Kohlenstoffatome aufweist, bedeuten,
oder gemeinsam mit dem sie tragenden Stickstoffatom ein cyclisches System ausgewählt aus: 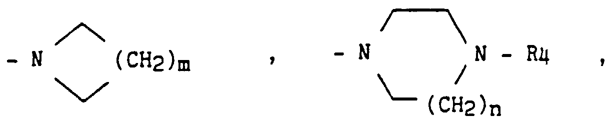 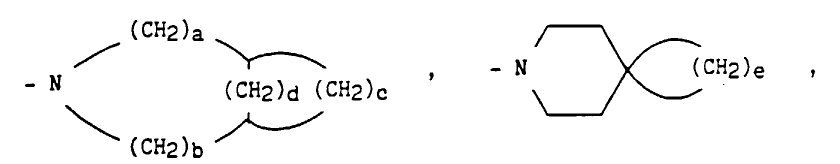 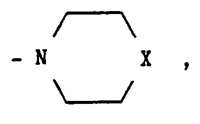 biden, worin
- X ein Sauerstoffatom oder ein Schwefelatom,
- m eine ganze Zahl mit Werten von 0, 1, 2, 3, 4 oder 5,
- n eine ganze Zahl mit Werten von 1 oder 2,
- a und b ganze Zahlen mit Werten von 0, 1 oder 2 mit der Maßgabe, daß a + b ≠ 0 ist,
- c und d ganze Zahlen mit Werten von 0, 1, 2, 3 oder 4 mit der Maßgabe, daß c + d ≠ 0 ist,
- e eine ganze Zahl mit Werten von 4 oder 5 und
- R₄:
- ein Wasserstoffatom.
- eine geradkettige oder verzweigte Alkylgruppe mit 1 bis 6 Kohlenstoffatomen,
- eine gegebenenfalls substituierte Arylgruppe,
- eine gegebenenfalls substituierte Aralkylgruppe, deren Alkylkette 1 bis 3 Kohlenstoffatome aufweist,
- eine Gruppe 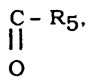 in der R₅ eine geradkettige oder verzweigte Alkylgruppe mit 1 bis 6 Kohlenstoffatomen, eine Phenylgruppe oder eine gegebenenfalls substituierte Naphthylgruppe bedeutet, darstellen, und
- R₃:
- eine Gruppe 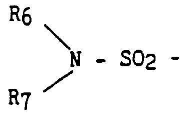 oder
- eine Gruppe 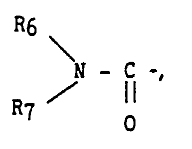
- worin R₆ und R₇, die gleichartig oder verschieden sein können, die gleiche Bedeutung besitzen, wie R₁ und R₂,
bedeutet,
- von deren Isomeren, Enantiomeren und Diastereoisomeren in isolierter Form oder in Form einer Mischung, und
- von deren Additionssalzen mit einer anorganischen oder organischen, pharmazeutisch annehmbaren Säure,
- wobei unter einer Arylgruppe eine Phenyl-, Naphthyl-, Pyrimidyl- und Pyridylgruppe zu verstehen ist und
- die Begriffe gegebenenfalls substituierte Arylgruppe und gegebenenfalls substituierte Aralkylgruppe bedeuten, daß der oder die aromatischen Kerne durch einen oder mehrere Reste substituiert sein können, die aus Hydroxylgruppen, Halogenatomen, Trifluormethylgruppen, Nitrogruppen, geradkettigen oder verzweigten Alkylgruppen mit 1 bis 6 Kohlenstoffatomen oder geradkettigen oder verzweigten Alkoxygruppen mit 1 bis 6 Kohlenstoffatomen ausgewählt sind,
**dadurch gekennzeichnet**, daß man von einer Naphthalinverbindung der Formel (II) ausgeht: 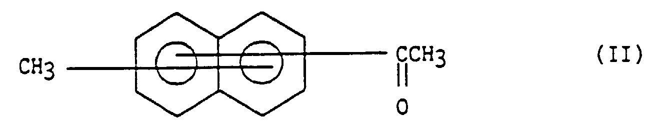 welche man mit N-Bromsuccinimid behandelt zur Bildung der Bromverbindung der Formel (III): 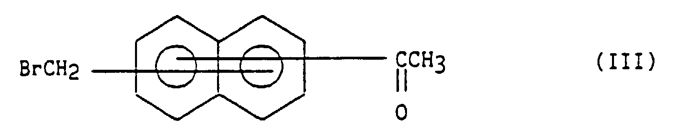 welche man:
a) entweder mit Natriumsulfit in einer Aceton/Wasser-Mischung behandeln kann zur Bildung des Methansulfonats der Formel (IV): 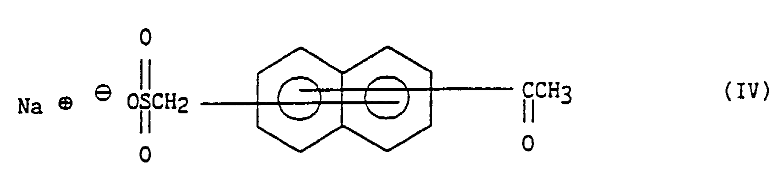 welches man anschließend mit Phosphoroxidchlorid behandelt zur Bildung des Sulfonylchlorids der Formel (V): 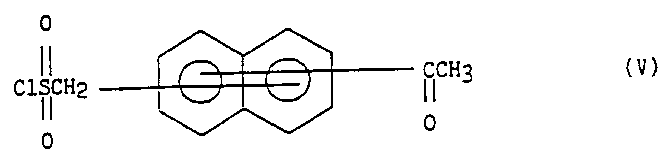 welches man anschließend mit einem Amin der Formel (VI) umsetzt: 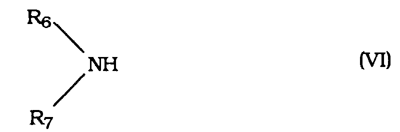 in der R₆ und R₇ die bezüglich der allgemeinen Formel (I) angegebenen Bedeutungen besitzen, so daß man die Sulfonamidverbindung der Formel (VII) erhält: 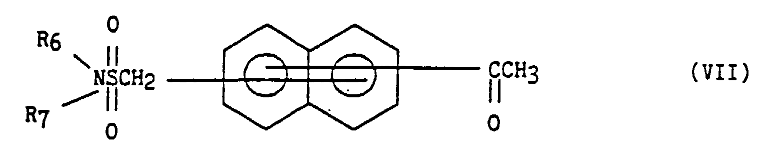 in der R₆ und R₇ die bezüglich der allgemeinen Formel (I) angegebenen Bedeutungen besitzen, welche man anschließend mit Brom behandelt zur Bildung der Bromverbindung der Formel (VIII): 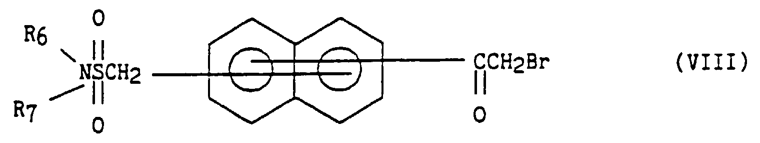 in der R₆ und R₇ die bezüglich der allgemeinen Formel (I) angegebenen Bedeutungen besitzen, welche man anschließend mit Triethylsilan in Trifluoressigsäure reduziert zur Bildung der Verbindung der Formel (IX): 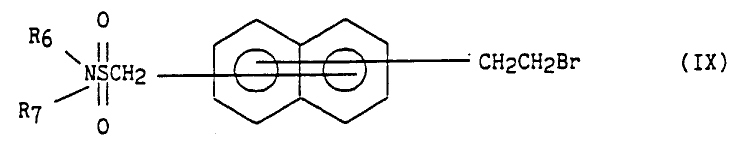 in der R₆ und R₇ die bezüglich der Verbindungen der allgemeinen Formel (I) angegebenen Bedeutungen besitzen, welche man:
- entweder mit einem Amin der Formel (X): 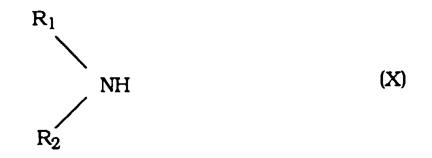 in der R₁ und R₂ die bezüglich der Verbindungen der allgemeinen Formel (I) angegebenen Bedeutungen besitzen, umsetzt zur Bildung der Verbindung der Formel (I_{A1}): 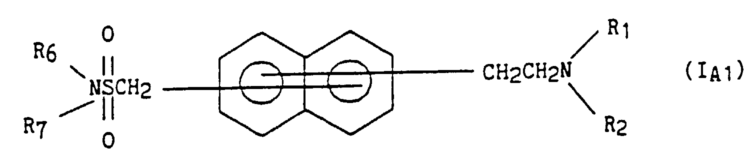 in der R₁, R₂, R₆ und R₇ die bezüglich der Verbindungen der allgemeinen Formel (I) angegebenen Bedeutungen besitzen,
- oder mit Kaliumphthalimid in DMF umsetzt zur Bildung der Verbindung der Formel (XVII_{A}): 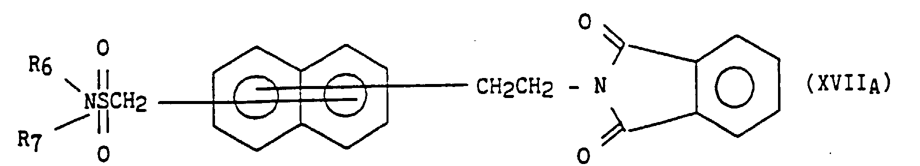 in der R₆ und R₇ die bezüglich der Verbindungen der allgemeinen Formel (I) angegebenen Bedeutungen besitzen, welche man in alkoholischem Medium mit Hydrazinhydrat umsetzt zur Bildung des primären Amins der Formel (I_{A2}): 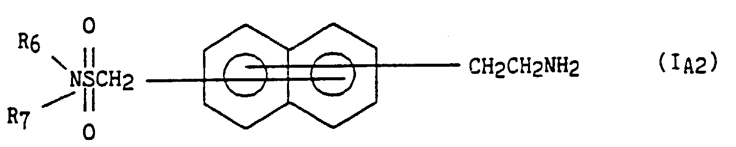 in der R₆ und R₇ die bezüglich der Verbindungen der allgemeinen Formel (I) angegebenen Bedeutungen besitzen,
b) oder mit einem Alkalimetallcyanid in DMSO oder in alkoholischem Medium umsetzt zur Bildung der Cyanomethylverbindung der Formel (XI): 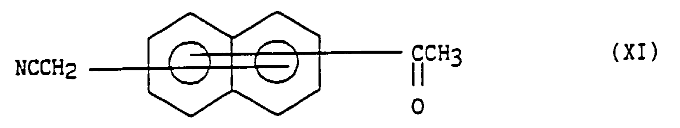 welche man anschließend entweder in wäßrig-alkoholischem Medium mit einem Alkalimetallhydroxid oder ein saurem Medium hydrolysiert zur Bildung der Naphthylessigsäure der Formel (XII): 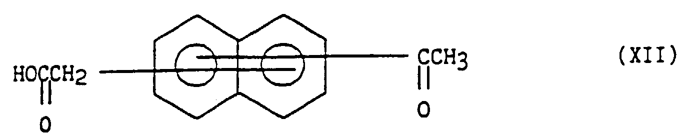 welche man anschließend in klassischer Weise mit Thionylchlorid behandelt zur Bildung des Säurechlorids der Formel (XIII): 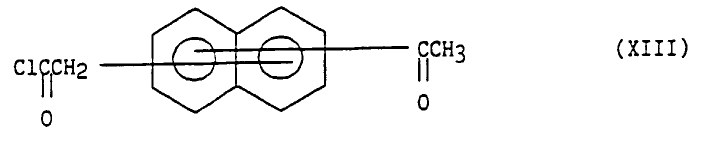 welches man anschließend mit einem Amin der Formel (VI) umsetzt: 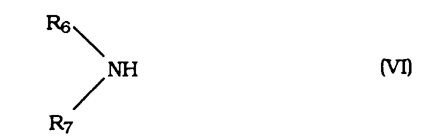 in der R₆ und R₇ die bezüglich der Verbindungen der allgemeinen Formel (I) angegebenen Bedeutungen besitzen, so daß man die Amidverbindung der Formel (XIV) erhält: 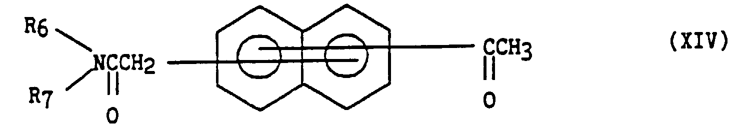 in der R₆ und R₇ die bezüglich der Verbindungen der allgemeinen Formel (I) angegebenen Bedeutungen besitzen, welche man anschließend mit Brom behandelt zur Bildung der Bromverbindung der Formel (XV): 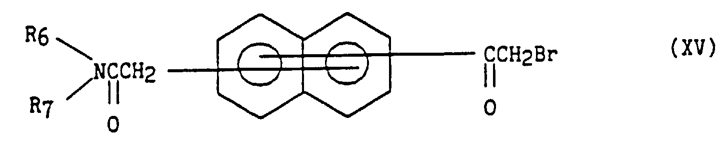 in der R₆ und R₇ die bezüglich der Verbindungen der allgemeinen Formel (I) angegebenen Bedeutungen besitzen, welche man anschließend mit Triethylsilan in Trifluoressigsäure reduziert zur Bildung der Verbindung der Formel (XVI): 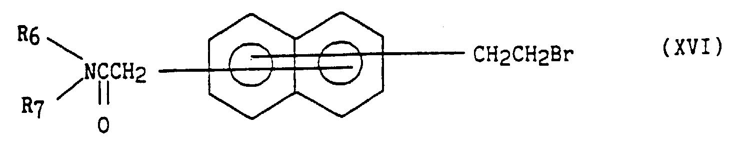 in der R₆ und R₇ die bezüglich der Verbindungen der allgemeinen Formel (I) angegebenen Bedeutungen besitzen, welche man:
- entweder mit einem Amin der Formel (X): 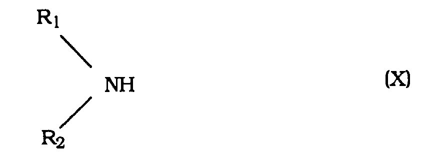 in der R₁ und R₂ die bezüglich der Verbindungen der allgemeinen Formel (I) angegebenen Bedeutungen besitzen, umsetzt zur Bildung der Verbindung der Formel (I_{B1}): 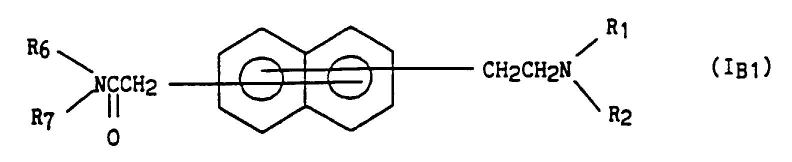 in der R₁, R₂, R₆ und R₇ die bezüglich der Verbindungen der allgemeinen Formel (I) angegebenen Bedeutungen besitzen,
- oder mit Kaliumphthalimid in DMF umsetzt zur Bildung der Verbindungen der Formel (XVII_{B}): 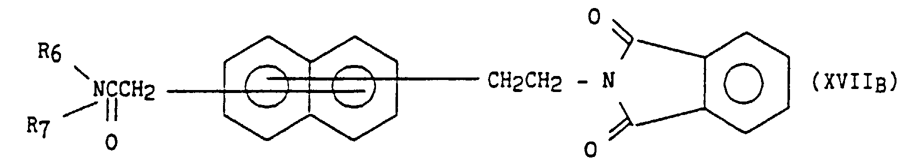 in der R₆ und R₇ die bezüglich der Verbindungen der allgemeinen Formel (I) angegebenen Bedeutungen besitzen, welche man in wäßrig-alkoholischem Medium mit Hydrazinhydrat umsetzt zur Bildung des primären Amins der Formel (I_{B2}): 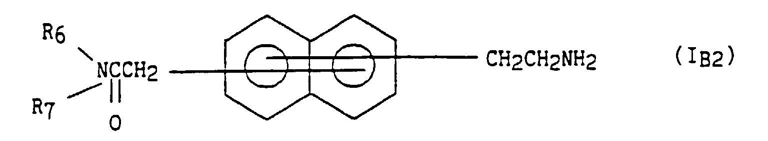 in der R₆ und R₇ die bezüglich der Verbindungen der allgemeinen Formel (I) angegebenen Bedeutungen besitzen,
wobei es sich versteht, daß die Verbindungen der allgemeinen Formeln (I_{A1}), (I_{A2}), (I_{B1}) und (I_{B2}) gemeinsam die Verbindungen der Formel (I) bilden und gewünschtenfalls mit einer pharmazeutisch annehmbaren Säure in ihre Salze überführt werden können und erforderlichenfalls in ihre verschiedenen Isomere aufgetrennt werden können.

2. Verfahren nach Anspruch 1 zur Herstellung der Verbindungen der allgemeinen Formel (I_{A}): 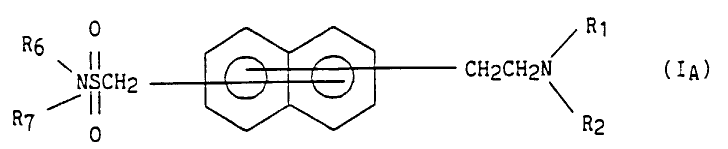 worin die beiden Substituenten unabhängig voneinander an irgendeinem der beiden Ringe des Naphthalins stehen können und R₁, R₂, R₆ und R₇ die in Anspruch 1 angegebenen Bedeutungen besitzen. von deren Isomeren, Enantiomeren und Diastereoisomeren in isolierter Form oder in Form einer Mischung sowie von deren Additionssalzen mit einer anorganischen oder organischen, pharmazeutisch annehmbaren Säure.

3. Verfahren nach Anspruch 1 zur Herstellung der Verbindungen der allgemeinen Formel (I_{B}): 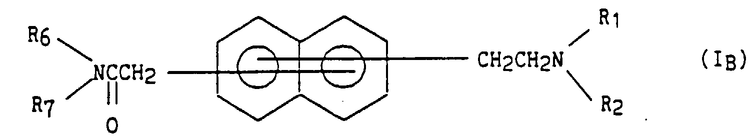 in der die beiden Substituenten unabhängig voneinander an irgendeinem der beiden Ringe des Naphthalins stehen können und R₁, R₂, R₆ und R₇ die in Anspruch 1 angegebenen Bedeutungen besitzen, von deren Isomeren, Enantiomeren und Diastereoisomeren in isolierter Form oder in Form einer Mischung sowie von deren Additionssalzen mit einer anorganischen oder organischen, pharmazeutisch annehmbaren Säure.

4. Verfahren nach Anspruch 1 zur Herstellung von Verbindungen, bei denen die beiden Substituenten in den Stellungen 1 und 7 des Naphthalinkerns stehen und die der folgenden allgemeinen Formel entsprechen: 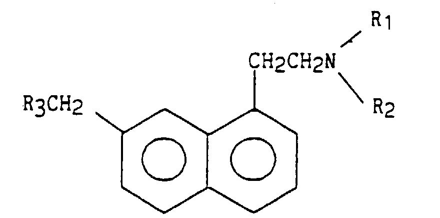 in der R₁, R₂ und R₃ die in Anspruch 1 angegebenen Bedeutungen besitzen, von deren Isomeren, Enantiomeren und Diastereoisomeren in isolierter Form oder in Form einer Mischung sowie von deren Additionssalzen mit einer anorganischen oder organischen, pharmazeutisch annehmbaren Säure.

5. Verfahren nach Anspruch 1 zur Herstellung der Verbindungen der allgemeinen Formel (I), in denen die beiden Substituenten in den Stellungen 1 und 7 des Naphthalinkerns stehen und die der folgenden allgemeinen Formel entsprechen: 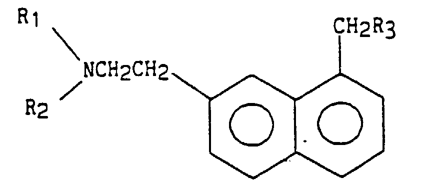 in der R₁, R₂ und R₃ die in Anspruch 1 angegebenen Bedeutungen besitzen, von deren Isomeren, Enantiomeren und Diastereoisomeren in isolierter Form oder in Form einer Mischung sowie von deren Additionssalzen mit einer anorganischen oder organischen, pharmazeutisch annehmbaren Säure.

6. Verfahren nach Anspruch 1 zur Herstellung der Verbindung 2-{1-[2-(Dimethylamino)-ethyl]-naphth-7-yl}-N-methyl-methansulfonamid der folgenden Formel: 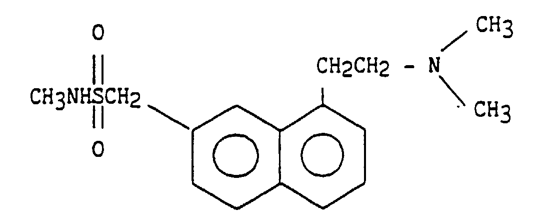 sowie von dessen Additionssalzen mit einer anorganischen oder organischen, pharmazeutisch annehmbaren Säure.

7. Verfahren nach Anspruch 1 zur Herstellung der Verbindung 2-{7-[2-(Dimethylamino)-ethyl]-naphth-1-yl}-N-methyl-methansulfonamid der folgenden Formel: 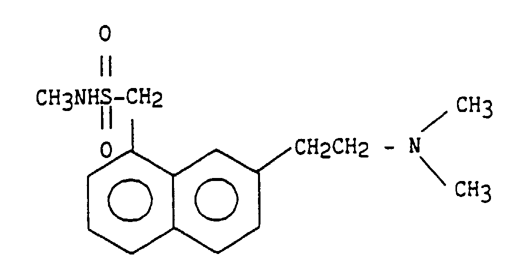 sowie von dessen Additionssalzen mit einer anorganischen oder organischen, pharmazeutisch annehmbaren Säure.

8. Verfahren nach Anspruch 1 zur Herstellung der Verbindung 2-{1-[2-(N-Methyl-N-ethylamino)-ethyl]-naphth-7-yl}-N methyl-methansulfonamid der folgenden Formel: 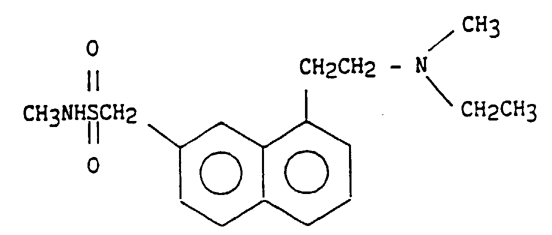 sowie von dessen Additionssalzen mit einer anorganischen oder organischen, pharmazeutisch annehmbaren Säure.

9. Verfahren nach Anspruch 1 zur Herstellung der Verbindung 2-{1-[2-(4-meta-Trifluormethylphenyl-piperazin-1-yl)-ethyl]-naphth-7-yl}-N-methyl-methansulfonamid der folgenden Formel: 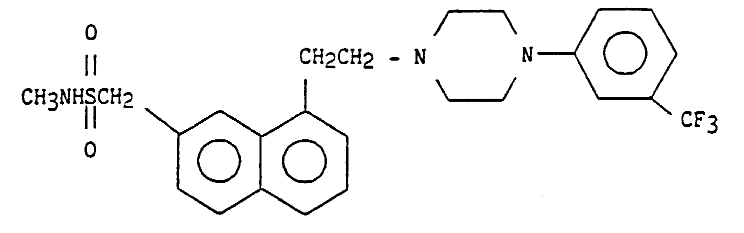 sowie von dessen Additionssalzen mit einer anorganischen oder organischen, pharmazeutisch annehmbaren Säure.

10. Verfahren nach Anspruch 1 zur Herstellung der Verbindung 2-{1-[2-(Dimethylamino)-ethyl]-naphth-7-yl}-N-methyl-acetamid der folgenden Formel: 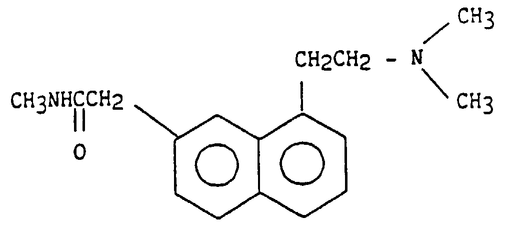 sowie von dessen Additionssalzen mit einer anorganischen oder organischen, pharmazeutisch annehmbaren Säure.

11. Verfahren nach Anspruch 1 zur Herstellung der Verbindung 2-{1-[2-(Dimethylamino)-ethyl]-naphth-7-yl},N-N-dimethyl-methansulfonamid der folgenden Formel: 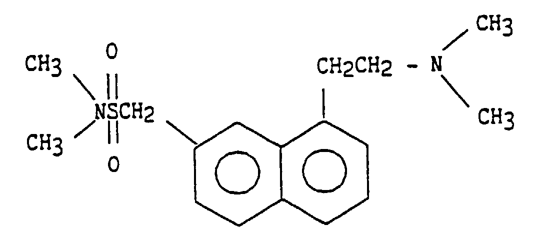 sowie von dessen Additionssalzen mit einer anorganischen oder organischen, pharmazeutisch annehmbaren Säure.

12. Verfahren nach Anspruch 1 zur Herstellung der Verbindung 2-[1-(2-Piperidinoethyl)-naphth-7-yl}-N-methyl-methansulfonamid der folgenden Formel: 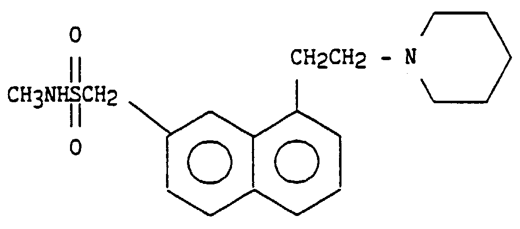 sowie von dessen Additionssalzen mit einer anorganischen oder organischen, pharmazeutisch annehmbaren Säure.

13. Verfahren nach Anspruch 1 zur Herstellung der Verbindung 2-{1-[2-(N-Methylamino)-ethyl]-naphth-7-yl}-N-methyl-methansulfonamid der folgenden Formel: 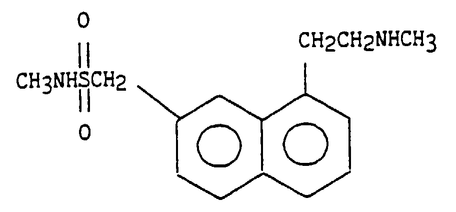 sowie von dessen Additionssalzen mit einer anorganischen oder organischen, pharmazeutisch annehmbaren Säure.

14. Verfahren zur Herstellung einer pharmazeutischen Zubereitung enthaltend als Wirkstoff mindestens eine Verbindung hergestellt nach einem der Ansprüche 1 bis 13 in Kombination mit einem oder mehreren inerten, nichttoxischen, pharmazeutisch annehmbaren Trägermaterialien oder Bindemitteln.

15. Verfahren zur Herstellung einer pharmazeutischen Zubereitung nach Anspruch 14 zur Verwendung bei der Behandlung von Störungen als Folge einer Gefäßerweiterung des vaskulären Systems und insbesondere von Migräne, Kopfschmerzen, Gesichtsgefäßschmerzen und Störungen des arteriellen und venösen Kreislaufs.

## Claims (Claims for the following Contracting State(s): AT, BE, CH, DE, DK, FR, GB, GR, IE, IT, LI, LU, NL, PT, SE)

1. Compounds of the general formula (I) 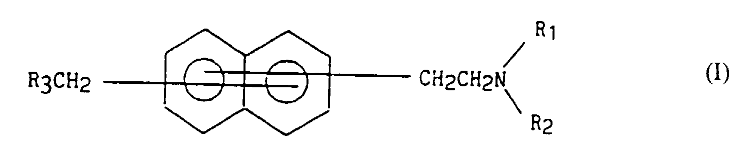 in which the two substituents, each independently of the other, may be located on either of the two rings of the naphthalene,
- R₁ and R₂, which are identical or different, each represents, independently of the other:
- a hydrogen atom,
- a linear or branched alkyl radical having from 1 to 6 carbon atoms,
- a cycloalkyl radical having from 3 to 7 carbon atoms, or a cycloalkyl-(C₁-C₄)-alkyl radical,
- a linear or branched alkenyl radical having from 2 to 6 carbon atoms,
- an optionally substituted aryl radical,
- an optionally substituted aralkyl radical in which the alkyl chain contains from 1 to 3 carbon atoms,
or, with the nitrogen atom carrying them, together form a ring system selected from : 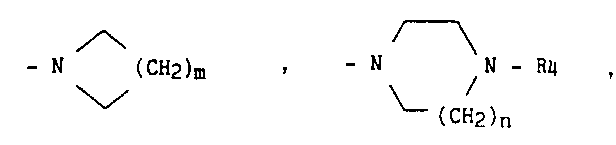 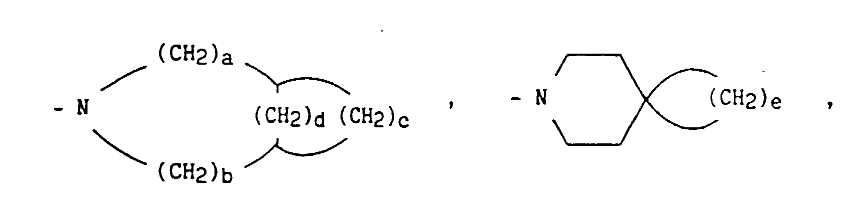 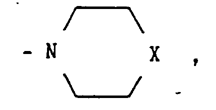
- X represents an oxygen or sulphur atom,
- m, an integer, may be 0, 1, 2, 3, 4 or 5,
- n, an integer, may be 1 or 2,
- a and b, integers, may be 0, 1, or 2 with a + b ≠ 0,
- c and d, integers, may be 0, 1, 2, 3 or 4, with c + d ≠ 0,
- e, an integer, may be 4 or 5,
- R₄ represents :
- a hydrogen atom,
- a linear or branched alkyl radical having from 1 to 6 carbon atoms,
- an optionally substituted aryl radical,
- an optionally substituted aralkyl radical in which the alkyl chain contains from 1 to 3 carbon atoms,
- a group 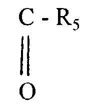 wherein R₅ represents a linear or branched alkyl radical having from 1 to 6 carbon atoms, or an optionally substituted phenyl or naphthyl radical,
- R₃ represents :
- a group 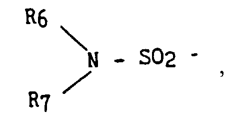
- a group 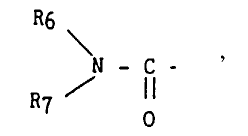
- R₆ and R₇, which are identical or different, have the same meanings as R₁ and R₂,
- their isomers, enantiomers and diastereoisomers, isolated or in the form of a mixture,
- the addition salts thereof with a pharmaceutically acceptable mineral or organic acid,
- there is understood by "aryl group" phenyl, naphthyl, pyrimidyl or pyridyl,
- the expressions "optionally substituted aryl" and "optionally substituted aralkyl" denote that the aromatic nucleus or nuclei may be substituted by one or more radicals selected from hydroxy, halogen, trifluoromethyl, nitro, linear or branched alkyl having from 1 to 6 carbon atoms and linear or branched alkoxy having from 1 to 6 carbon atoms.

2. Compounds according to claim 1 of the general formula (I_{A}) 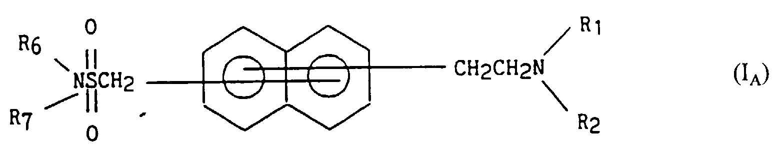 in which the two substituents, each independently of the other, may be located on either of the two rings of the naphthalene, and R₁, R₂, R₆ and R₇ are as defined in claim 1 and have the same meanings as in the compounds of the general formula (I), their isomers, enantiomers and diastereoisomers, isolated or in the form of a mixture, and also the addition salts thereof with a pharmaceutically acceptable mineral or organic acid.

3. Compounds according to claim 1 of the general formula (I_{B}) 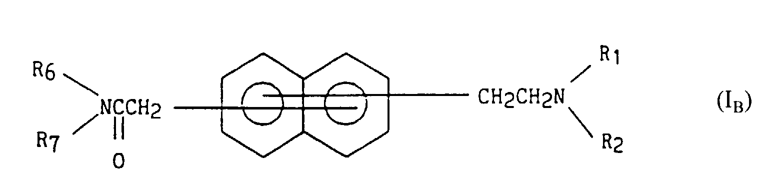 in which the two substituents, each independently of the other, may be located on either of the two rings of the naphthalene, and R₁, R₂, R₆ and R₇ are as defined in claim 1 and have the same meanings as in the compounds of the general formula (I), their isomers, enantiomers and diastereoisomers, isolated or in the form of a mixture, and also the addition salts thereof with a pharmaceutically acceptable mineral or organic acid.

4. Compounds of the general formula (I) according to claim 1 in which the two substituents are located at positions 1 and 7 of the naphthalene nucleus and which correspond to compounds of the following general formula : 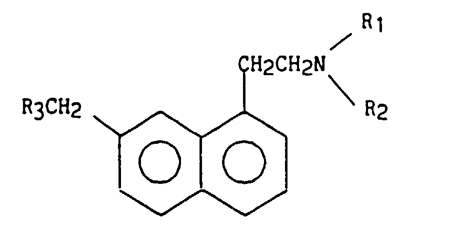 in which R₁, R₂ and R₃ are as defined in claim 1 and have the same meanings as in the compounds of the general formula (I), their isomers, enantiomers and diastereoisomers, isolated or in the form of a mixture, and also the addition salts thereof with a pharmaceutically acceptable mineral or organic acid.

5. Compounds of the general formula (I) according to claim 1 in which the two substituents are located at positions 1 and 7 of the naphthalene nucleus and which correspond to compounds of the following general formula : 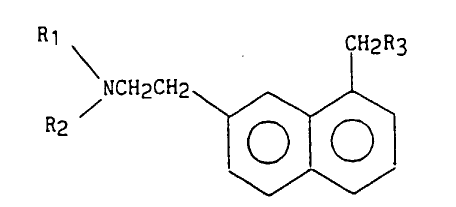 in which R₁, R₂ and R₃ are as defined in claim 1 and have the same meanings as in the compounds of the general formula (I), their isomers, enantiomers and diastereoisomers, isolated or in the form of a mixture, and also the addition salts thereof with a pharmaceutically acceptable mineral or organic acid.

6. Compound according to claim 1 which is 2-{1-[2-(dimethylamino)ethyl]naphth-7-yl}-N-methylmethanesulphonamide of which the formula is represented below, and also the addition salts thereof with a pharmaceutically acceptable mineral or organic acid.

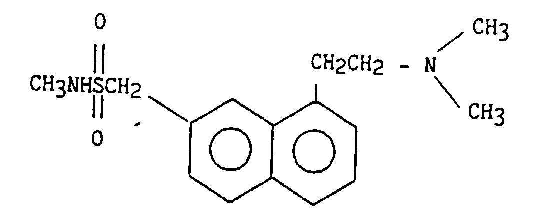

7. Compound according to claim 1 which is 2-{7-[2-(dimethylamino)ethyl]naphth-1-yl}-N-methylmethanesulphonamide of which the formula is represented below, and also the addition salts thereof with a pharmaceutically acceptable mineral or organic acid.

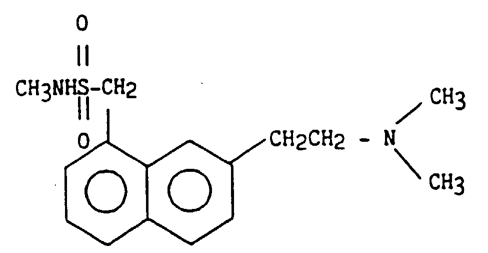

8. Compound according to claim 1 which is 2-{1-[2-(N-methyl-N-ethylamino)ethyl]-naphth-7-yl}-N-methylmethanesulphonamide of which the formula is represented below, and also the addition salts thereof with a pharmaceutically acceptable mineral or organic acid.

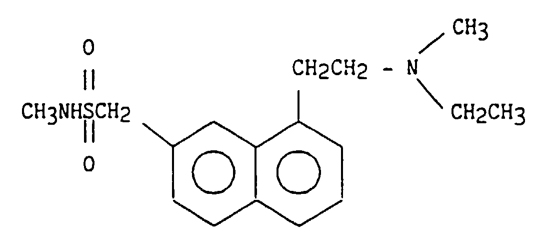

9. Compound according to claim 1 which is 2-{1-[2-(4-meta-trifluoromethylphenylpiperazin-1-yl)ethyl]naphth-7-yl}-N-methylmethanesulphonamide of which the formula is represented below, and also the addition salts thereof with a pharmaceutically acceptable mineral or organic acid.

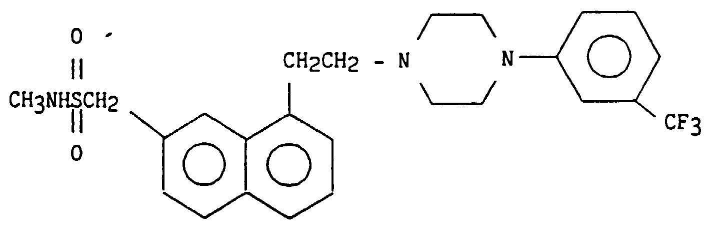

10. Compound according to claim 1 which is 2-{1-[2-(dimethylamino)ethyl]naphth-7-yl}-N-methylacetamide of which the formula is represented below, and also the addition salts thereof with a pharmaceutically acceptable mineral or organic acid.

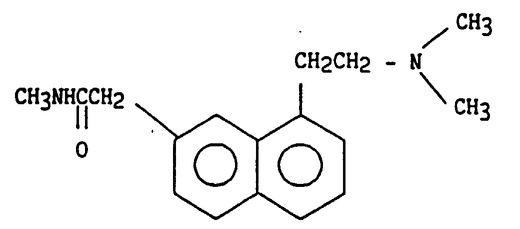

11. Compound according to claim 1 which is 2-{1-[2-(dimethylamino)ethyl]naphth-7-yl}-N,N-dimethylmethanesulphonamide of which the formula is represented below, and also the addition salts thereof with a pharmaceutically acceptable mineral or organic acid.

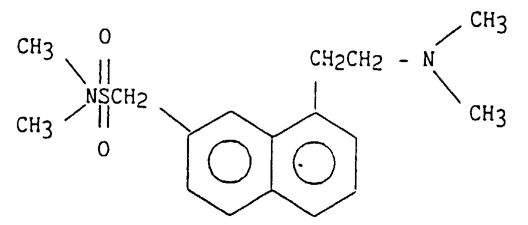

12. Compound according to claim 1 which is 2-[1-(2-piperidinoethyl]naphth-7-yl}-N-methylmethanesulphonamide of which the formula is represented below, and also the addition salts thereof with a pharmaceutically acceptable mineral or organic acid.

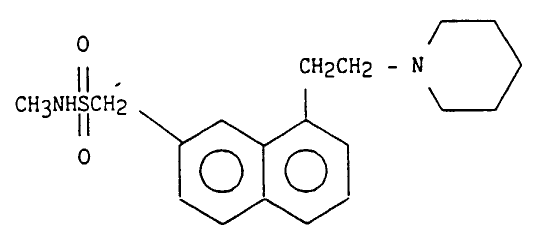

13. Compound according to claim 1 which is 2-{1-[2-(N-methylamino)ethyl]naphth-7-yl}-N-methylmethanesulphonamide of which the formula is represented below, and also the addition salts thereof with a pharmaceutically acceptable mineral or organic acid.

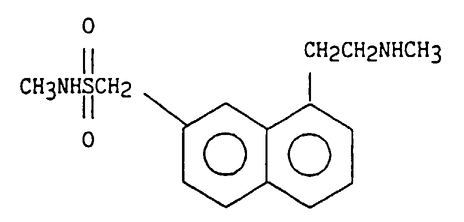

14. Process for the preparation of compounds of the general formula (I), characterised in that there is used as starting material a naphthalene compound of formula (II) : 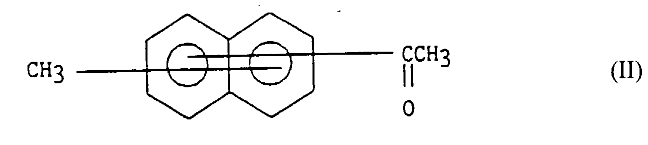 which is treated with N-bromosuccinimide to obtain the brominated compound of formula (III) : 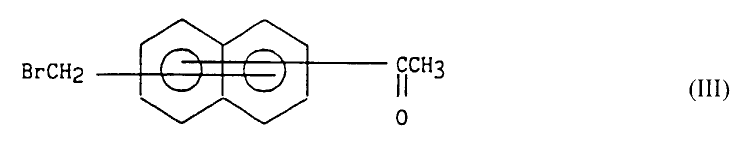 which may either :
a) be treated with sodium sulphite in an acetone/water mixture to obtain the methanesulphonate of formula (IV) : 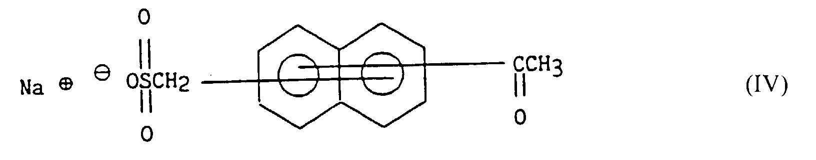 which is then treated with phosphorus oxychloride to obtain the sulphonyl chloride of formula (V) : 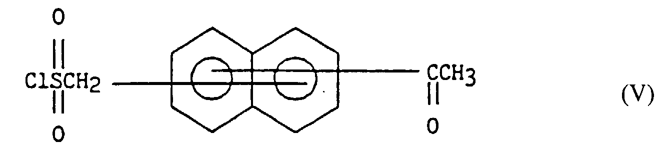 which is then reacted with an amine of formula (VI) : 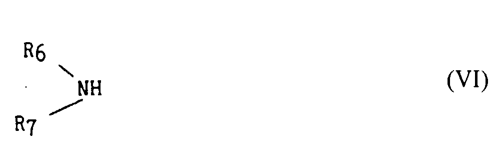 wherein R₆ and R₇ are as defined for the compounds of the general formula (I), to obtain the sulphonamide compound of formula (VII) : 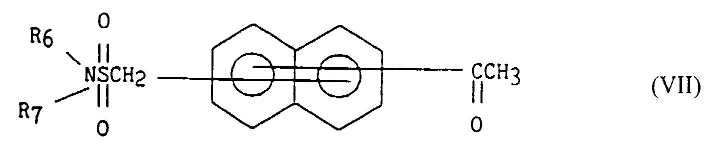 wherein R₆ and R₇ are as defined for the compounds of the general formula (I), which is then treated with bromine to obtain the brominated compound of formula (VIII) : 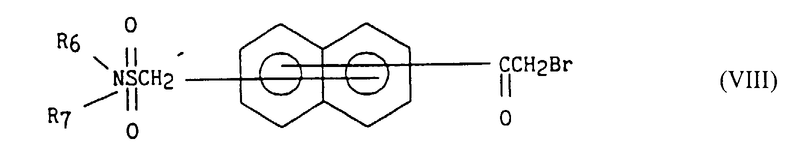 wherein R₆ and R₇ are as defined for the compounds of the general formula (I), which is then reduced with triethylsilane in trifluoroacetic acid to obtain the compound of formula (IX) : 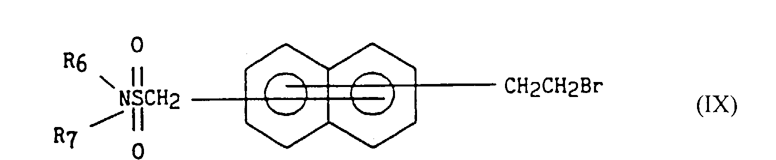 wherein R₆ and R₇ are as defined for the compounds of the general formula (I), which is reacted :
- either with an amine of formula (X) : 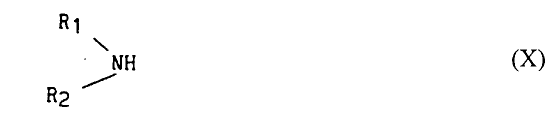 wherein R₁ and R₂ are as defined for the compounds of the general formula (I) , to obtain the compound of formula (I_{A1}) : 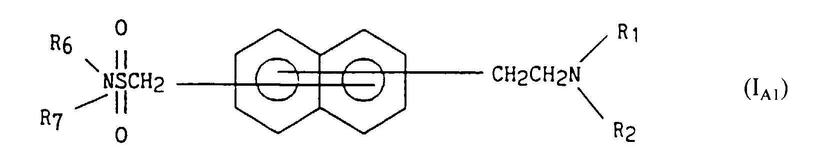 wherein R₁, R₂, R₆ and R₇ are as defined for the compounds of the general formula (I),
- or with potassium phthalimide in DMF to obtain the compound of formula (XVII_{A}) : 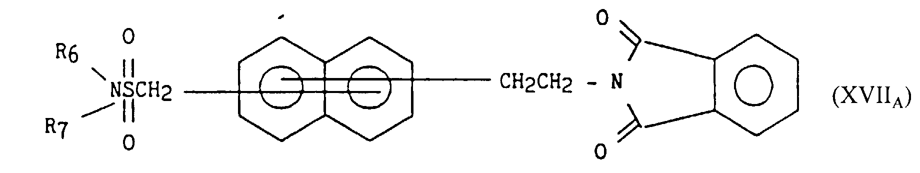 wherein R₆ and R₇ are as defined for the compounds of the general formula (I), which is reacted in an alcoholic medium with hydrazine hydrate to obtain the primary amine of formula (I_{A2}) 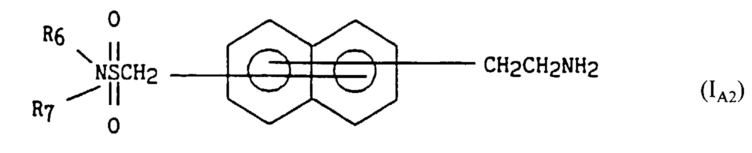 wherein R₆ and R₇ are as defined for the compounds of the general formula (I),
or
b) reacted with an alkali cyanide in DMSO or in an alcoholic medium to obtain the cyanomethyl compound of formula (XI) : 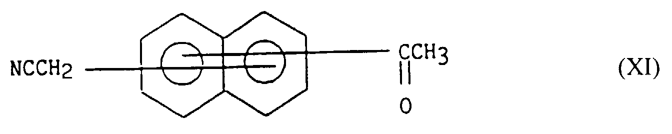 which is then hydrolysed, either in aqueous alcoholic medium with an alkali metal hydroxide, or in acidic medium, to obtain the naphthylacetic acid of formula (XII) : 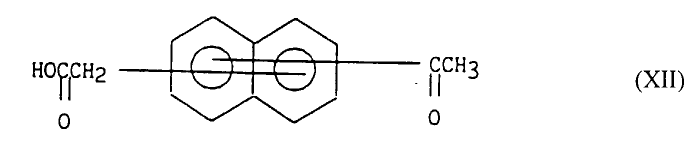 which is then treated in conventional manner with thionyl chloride, to obtain the acid chloride of formula (XIII) : 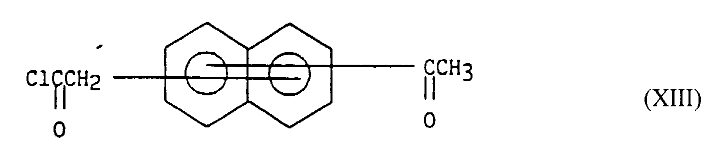 which is then reacted with an amine of formula (VI) : 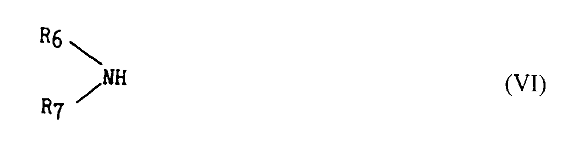 wherein R₆ and R₇ are as defined for the compounds of the general formula (I), to obtain the amide compound of formula (XIV) 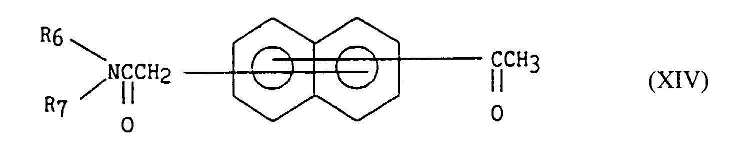 wherein R₆ and R₇ are as defined for the compounds of the general formula (I), which is then treated with bromine to obtain the brominated compound of formula (XV) : 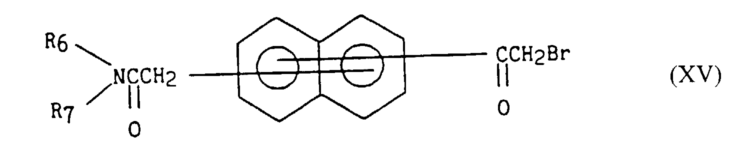 wherein R₆ and R₇ are as defined for the compounds of the general formula (I), which is then reduced with triethylsilane in trifluoroacetic acid to obtain the compound of formula (XVI) : 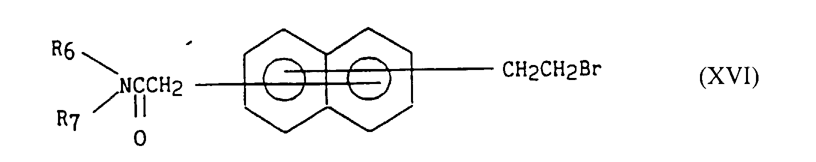 wherein R₆ and R₇ are as defined for the compounds of the general formula (I), which is reacted :
- either with an amine of formula (X) : 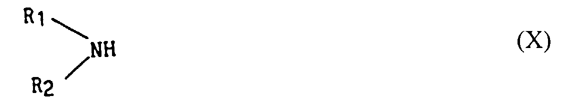 wherein R₁ and R₂ are as defined for the compounds of the general formula (I), to obtain the compound of formula (I_{B1}) : 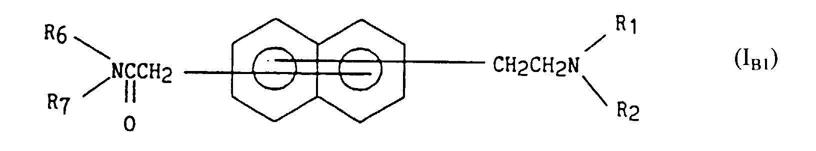 wherein R₁, R₂, R₆ and R₇ are as defined for the compounds of the general formula (I),
- or with potassium phthalimide in DMF to obtain the compound of formula (XVII_{B}) : 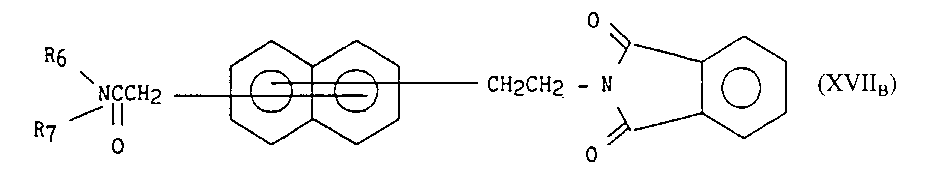 wherein R₆ and R₇ are as defined for the compounds of the general formula (I), which is reacted in alcoholic medium with hydrazine hydrate to obtain the primary amine of formula (I_{B2}) 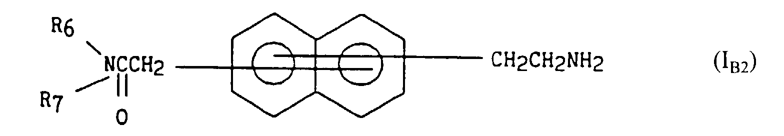 wherein R₆ and R₇ are as defined for the compounds of the general formula (I),
it being understood that the compounds of the general formulae (I_{A1}), (I_{A2}), (I_{B1}) and (I_{B2}) represent the totality of the compounds of formula (I) and, if desired, may be converted into salts with a pharmaceutically acceptable acid and, if necessary, may be separated into their different isomers.

15. Pharmaceutical composition comprising as active ingredient at least one compound according to any one of claims 1 to 13 in combination with one or more pharmaceutically acceptable, inert, non-toxic excipients or carriers.

16. Pharmaceutical composition according to claim 15 comprising at least one active ingredient according to any one of claims 1 to 13 that can be used in the treatment of disorders resulting from vasodilatation of the vascular system, more especially migraines, headaches, facial vascular neuralgia, and arterial and venous circulation disorders.

## Claims (Claims for the following Contracting State(s): ES)

1. Process for the preparation of compounds of the general formula (I) : 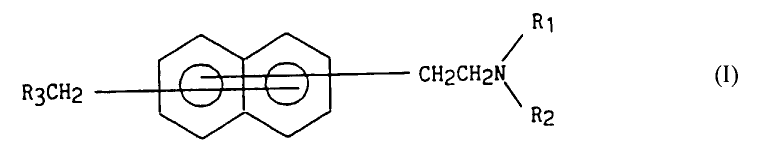 in which the two substituents, each independently of the other, may be located on either of the two rings of the naphthalene,
- R₁ and R₂, which are identical or different, each represents, independently of the other:
- a hydrogen atom,
- a linear or branched alkyl radical having from 1 to 6 carbon atoms,
- a cycloalkyl radical having from 3 to 7 carbon atoms, or a cycloalkyl-(C₁-C₄)-alkyl radical,
- a linear or branched alkenyl radical having from 2 to 6 carbon atoms,
- an optionally substituted aryl radical,
- an optionally substituted aralkyl radical in which the alkyl chain contains from 1 to 3 carbon atoms,
or, with the nitrogen atom carrying them, together form a ring system selected from : 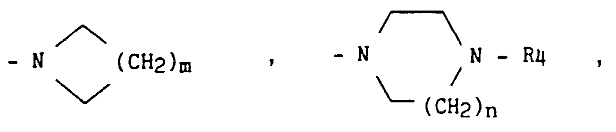 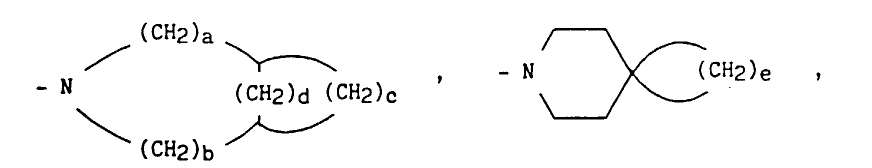 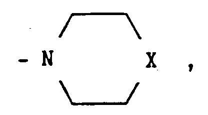
- X represents an oxygen or sulphur atom,
- m, an integer, may be 0, 1, 2, 3, 4 or 5,
- n, an integer, may be 1 or 2,
- a and b, integers, may be 0, 1, or 2 with a + b ≠ 0,
- c and d, integers, may be 0, 1, 2, 3 or 4, with c + d ≠ 0,
- e, an integer, may be 4 or 5,
- R₄ represents :
- a hydrogen atom,
- a linear or branched alkyl radical having from 1 to 6 carbon atoms,
- an optionally substituted aryl radical,
- an optionally substituted aralkyl radical in which the alkyl chain contains from 1 to 3 carbon atoms,
- a group 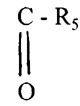 wherein R₅ represents a linear or branched alkyl radical having from 1 to 6 carbon atoms, or an optionally substituted phenyl or naphthyl radical,
- R₃ represents :
- a group 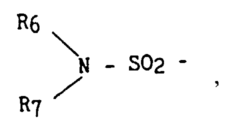
- a group 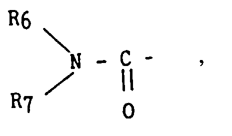
- R₆ and R₇, which are identical or different, have the same meanings as R₁ and R₂,
- their isomers, enantiomers and diastereoisomers, isolated or in the form of a mixture,
- the addition salts thereof with a pharmaceutically acceptable mineral or organic acid,
it being understood that :
- there is understood by "aryl group" phenyl, naphthyl, pyrimidyl or pyridyl,
- the expressions "optionally substituted aryl" and "optionally substituted aralkyl" denote that the aromatic nucleus or nuclei may be substituted by one or more radicals selected from hydroxy, halogen, trifluoromethyl, nitro, linear or branched alkyl having from 1 to 6 carbon atoms and linear or branched alkoxy having from 1 to 6 carbon atoms,
characterised in that there is used as starting material a naphthalene compound of formula (II) : 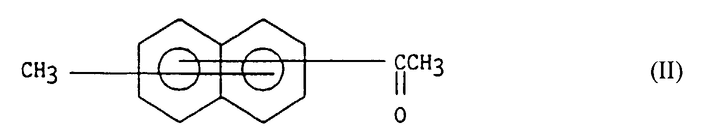 which is treated with N-bromosuccinimide to obtain the brominated compound of formula (III) : 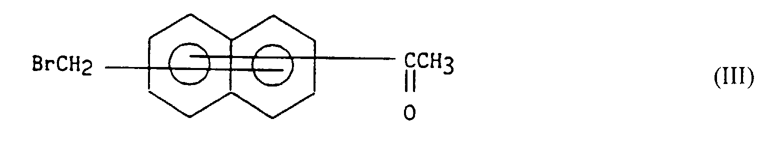 which may either :
a) be treated with sodium sulphite in an acetone/water mixture to obtain the methanesulphonate of formula (IV) : 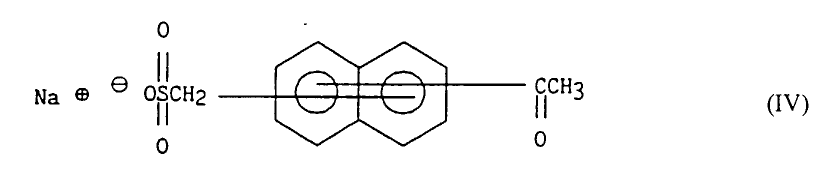 which is then treated with phosphorus oxychloride to obtain the sulphonyl chloride of formula (V) : 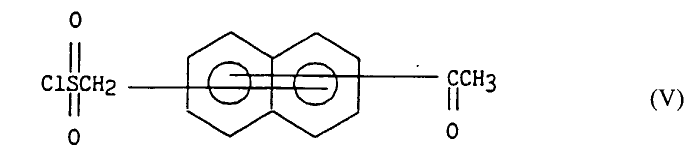 which is then reacted with an amine of formula (VI) : 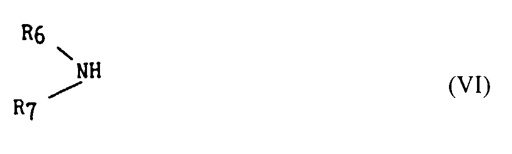 wherein R₆ and R₇ are as defined for the compounds of the general formula (I), to obtain the sulphonamide compound of formula (VII) : 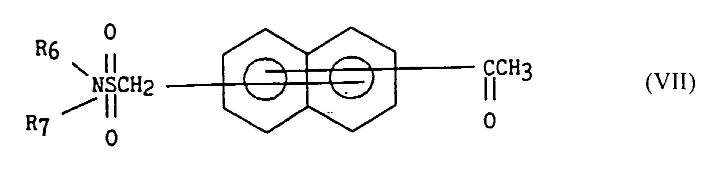 wherein R₆ and R₇ are as defined for the compounds of the general formula (I), which is then treated with bromine to obtain the brominated compound of formula (VIII) : 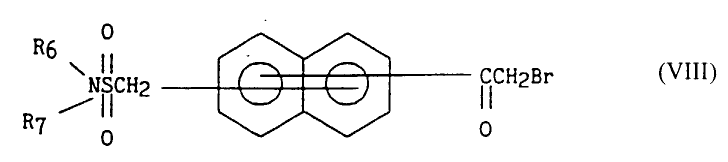 wherein R₆ and R₇ are as defined for the compounds of the general formula (I), which is then reduced with triethylsilane in trifluoroacetic acid to obtain the compound of formula (IX) : 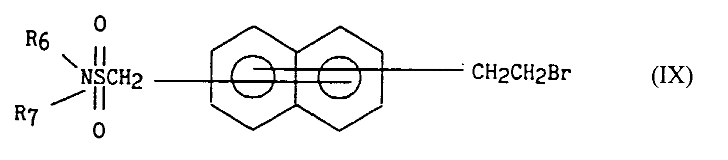 wherein R₆ and R₇ are as defined for the compounds of the general formula (I), which is reacted :
- either with an amine of formula (X) : 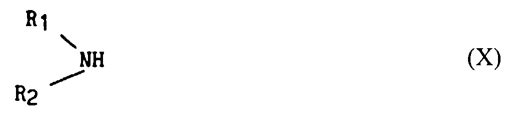 wherein R₁ and R₂ are as defined for the compounds of the general formula (I) , to obtain the compound of formula (I_{A1}) : 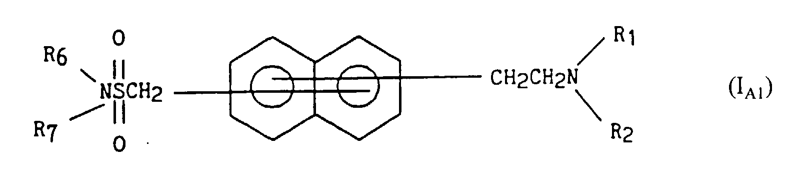 wherein R₁, R₂, R₆ and R₇ are as defined for the compounds of the general formula (I),
- or with potassium phthalimide in DMF to obtain the compound of formula (XVII_{A}) : 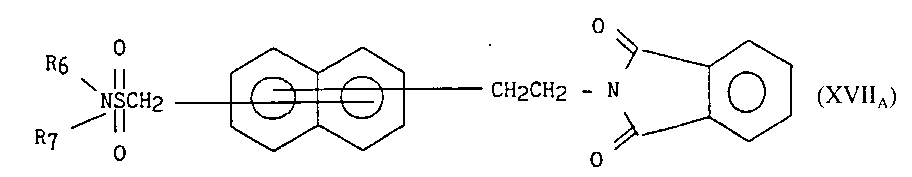 wherein R₆ and R₇ are as defined for the compounds of the general formula (I), which is reacted in an alcoholic medium with hydrazine hydrate to obtain the primary amine of formula (I_{A2}) 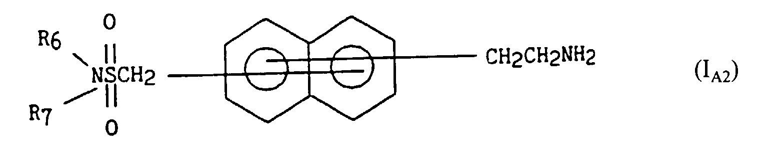 wherein R₆ and R₇ are as defined for the compounds of the general formula (I),
or
b) reacted with an alkali cyanide in DMSO or in an alcoholic medium to obtain the cyanomethyl compound of formula (XI) : 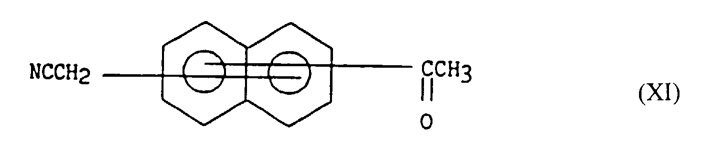 which is then hydrolysed, either in aqueous alcoholic medium with an alkali metal hydroxide, or in acidic medium, to obtain the naphthylacetic acid of formula (XII) : 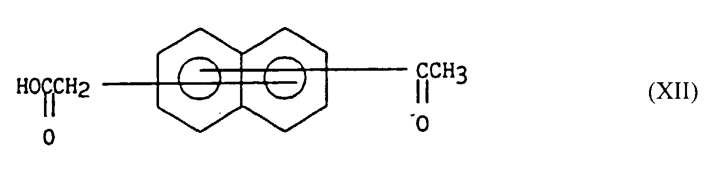 which is then treated in conventional manner with thionyl chloride, to obtain the acid chloride of formula (XIII) : 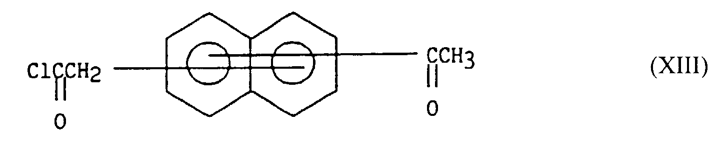 which is then reacted with an amine of formula (VI) : 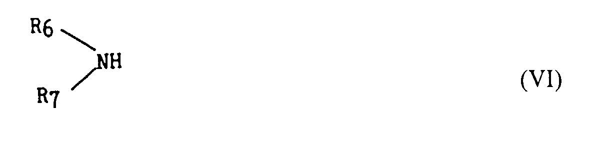 wherein R₆ and R₇ are as defined for the compounds of the general formula (I), to obtain the amide compound of formula (XIV) 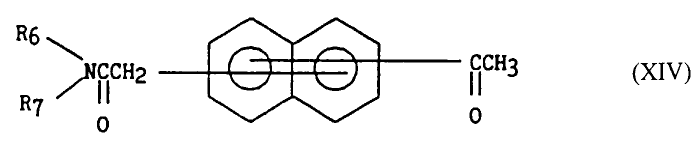 wherein R₆ and R₇ are as defined for the compounds of the general formula (I), which is then treated with bromine to obtain the brominated compound of formula (XV) : 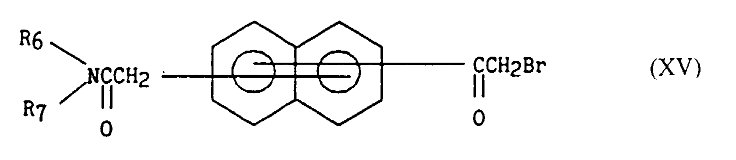 wherein R₆ and R₇ are as defined for the compounds of the general formula (I), which is then reduced with triethylsilane in trifluoroacetic acid to obtain the compound of formula (XVI) : 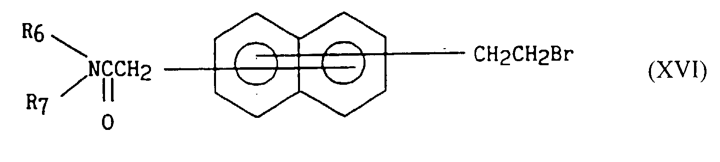 wherein R₆ and R₇ are as defined for the compounds of the general formula (I), which is reacted :
- either with an amine of formula (X) : 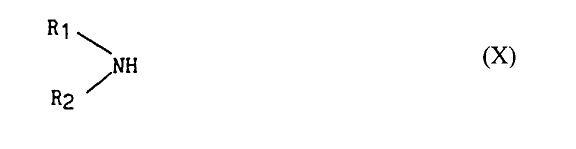 wherein R₁ and R₂ are as defined for the compounds of the general formula (I), to obtain the compound of formula (I_{B1}) : 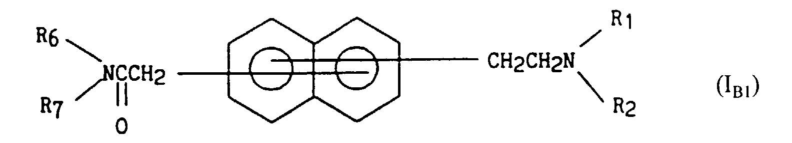 wherein R₁, R₂, R₆ and R₇ are as defined for the compounds of the general formula (I),
- or with potassium phthalimide in DMF to obtain the compound of formula (XVII_{B}) : 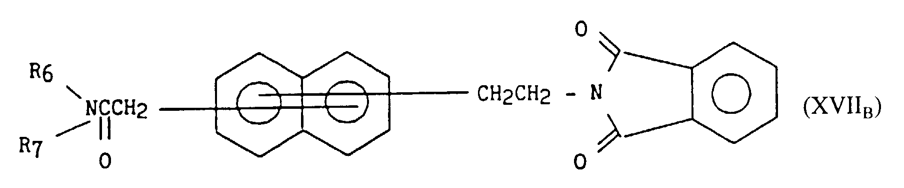 wherein R₆ and R₇ are as defined for the compounds of the general formula (I), which is reacted in alcoholic medium with hydrazine hydrate to obtain the primary amine of formula (I_{B2}) 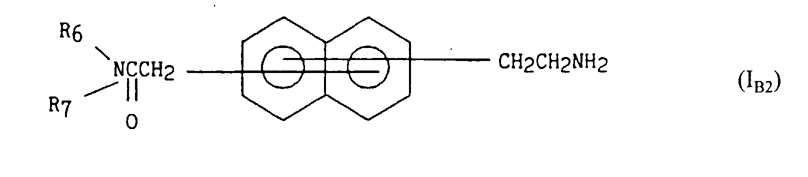 wherein R₆ and R₇ are as defined for the compounds of the general formula (I),
it being understood that the compounds of the general formulae (I_{A1}), (I_{A2}), (I_{B1}) and (I_{B2}) represent the totality of the compounds of formula (I) and, if desired, may be converted into salts with a pharmaceutically acceptable acid and, if necessary, may be separated into their different isomers.

2. Process according to claim 1 for the preparation of compounds of the general formula (I_{A}) 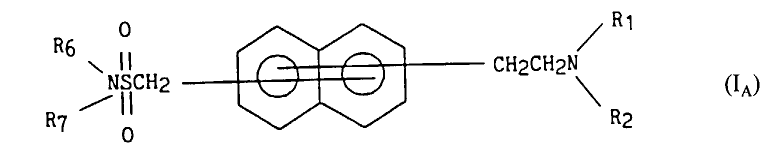 in which the two substituents, each independently of the other, may be located on either of the two rings of the naphthalene, and R₁, R₂, R₆ and R₇ are as defined in claim 1, their isomers, enantiomers and diastereoisomers, isolated or in the form of a mixture, and also the addition salts thereof with a pharmaceutically acceptable mineral or organic acid.

3. Process according to claim 1 for the preparation of compounds of the general formula (I_{B}) 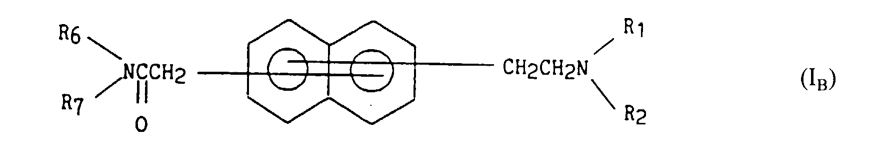 in which the two substituents, each independently of the other, may be located on either of the two rings of the naphthalene, and R₁, R₂, R₆ and R₇ are as defined in claim 1, their isomers, enantiomers and diastereoisomers, isolated or in the form of a mixture, and also the addition salts thereof with a pharmaceutically acceptable mineral or organic acid.

4. Process according to claim 1 for the preparation of compounds in which the two substituents are located at positions 1 and 7 of the naphthalene nucleus and which correspond to compounds of the following general formula : 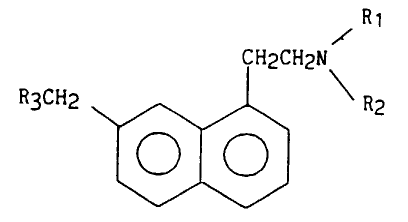 in which R₁, R₂ and R₃ are as defined in claim 1, their isomers, enantiomers and diastereoisomers, isolated or in the form of a mixture, and also the addition salts thereof with a pharmaceutically acceptable mineral or organic acid.

5. Process according to claim 1 for the preparation of compounds of the general formula (I) in which the two substituents are located at positions 1 and 7 of the naphthalene nucleus and which correspond to compounds of the following general formula : 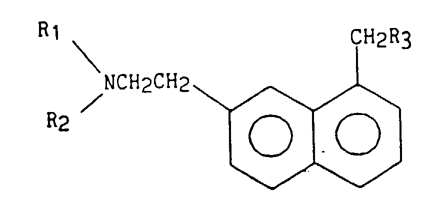 in which R₁, R₂ and R₃ are as defined in claim 1, their isomers, enantiomers and diastereoisomers, isolated or in the form of a mixture, and also the addition salts thereof with a pharmaceutically acceptable mineral or organic acid.

6. Process according to claim 1 for the preparation of the compound 2-{1-[2-(dimethylamino)ethyl]naphth-7-yl}-N-methylmethanesulphonamide of which the formula is represented below, and also the addition salts thereof with a pharmaceutically acceptable mineral or organic acid.

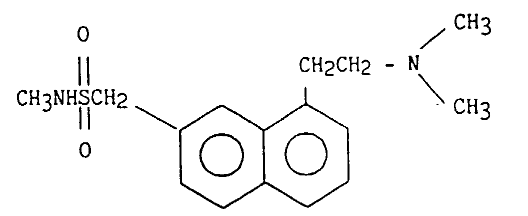

7. Process according to claim 1 for the preparation of the compound 2-{7-[2-(dimethylamino)ethyl]naphth-1-yl}-N-methylmethanesulphonamide of which the formula is represented below, and also the addition salts thereof with a pharmaceutically acceptable mineral or organic acid.

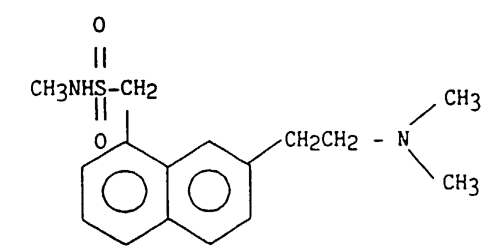

8. Process according to claim 1 for the preparation of the compound 2-{1-[2-(N-methyl-N-ethylamino)ethyl]naphth-7-yl}-N-methylmethanesulphonamide of which the formula is represented below, and also the addition salts thereof with a pharmaceutically acceptable mineral or organic acid.

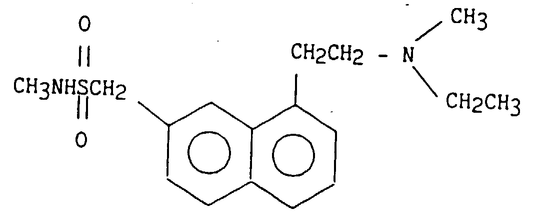

9. Process according to claim 1 for the preparation of the compound 2-{1-[2-(4-metatrifluoromethylphenylpiperazin-1-yl)ethyl]naphth-7-yl}-N-methylmethanesulphonamide of which the formula is represented below, and also the addition salts thereof with a pharmaceutically acceptable mineral or organic acid.

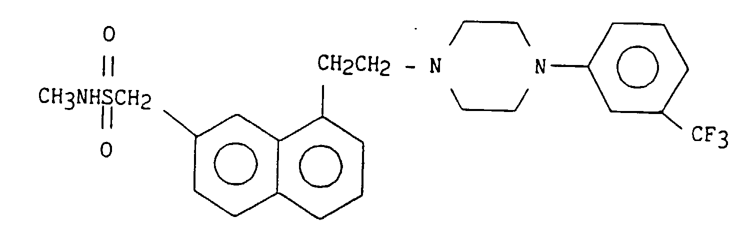

10. Process according to claim 1 for the preparation of the compound 2-{1-[2-(dimethylamino)ethyl]naphth-7-yl}-N-methylacetamide of which the formula is represented below, and also the addition salts thereof with a pharmaceutically acceptable mineral or organic acid.

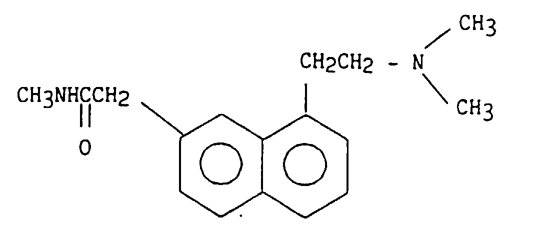

11. Process according to claim 1 for the preparation of the compound 2-{1-[2-(dimethylamino)ethyl]naphth-7-yl}-N,N-dimethylmethanesulphonamide of which the formula is represented below, and also the addition salts thereof with a pharmaceutically acceptable mineral or organic acid.

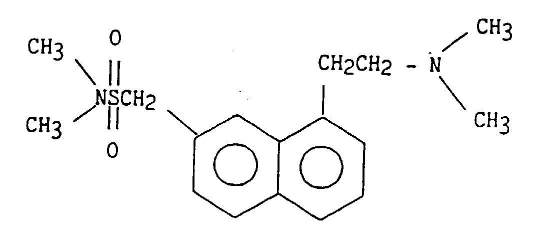

12. Process according to claim 1 for the preparation of the compound 2-[1-(2-piperidinoethyl]naphth-7-yl}-N-methylmethanesulphonamide of which the formula is represented below, and also the addition salts thereof with a pharmaceutically acceptable mineral or organic acid.

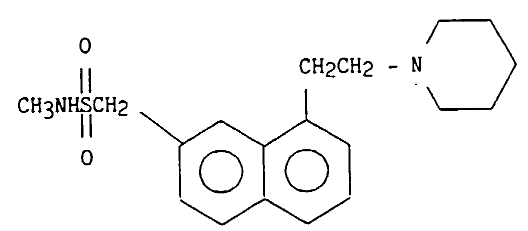

13. Process according to claim 1 for the preparation of the compound 2-{1-[2-(N-methylamino)ethyl]naphth-7-yl}-N-methylmethanesulphonamide of which the formula is represented below, and also the addition salts thereof with a pharmaceutically acceptable mineral or organic acid.

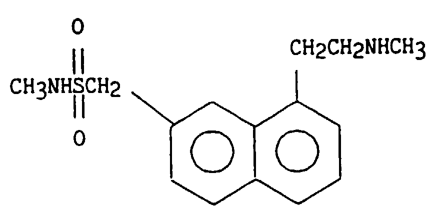

14. Process for the preparation of a pharmaceutical composition comprising as active ingredient at least one compound prepared according to any one of claims 1 to 13 in combination with one or more pharmaceutically acceptable, inert, non-toxic excipients or carriers.

15. Process for the preparation of a pharmaceutical composition according to claim 14 that can be used in the treatment of disorders resulting from vasodilatation of the vascular system, more especially migraines, headaches, facial vascular neuralgia, and arterial and venous circulation disorders.
